# EUROPEAN PATENT SPECIFICATION

(11) **EP 1 250 353 B1**
(45) Date of publication and mention of the grant of the patent: **08.04.2009**
(21) Application number: 00979782.0
(22) Date of filing: 29.11.2000
(51) Int. Cl.: C07K 14/47, A61K 38/17, G01N 33/68

(54) **P21 DERIVED PEPTIDES CAPABLE OF INHIBITING CDK/CYCLIN COMPLEXES**
P21-PEPTIDE ZUR HEMMUNG VON CDK/CYCLIN-KOMPLEXEN
PEPTIDES DERIVES DE P21 POUR L'INHIBITION DES COMPLEXES CDK/CYCLINE

(30) Priority: 30.11.1999 GB 9928323
(43) Date of publication of application: 23.10.2002
(73) Proprietor: Cyclacel Limited, London N1 7JQ (GB)
(72) Inventor: ZHELEVA, Daniella, I., Newport on Tay, Fife DD6 8NR (GB); FISCHER, Peter, M., Arbroath, Angus DD11 2EN (GB); MCINNES, Campbell, Longforgan, Dundee DD2 5JE (GB); ANDREWS, Martin, J., I., Dundee DD2 1HP (GB); CHAN, Weng, C., Nottingham, Nottinghamshire NG9 4DU (GB); ATKINSON, Gail, E., Beverley, East Yorkshire HU17 0AU (GB)
(74) Representative: Nachshen, Neil Jacob
(86) International application number: PCT/GB2000/004550
(87) International publication number: WO 2001/040142

(56) References cited:
- WO-A-96/08264
- WO-A-96/14334
- WO-A-96/35715
- WO-A-97/42222
- BALL K L ET AL: "CELL-CYCLE ARREST AND INHIBITION OF CDK4 ACTIVITY BY SMALL PEPTIDES BASED ON THE CARBOXY-TERMINAL DOMAIN OF P21WAF1" CURRENT BIOLOGY,GB,CURRENT SCIENCE,, vol. 7, no. 1, 1997, pages 71-80, XP002039815 ISSN: 0960-9822 cited in the application
- MUTOH MASATO ET AL: "A p21Waf1/Cip1 carboxyl-terminal peptide exhibited cyclin-dependent kinase-inhibitory activity and cytotoxicity when introduced into human cells." CANCER RESEARCH, vol. 59, no. 14, 15 July 1999 (1999-07-15), pages 3480-3488, XP002164635 ISSN: 0008-5472
- CHEN J ET AL: "CYCLIN-BINDING MOTIFS ARE ESSENTIAL FOR THE FUNCTION OF P21CIPI" MOLECULAR AND CELLULAR BIOLOGY,US,WASHINGTON, DC, vol. 16, no. 9, 1 September 1996 (1996-09-01), pages 4673-4682, XP000673877 ISSN: 0270-7306 cited in the application
- WARBRICK EMMA ET AL: "PCNA binding proteins in Drosophila melanogaster: The analysis of a conserved PCNA binding domain." NUCLEIC ACIDS RESEARCH, vol. 26, no. 17, pages 3925-3932, XP002164636 ISSN: 0305-1048
- CHEN, YING-NAN P. ET AL.: "SELECTIVE KILLING OF TRANSFORMED CELLS BY CYCLIN/CYCLIN-DEPENDENT KINASE 2 ANTAGONISTS" PROC NATL ACAD SCI U S A (1999) 96(8) 4325-4329, XP002164637

## Description

The present invention relates to substances and their therapeutic use, and in particular to specific regions of p21 ^{WAF1} that bind to G1 and S phase specific cyclins, preferably ones activating CDK2 and to substances and mimetics based on this region. The invention also relates to assay methods and means for identifying substances useful for interfering with protein-protein interactions involving cyclins, particularly CDK/cyclin interactions and preferably capable of inhibiting CDK2 activity.

p21^{WAF1} is an inhibitor of both the G1 cyclin dependent protein kinases (CDKs; which control the progression from G1 into S phase) (Harper et al., 1995) and proliferating cell nuclear antigen (PCNA an essential DNA-replication factor) (Florez-Rozas et al., 1994; Waga et al., 1994). Thus, inhibition of the function of either CDKs or PCNA provides, in theory, two distinct avenues for drug discovery based on the activity of p21 ^{WAF1}. The PCNA binding function of p21 ^{WAF1} can be mimicked by a 20-amino acid peptide derived from the C-terminal domain of p21^{WAF1} and this peptide is sufficient partially to inhibit SV40 replication in vitro (Warbrick et al., 1995).

Despite its PCNA binding role, the primary function of the p21^{WAF1} protein as a growth suppressor appears to be inhibition of the G1 cyclin-CDK complexes (Chen et al., 1995; Harper et al., 1995; Luo et al., 1995; Nakanishi et al., 1995b). Luo et al. (1995) reported the N-terminal domain of p21, composed of residues 1-75, to act as a CDK-inhibitor in vitro, inhibiting cyclin E-CDK2.

WO 97/42222 (Cyclacel Ltd) discloses peptide fragments of p21^{WAF1} that interact with CDK4/cyclin D1. Thus it was observed that p21(₁₆₋₃₅) and p21(₄₆₋₆₅) bind to CDK4 and cyclin D1 respectively. Of these, only p21(₁₆₋₃₅) was observed to inhibit CDK activity. p21(₁₄₁₋₁₆₀) was observed to bind to CDK4 and cyclin D1 and to be a potent inhibitor of CDK4.

This data supported the known phenomenon of peptides including the sequence LFG as being the binding motif essential for the interaction of the p21 family with cyclins [Chen J et al.(1996), Lin J et al. and Russo AA et al.] and the further known properties of the amino-terminal half of p21 as being required for binding to CDK complex.

It should be borne in mind when considering the prior art discussed herein that unless otherwise explicitly stated the references to "motifs" is made with reference to papers that have made deductions and predictions based upon the activity of longer peptides usually consisting of at least 12 amino acids. Thus, the motifs are no more than conjecture based upon the a specific set of reactions. Such motifs provide no indication as to the actual length of peptide or modifications that could be made to retain and/or even enhance activity or specificity.

The sequence p21(₁₄₁₋₁₆₀) (disclosed in WO97/42222 and Ball K. et al) in respect of cyclin D1/CDK4 inhibition was subjected to analysis in order to determine the minimum length of an inhibitory peptide upon which novel antiproliferative drugs could be designed. Observations of CDK4/cyclin D1 inhibitory activity led to the identification of an inhibitory motif comprising **RRLIF** (p21₍₁₅₅₋₁₅₉₎), the bold residues being described as essential for activity and the underlined residue contributing towards inhibitory activity. Further observations in these disclosures include the retention of inhibitory activity against cyclin D1-CDK4 by the peptide KRRLIFSK (p21(₁₅₄₋₁₆₁₎) albeit at a concentration 1000 times greater than the parent sequence p21₁₄₁₋₁₆₀ and that the substitution of aspartic acid at position 149 of p21₁₄₁₋₁₆₀ by alanine surprisingly reduced the IC₅₀ of the full length peptide from 100 nM to 46 nM. Thus, although identifying the RRLIF motif as being important to cyclinD1/CDK4 inhibition, Ball et al. is inconclusive as to the actual minimum length peptide required for enhanced activity. The effect of the Asp149 to Ala substitution has not proven reproducible.

In summary, WO97/42222 and Ball et al teach that there are sequences within the carboxy terminal region of p21 that are capable of interacting with CDK4/cyclin D in a manner that is inhibitory to CDK4 and further involves specific binding to cyclin D. Though the peptide p21(₁₄₁₋₁₆₀) is described as being preferred, an 8-mer comprising p21(₁₅₄₋₁₆₁) (KRRLIFSK) was inhibitory, but at higher concentrations. Finally, alone replacement at position 149 within p21₁₄₁₋₁₆₀ increased the inhibitory activity. Thus, although the art indicates that this is an interesting region of p21 to investigate, no guidance is provided as to the identity of further fragments that would be preferably active against CDK4/cyclin D or any other CDK/cyclin enzymes.

Chen J et al. (Mol Cell Biol (1996) 16(9) 4673-4682) disclose a 12-mer corresponding to p21₁₇₋₂₄ as being a cyclin binding domain of p21. They further identify a less avid cyclin binding region as p21₁₅₀₋₁₆₁. Mutation and inhibition analysis demonstrated that the principal site of interaction with cyclin A was p21₁₇₋₂₄, being a better inhibitor than p21₁₅₀₋₁₆₁ consistent with its greater avidity for cyclins such that it can be detected by pull-down assay. Interaction of p21₁₅₀₋₁₆₁ could only "be inferred from competition for binding and kinase inhibition assays. The importance of the p21₁₅₀₋₁₆₁ *in vivo* was questioned due to the possibility of the relevant site being occupied by PCNA.

Adams DA et al. (Mol Cell Biol (1996) 16(12) 6623-6633) discloses N- and C-terminal regions of p21 that putatively bind to CDK2/cyclin. A 14-mer (p21₁₄₉₋₁₆₂) is disclosed as inhibiting the binding of cyclin A to E2F1 and the binding of cyclins A and E to GST-p21. An amino acid sequence containing 8 amino acid residues (PVKRRLDL) derived from the transcription factor E2F1 was shown to bind to cyclin A/E-CDK2 complexes. An alanine scan of the 8-mer identified, on a qualitative level that certain modified forms of the peptide retained this activity. Noteworthy is that deletion or alanine replacement of either terminal amino acid reduced or abolished the ability to compete with GST-E2F 1 for cyclin A binding.

In a further paper, Adams DA et al. (Mol Cell Biol (1999) 19(2) 1068-1080) investigated the existence of an E2F1-like motif within pRB as a means to explain its interaction with cyclin A/CDK2. A single 10-mer, pRB869-878 was the shortest pRB derived peptide investigated.

In a subsequent paper, Chen et al. (Proc. Natn. Acad. Sci. (1999) 96, 4325-4329) disclosed two E2F1 derived 8-mers as possessing the ability to interact with the cyclin A/CDK2 complex, being PVKRRLFG and PVKRRLDL. These peptides were tested in whole cell assays using membrane translocation carrier peptides HIV-TAT or Penetratin®.

brown NR et al. (Nature Cell Biol. (1999) 1, 438-443) describe a crystal structure of the cyclin A3/phospho-CDK2 complex with an 11-mer derived from p107 including the RXLF motif. Of the 11-mer, the region RRLFGE was found to be within the binding region of cyclin A forming interactions with M210, 1213, W217, E220, L253 and Q254.

An aim of the present invention has been to identify further peptides derived from p21 1 that retain or improve upon the inhibitory activities described in the art, particularly with regard to substrate specificity and peptide chain length as described in detail below.

A first aspect of the present invention therefore relates to a p21 derived peptide preferentially inhibiting CDK2 activity over CDK4 activity of formula;

DFYHSKRRLIF (SEQ ID No. 1)

or such a peptide
(i) bearing a further amino acid residue at either end ; or,
(ii) having up to 7 amino acid residues deleted from the N-terminal end; and variants thereof wherein at least one amino acid residue is replaced by an alternative natural or unnatural replacement amino acid residue, with the proviso that the motif XLXF is retained, wherein said peptide is other than of formula

   FLRF;

   WLRF;

   FHLRF;

   or XXXLRF, XXKLRF, XXRLRF, XXHLRF, XXTLRF XXFLRF, XXSLRF, XXILRF, XXLLRF, XXALRF, XXWLRF, XXMLRF, where X is any amino acid. The peptide of SEQ ID.No. 1 corresponds to pH (149-159). In an embodiment of this aspect up to 5 amino acid residues, or more preferably 2 to 4 amino acid residues, are deleted from the N-terminal and the motif RXLXF is retained. Preferably, a further amino acid is added to the C-terminal end of the peptide. More preferably, the further amino acid is serine.

A second aspect of the present invention relates to a p21 derived peptide preferentially inhibiting CDK2 activity over CDK4 activity of formula:

X₁X₂X₃RX₄LX₅F (SEQ ID No. 2)

wherein X₁, X₃, X₄ and X₅ are any amino acid and X₂ is serine or alanine; and variants thereof.

Although the peptides of the first aspect and in some embodiments of the second aspect, include the described CDK4-inhibitory motif RRLIF, the peptides of the present invention have been shown to display preferential selectivity for CDK2 over CDK4 in contrast to those described in Ball et al.(supra) who concluded that such p21 carboxy-terminal peptides "do not have high specific activity for CDK2 inhibition, they are potent inhibitors of CDK4 activity". Thus, Ball et al. do not focus upon this region for further development for preferential CDK2 inhibitors, indeed p21₁₄₁₋₁₆₀ was shown by these authors to be 40 times more active against cyclinD1/CDK than cyclinE/CDK2. Thus, further surprising advantages of the above peptides relate to their specificity, particularly for G1 control CDK's, such as CDK2/cyclinE and CDK2/cyclin A, as opposed to mitotic control enzymes including CDK's such as CDK1/cyclin B or A and protein kinase Cα (PKCα).

Further evidence of the unexpected observation that these peptides display activity against CDK4 and CDK2 is that Ball et al. described how N-terminal truncation of p21₁₄₁₋₁₆₀ reduced CDK4/cyclin D1 inhibitory activity. The disclosure therein of RRLIF as being the CDK4-inhibitory motif was made on a theoretical basis rather than a demonstration that a peptide of that size would retain inhibitory activity. Furthermore, of the prior art disclosures discussed above, only two 8-mer peptides have been shown to be active against cyclin A/CDK2, these being the E2F1 derived peptides PVKRRLFG and PVKRRLDL. Thus, the present invention has demonstrated, in contrast to the information available in the art, that shorter, in some cases more specific and/or potent inhibitors of cyclib-CDK, especially cyclin E/CDK2 and cyclin A/CDK2 interaction may derived from within the sequence p21_{141-160.}

In one embodiment of the first aspect of the invention, the peptide may include a further amino acid residue at either the N- or C-terminus. The further residue is preferably selected from the polar residues C, N, Q, S, T and Y, and is preferably threonine when added to the N-terminus and serine, when added to the C-terminus. These last recited preferred embodiments correspond to the sequences 148-159 and 149-160 of p21 respectively. In an alternative embodiment, upto 7 amino acid residues may be deleted from the N-tenninal end of SEQ ID No. 1. In one particularly preferred embodiment 6 or 7 amino acids are deleted from the N-terminal end of SEQ ID NO:1. Such truncation may therefore give rise to peptides corresponding to p21 (150-159). p21 (151-159), p21 (152-159), p21 (153-159), p21 (154-159) p21 (155-159) and p21 (156-159) or wherein an additional serine residue is added to the C-terminal end to p21 (150-160), p21 (151-160), p21 (152-160), p21 (153-160), p21 (154-160), p21 (155-160) and p21 (156-160). Preferably, from 2 to 7 residues are deleted, most preferably seven are deleted. In each of these preferred embodiments it is preferable that, when present the serine residue corresponding to p21 (153) is replaced by an alanine residue.

Considering the second aspect of the invention, peptides and variants of the formula X₁X₂X₃RX₄LX₅F include peptides where one or more of:
(a) X1 may be deleted or may be any amino acid,
(b) X2 may be serine or alanine or a straight or branched chain amino,
(c) X3 may be a basic amino acid or straight or branched chain aliphatic amino acid,
(d) R may be unchanged or conservatively substituted (by basic amino acids),
(e) X4 may be any amino acid that is capable of providing at least one site for participating in hydrogen bonding,
(f) L may be unchanged or conservatively substituted,
(g) X5 may be any amino acid, or
(h) F may be unchanged or substituted by any aromatic amino acid.

More particularly, X₂ is preferably alanine as this provides a significant increase in the efficacy of the peptide and X₅ is preferably a non-polar amino acid residue, more preferably isoleucine or glycine, most preferably isoleucine. Of the remaining groups,

X₁, X₃ and X₄, X₁ and X₄ are both preferably basic amino acid residue, X₁ is more preferably histidine and X₄ more preferably arginine. X₃ may be a basic or polar residue, preferably lysine or cysteine. A preferred peptide in accordance with the second aspect is that of SEQ ID No.3;

HX₂KRRLX₅F (SEQ ID No. 3)

wherein X₂ and X₅ have the same meanings and preferences as above. When X₂ is serine and X₅ isoleucine the peptide corresponds to the sequence 152-159 of p21 and may hereinafter be referred to as p21(152-159). A further aspect of the invention therefore relates to a peptide HX₂KRRLX₅F (SEQ ID No. 3) and variants thereof, especially, wherein at least one amino acid residue is replaced by an alternative natural or unnatural replacement amino acid residue.

As used herein the term "variant" is used to include the peptides of SEQ ID Nos 1, 2 and 3 being modified by at least one of; deletion, addition or substitution of one or more amino acid residues, or by substitution of one or more natural amino acid residues by the corresponding D-stereomer or by a non-natural amino acid residue, chemical derivatives of the peptides, cyclic peptides derived from the peptides or from the peptide derivatives, dual peptides, multimers of the peptides and any of said peptides in the D-stereisomer form or the order of the final two residues at the C-terminus residues are reversed; provided that such variants retain the activity of the parent peptide. As used hereinafter, the term "substitution" is used as to mean "replacement" i.e. substitution of an amino acid residue means its replacement.

Preferably, the variants involve the replacement of an amino acid residue by one or more, preferably one, of those selected from the residues of alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, and valine.

Such variants may arise from homologous substitution i.e. like-for-like substitution such as basic for basic, acidic for acidic, polar for polar etc. Non-homologous substitution may also occur i.e. from one class of residue to another or alternatively involving the inclusion of unnatural amino acids such as ornithine, diaminobutyric acid, norleucine, pyriylalanine, thienylalanine, naphthylalanine and phenylglycine.

As used herein, amino acids are classified according to the following classes;
basic; H, K, R
acidic; D, E
non-polar; A, F, G, I, L, M, P, V, W
polar; C, N, Q, S, T, Y,
(using the internationally accepted amino acid single letter codes)
and homologous and non-homologous substitution is defined using these classes. Thus, homologous substitution is used to refer to substitution from within the same class, whereas non-homologous substitution refers to substitution from a different class or by an unnatural amino acid.

The variants may also arise from replacement of an amino acid residue by an unnatural amino acid residue that may be homologous or non-homologous with that it is replacing. Such unnatural amino acid residues may be selected from;-alpha* and alpha-disubstituted* amino acids, N-alkyl amino acids*, lactic acid*, halide derivatives of natural amino acids such as trifluorotyrosine*, p-Cl-phenylalanine*, p-Br-phenylalanine*, p-1-phenylalanine*, L-allyl-glycine*, B-alanine*, L-α-amino butyric acid*, L-γ-amino butyric acid*, L-α-amino isobutyric acid*, L-ε-amino caproic acid^{#}, 7-amino heptanoic acid*, L-methionine sulfone^{#*}, L-norleucine*, L-norvaline*, p-nitro-L-phenylalanine*, L-hydroxyproline^{#}, L-thioproline*, methyl derivatives of phenylalanine (Phe) such as 4-methyl-Phe*, pentamethyl-Phe*, L-Phe (4-arnino)^{#},L-Tyr (methyl)*, L-Phe (4-isopropyl)*, L-Tic (1,2,3,4-tetrahydroisoquinoline-3-carboxyl acid)*, L-diaminopropionic acid ^{#'} and L-Phe (4-benzyl)*. The notation * has been utilised for the purpose of the discussion above, to indicate the hydrophobic nature of the derivative whereas # has been utilised to indicate the hydrophilic nature of the derivative, ^{#}* indicates amphipathic characteristics. The structures and accepted three letter codes of some of these and other unnatural amino acids are given in the Examples section.

With particular reference to the first aspect of the invention (SEQ ID No. 1), a variant peptide may involve the replacement of an amino acid residue by an alanine residue. In the first aspect of the present invention, such substitution preferably takes place at any of positions 150, 151, 152, 153, 154, 158 or 160 which all display a greater selectivity for CDK2/cyclin E inhibition than CDK4/cyclin D1 inhibition as described below. Most preferably, such alanine replacement occurs at position 153 where in addition to an increase in selectivity, the observed IC₅₀ is at least two orders of magnitude greater that for the corresponding parent peptide (p21₁₄₁₋₁₆₀). In respect of the second aspect of the invention, it is also preferable that amino acid replacement is by an alanine residue, most preferably at the 153 position (X₂). Furthermore, in respect of this aspect of the invention, the variant may include the deletion of the N-terminal asparagine residue resulting in the peptide corresponding to p21(150-159). According the first aspect, a preferable peptide is one including a serine residue at the C-terminus such as the peptide D F Y H A K R R L I F S.

As discussed above, variants also include inversion of the two C-terminal amino acid residues and cyclic peptides, both of which are preferred independently as well as when taken together or in combination with any other variant. When such a variant is applied to the second or third aspects of the invention, it is to the exclusion of the peptide PVKRRLFG, unless in cyclic form.

With regard to cyclic peptides, these are preferably formed by linkage between the C-terminal amino acid residue and any upstream amino acid residue, preferably 3 amino acid residues upstream to it. Those skilled in the art will be aware as to the nature of such cyclic linkages. In some instances the participating amino acids may require modification in order to facilitate such linkage. In the context of the present invention, cyclic peptides are most conveniently prepared using variants wherein the two C-terminal amino acids are reversed, I and F when considering the first aspect of the invention, X₅ and the terminal phenylalanine residue in the second aspect etc. resulting in a linkage between I or X₅ and an upstream residue. In such circumstances the terminal amino acid residue (I or X₅) is preferably modified to be glycine, the upstream amino acid residue preferably being modified to be lysine or ornithine.

Thus, in accordance with the first aspect of the invention, the peptide may be selected from;
D F Y H A K R R L I F S
T D F Y H S K R R L I F,
A F Y H S K R R L I F S,
D A Y H S K R R L I F S,
D F A H S K R R L I F S,
D F Y A S K R R L I F S,
D F Y H A K R R L I F S,
D F Y H S A R R L I F S,
D F Y H S K R A L I F S,
D F Y H S K R RL A F S,
D F Y H S K R R L I F A,
F Y H S K R R L I F S,
Y H S K R R L I F S,
H S K R R L I F S,
D F Y H S K R R L I F,
F Y H S K R R L I F,
Y H S K R R L I F,
H S K R R L I F,
S K R R L I F,
K R R L I F,
H- Arg- Leu- Ile- Phe -NH₂
H- Arg- Arg- Leu- Ile- Phe -NH₂
H- Lys- Arg- Arg- Leu- Ile- Phe -NH₂
H- Ala- Lys- Arg- Arg- Leu- Ile- Phe -NH₂
H- His- Ala- Lys- Arg- Arg- Leu- Ile- Phe -NH₂
H- Asn- Leu- Phe- Gly -NH₂
H- Arg- Asn- Leu- Phe- Gly -NH₂
H- Abu- Arg- Asn- Leu- Phe- Gly -NH₂
H- Ala- Abu- Arg- Asn- Leu- Phe- Gly -NH₂
H- Ser- Ala- Abu- Arg- Asn- Leu- Phe- Gly -NH₂

Considering X₁X₂X₃RX₄LX₅F (SEQ ID No. 2), preferred peptides and variants thereof may include any one of or optionally at least one or more of the following;
(a) X1 is histidine, deleted or replaced by a natural or unnatural amino acid residue-such as alanine, 3-pyridylalani.ne (Pya), 2-thienylalanine (Thi), homoserine (Hse), phenylalanine, or diaminobutyric acid (Dab),
(b) X2 is alanine or an alternative natural or unnatural amino acid residue having a smaller or slightly larger aromatic or aliphatic side chain, such as glycine, aminobutyric acid (Abu), norvaline (Nva), t-butylglycine(Bug), valine, isoleucine, phenylglycine (Phg) or phenylalanine,
(c) X3 is lysine or either a basic residue such as arginine or an uncharged natural or unnatural amino acid residue, such as norleucine (Nle), aminobutyric acid (Abu) or leucine,
(d) arginine is replaced by either a basic residue such as lysine or an uncharged natural or unnatural amino acid residue, such as citrulline (Cit), homoserine, histidine, norleucine (Nle) or glutamine,
(e) X4 is or a natural or unnatural amino acid residue, such as asparagine, proline, serine, aminoisobutyric acid (Aib) or sarcosine (Sar), or an amino acid residue capable of forming a cyclic linkage such as lysine or ornithine,
(f) leucine is replaced with a natural or unnatural amino acid residue having a slightly larger aromatic or aliphatic side chain, such as norleucine, norvaline, cyclohexylalanine (Cha), phenylalanine or 1-naphthylalanine (1Nal),
(g) X5 is isoleucine or an alternative natural or unnatural amino acid residue having a slightly larger aromatic or aliphatic side chain, such as norleucine, norvaline, cyclohexylalanine (Cha), phenylalanine or 1-naphthylalanine (1Nal),
(h) phenylalanine is replaced with a natural or unnatural amino acid such as leucine, cyclohexylalanine (Cha), homophenylalanine (Hof), tyrosine, para-fluorophenylalanine (pFPhe), meta-fluorophenylalanine (mFPhe), trptophan, 1-naphthylalanine (1Nal), 2-naphthylalanine (2Nal), biphenylalanine(Bip) or (Tic),
(i) X₅ and the terminal phenylalanine residue are reversed, or
(j) the peptide is in cyclic form by for example, the formation of a linkage between the side chain of X₄ and the C-terminus residue.

In accordance with the second embodiment of the invention, the peptide may be selected from;
H S K R R L I F,
H A K R R L I F,
H S K R R L F G,
H A K R R L F G,
K A C R R L F G,
K A C R R L I F.

| | X1 | X2 | X3 | R | X4 | L | X5 | F | |
|---|---|---|---|---|---|---|---|---|---|
| H- | Ala- | Ala- | Lys- | Arg- | Arg- | Leu- | Ile- | Phe | -NH2 |
| | H- | Ala- | Lys- | Arg- | Arg- | Leu- | Ile- | Phe | -NH2 |
| H- | Pya- | Ala- | Lys- | Arg- | Arg- | Leu- | Ile- | Phe | -NH2 |
| H- | Thi- | Ala- | Lys- | Arg- | Arg- | Leu- | Ile- | Phe | -NH2 |
| H- | Hse- | Ala- | Lys- | Arg- | Arg- | Leu- | Ile- | Phe | -NH2 |
| H- | Phe- | Ala- | Lys- | Arg- | Arg- | Leu- | Ile- | Phe | -NH2 |
| H- | Dab- | Ala- | Lys- | Arg- | Arg- | Leu- | Ile- | Phe | -NH2 |
| H- | His- | Gly- | Lys- | Arg- | Arg- | Leu- | Ile- | Phe | -NH2 |
| H- | His- | Abu- | Lys- | Arg- | Arg- | Leu- | Ile- | Phe | -NH2 |
| H- | His- | Nva- | Lys- | Arg- | Arg- | Leu- | Ile- | Phe | -NH2 |
| H- | His- | Bug- | Lys- | Arg- | Arg- | Leu- | Ile- | Phe | -NH2 |
| H- | His- | Val- | Lys- | Arg- | Arg- | Leu- | Ile- | Phe | -NH2 |
| H- | His- | Ile | Lys- | Arg- | Arg- | Leu- | Ile- | Phe | -NH2 |
| H- | His- | Phg- | Lys- | Arg- | Arg- | Leu- | Ile- | Phe | -NH2 |
| H- | His- | Phe- | Lys- | Arg- | Arg- | Leu- | Ile- | Phe | -NH2 |
| H- | His- | Ala- | Lys- | Arg- | Arg- | Leu- | Ile- | Phe | -NH2 |
| H- | His- | Ala- | Ala- | Erg- | Arg- | Leu- | Ile- | Phe | -NH2 |
| H- | His- | Ala- | Nle- | Arg_ | Arg- | Leu- | Ile- | Phe | -NH2 |
| H- | His- | Ala- | Abu- | Arg- | Arg- | Leu- | Ile- | Phe | -NH2 |
| H- | His- | Ala- | Leu- | Arg- | Arg- | Leu- | Ile- | Phe | -NH2 |
| H- | His- | Ala- | Arg- | Arg- | Arg- | Leu- | Ile- | Phe | -NH2 |
| H- | His- | Ala- | Lys- | Ala- | Arg- | Leu- | Ile- | Phe | -NH2 |
| H- | His- | Ala- | Lys- | Cit- | Arg- | Leu- | Ile- | Phe | -NH2 |
| H- | His- | Ala- | Lys- | Hse- | Arg- | Leu- | Ile- | Phe | --NH2 |
| H- | His- | Ala- | Lys- | His- | Arg- | Leu- | Ile- | Phe | -NH2 |
| H- | His- | Ala- | Lys- | Nle- | Arg- | Leu- | Ile- | Phe | -NH2 |
| H- | His- | Ala- | Lys- | Gin- | Arg- | Leu- | Ile- | Phe | -NH2 |
| H- | His- | Ala- | Lys- | Lys- | Arg- | Leu- | Ile- | Phe | -NH2 |
| H- | His- | Ala- | Lys- | Arg- | Ala- | Leu- | Ile- | Phe | -NH2 |
| H- | His- | Ala- | Lys- | Arg- | Asn- | Leu- | Ile- | Phe | -NH2 |
| H- | His- | Ala- | Lys- | Arg- | Pro- | Leu- | Ile- | Phe | -NH2 |
| H- | His- | Ala- | Lys- | Arg- | Ser- | Leu- | Ile- | Phe | -NH2 |
| H- | His- | Ala- | Lys- | Arg- | Aib- | Leu- | Ile- | Phe | -NH2 |
| H- | His- | Ala- | Lys- | Arg- | Sar- | Leu- | Ile- | Phe | -NH2 |
| H- | His- | Ala- | Lys- | Arg- | Cit- | Leu- | Ile- | Phe | -NH2 |
| H- | His- | Ala- | Lys- | Arg- | Arg- | Leu- | Ile- | Phe | -NH2 |
| H- | His- | Ala- | Lys- | Arg- | Arg- | Ala- | Ile- | Phe | -NH2 |
| H- | His- | Ala- | Lys- | Arg- | Arg- | leu- | Ile- | Phe | -NH2 |
| H- | His- | Ala- | Lys- | Arg- | Arg | Ile- | Ile- | Phe | -NH2 |
| H- | His- | Ala- | Lys- | Arg- | Arg- | Val- | Ile- | Phe | -NH2 |
| H- | His- | Ala- | Lys- | Arg- | Arg- | Nle- | Ile- | Phe | -NH2 |
| H- | His- | Ala- | Lys- | Arg- | Arg- | Nva- | Ile- | Phe | -NH2 |
| H- | His- | Ala- | Lys- | Arg- | Arg- | Cha- | Ile- | Phe | -NH2 |
| H- | His- | Ala- | Lys- | Arg- | Arg- | Phe- | Ile- | Phe | -NH2 |
| H- | His- | Ala- | Lys- | Arg- | Arg- | 1Nap- | Ile- | Phe | -NH2 |
| H- | His- | Ala- | Lys- | Arg- | Arg- | Leu- | Ala- | Phe | -NH2 |
| H- | His- | Ala- | Lys- | Arg- | Arg- | Leu- | Leu- | Phe | -NH2 |
| H- | His- | Ala- | Lys- | Arg- | Arg- | led- | Val- | Phe | -NH2 |
| H- | His- | Ala- | Lys- | Arg- | Arg- | Leu- | Nle- | Phe | -NH2 |
| H- | His- | Ala- | Lys- | Arg- | Arg- | Leu- | Nva- | Phe | -NH2 |
| H- | His- | Ala- | Lys- | Arg- | Arg- | Leu- | Cha- | Phe | -NH2 |
| H- | His- | Ala- | Lys- | Arg- | Arg- | Leu- | Phe- | Phe | -NH2 |
| H- | His- | Ala- | Lys- | Arg- | Arg- | Leu- | 1Nap- | Phe | -NH2 |
| | H- | His- | Ala- | Lys- | Arg- | Arg- | Leu- | Phe | -NH2 |
| H- | His- | Ala- | Lys- | Arg- | Arg- | Leu- | Ile- | Phe | -NH2 |
| H- | His- | Ala- | Lys- | Arg- | Arg- | Leu- | Ile- | Leu | -NH2 |
| H- | His- | Ala- | Lys- | Arg- | Arg- | Leu- | Ile- | Cha | -NH2 |
| H- | His- | Ala- | Lys- | Arg- | Arg- | Leu- | Ile- | Hof | -NH2 |
| H- | His- | Ala- | Lays- | Arg- | Arg- | Leu- | Ile- | Tyr | -NH2 |
| H- | His- | Ala- | Lys- | Arg- | Arg- | Leu- | Ile- | pFPhe | -NH2 |
| H- | His- | Ala- | Lys- | Arg- | Arg- | Leu- | Ile- | mFPhe | -NH2 |
| H- | His- | Ala- | Lys- | Arg- | Arg- | Leu- | Ile- | Trp | -NH2 |
| H- | His- | Ala- | Lys- | Arg- | Arg- | Leu- | Ile- | 1Nap | -NH2 |
| H- | His- | Ala- | Lys- | Arg- | Arg- | Leu- | Ile- | 2Nap | -NH2 |
| H- | His- | Ala- | Lys- | Arg- | Arg- | Leu- | Ile- | Lys | -NH2 |
| H- | His- | Ala- | Lys- | Arg- | Arg- | Leu- | Ile- | Tic | -NH2 |
| H- | His | Ala | Lys | Arg | Arg | Leu | Ile | L-Pse | OOH |
| H- | His | Ala | Lys | Arg | Arg | Leu | Ile | D-Pse | OH |
| H- | His | Ser | Lys | Arg | Arg | Leu | Ile | L-Pse | OH |
| H- | His | Ser | Lys | Arg | Arg | Leu | Ile | D-Pse | OH |
| H- | His | Ala | Lys | Arg | Arg | Leu | Ile | L-Psa | OH |
| H- | His | Ala | Lys | Arg | Arg- | Leu | Ile | D-Psa | OH |
| H- | His | Ser | Lys | Arg | Arg | Leu | Ile | L-Psa | OH |
| H- | His | Ser | Lys | Arg | Arg | Leu | Ile | D-Psa | OH |
| H- | His | Ala | Lys | Arg | Arg | Leu | Ile | Dhp | OH |
| H- | His | Ser | Lys | Arg | Arg | Leu | Ile | Dhp | OH |
| H- | His | Ala | Lys | Arg | Arg | Leu | Ile | Pheol | |
| H- | His | Ser | Lys | Arg | Arg | Leu | Ile | Pheol | |
| H- | Ala- | Ala- | Abu- | Arg- | Arg- | Leu- | Ile- | pFPhe | -NH2 |
| H- | Ala- | Ala- | Lys- | Arg- | Arg- | Leu- | Ile- | pFPhe | -NH2 |
| H- | Ala- | Ala- | Lys- | Ars- | Cit- | Leu- | Ile- | pFPhe | -NH2 |
| H- | Ala- | Ala- | Lys- | Arg- | Arg- | Leu- | Ala- | pFPhe | -NH2 |
| H- | Ala- | Ala- | Abu- | Arg- | Ser- | Leu- | Ile- | pFPhe | -NH2 |
| H- | Ala- | Ala- | Lys- | Gln- | Arg- | Leu- | Ile- | pFPhe | -NH2 |
| H- | Ala- | Ala- | Lys- | Arg- | Arg- | Leu- | Ile- | pFPhe | -NH2 |
| H- | Gly- | Ala- | Lys- | Arg- | Arg- | Leu- | Ile- | pFPhe | -NH2 |
| H- | Ala- | Ala- | Lys- | hArg- | Arg- | Leu- | Ile- | pFPhe | -NH2 |
| H- | Ala- | Ala- | Lys- | Ser- | Arg- | Leu- | Ile- | pFPhe | -NH2 |
| H- | Ala- | Ala- | Lys- | Hse- | Arg- | Leu- | Ile- | pFPhe | -NH2 |
| H- | Ala- | Ala- | Lys- | Arg- | Lys- | Leu- | | pFPhe | -NH2 |
| H- | Ala- | Ala- | Lys- | Arg- | Om- | Leu- | Ile- | pFPhe | -NH2 |
| H- | Ala- | Ala- | Lys- | Arg- | Gin- | Leu- | Ile- | pFPhe | -NH2 |
| H- | Ala- | Ala- | Lys- | Arg- | Hse- | Leu- | Ile- | pFPhe | -NH2 |
| H- | Ala- | Ala- | Lys- | Arg- | Thr- | Leu- | Ile- | pFPhe | -NH2 |
| H- | Ala- | Ala- | Lys- | Arg- | Nva- | Leu- | Ile- | pFPhe | -NH2 |
| H- | Ala- | Ala- | Lys- | Arg- | Arg- | Phg- | Ile- | pFPhe | -NH2 |
| H- | Ala- | Ala- | Lys- | Arg- | Arg- | Met- | Ile- | pFPhe | -NH2 |
| H- | Ala- | Ala- | Lys- | Arg- | Arg- | Ala- | Ile- | pFPhe | -NH2 |
| H- | Ala- | Ala- | Lys- | Arg- | Arg- | Hof | Ile- | pFPhe | -NH2 |
| H- | Ala- | Ala- | Lys- | Arg- | Arg- | hLeu- | Ile- | pFPhe | -NH2 |
| H- | Ala- | Ala- | Lys- | Arg- | Arg- | aIle- | Ile- | pFPhe | -NH2 |
| H- | Ala- | Ala- | Lys- | Arg- | Arg- | Leu- | Gly- | pFPhe | -NH2 |
| H- | Ala- | Ala- | Lys- | Arg- | Arg- | Leu- | βAla- | pFPhe | -NH2 |
| H- | Ala- | Ala- | Lys- | Arg- | Arg- | Leu- | Phg- | pFPhe | -NH2 |
| H- | Ala- | Ala- | Lys- | Arg- | Arg- | Leu- | Aib- | pFPhe | -NH2 |
| H- | Ala- | Ala- | Lys- | Arg- | Arg- | Leu- | Sar- | pFPhe | NH2 |
| H- | Ala- | Ala- | Lys- | Arg- | Arg- | Leu- | Pro- | pFPhe | -NH2 |
| H- | Ala- | Ala- | Lys- | Arg- | Arg- | Leu- | Bug- | pFPhe | -NH2 |
| H- | Ala- | Ala- | Lys- | Arg- | Arg- | Leu- | Ser- | pFPhe | -NH2 |
| H- | Ala- | Ala- | Lys- | Arg- | Arg- | Leu- | Asp- | pFPhe | -NH2 |
| H- | Ala- | Ala- | Lys- | Arg. | Arg- | Leu- | Asn- | pFPhe | -NH2 |
| H- | Ala- | Ala- | Lys- | Arg- | Arg- | Leu- | pFPhe- | Phe | -NH2 |
| H- | Ala- | Ala- | Lys- | Arg- | Arg- | Leu- | diClPhe | Phe | -NH2 |
| H- | Ala- | Ala- | Lys- | Arg- | Arg- | Leu- | pClPhe- | Phe | -NH2 |
| H- | Ala- | Ala- | Lys- | Arg- | Arg- | Leu- | mClPhe | Phe | -NH2 |
| H- | Ala- | Ala- | Lys- | Arg- | Arg- | Leu- | oClPhe- | Phe | -NH2 |
| H- | Ala- | Ala- | Lys- | Arg- | Arg- | Leu- | pIPhe- | Phe | -NH2 |
| H- | Ala- | Ala- | Lys- | Arg- | Arg- | Leu- | TyrMe- | Phe | -NH2 |
| H- | Ala- | Ala- | Lys- | Arg- | Arg- | Leu- | Thi- | Phe | -NH2 |
| H- | Ala- | Ala- | Lys- | Arg- | Arg- | Leu- | Pya- | Phe | -NH2 |
| H- | Ala- | Ala- | Lys- | Arg- | Arg- | Leu- | Ile- | diClPhe | -NH2 |
| H- | Ala- | Ala- | Lys- | Arg- | Arg- | Leu- | Ile- | pClPhe | -NH2 |
| H- | Ala- | Ala- | Lys- | Arg- | Arg- | Leu- | Ile- | mClPhe | -NH2 |
| H- | Ala- | Ala- | Lys- | Arg- | Arg- | Leu- | Ile- | oClPhe | -NH2 |
| H- | Ala- | Ala- | Lys- | Arg- | Arg- | Leu- | Ile- | Phg | -NH2 |
| H- | Ala- | Ala- | Lys- | Arg- | Arg- | Leu- | Ile- | TyrMe | -NH2 |
| H- | Ala- | Ala- | Lys- | Arg- | Arg- | Leu- | Ile- | Thi | -NH2 |
| H- | Ala- | Ala- | Lys- | Arg- | Arg- | Leu- | Ile- | Pya | -NH2 |
| H- | Ala- | Ala- | Lys- | Arg- | Arg- | Leu- | Ile- | Inc | -NH2 |

and the cyclic peptides;
5,8-cyclo-[H-His-Ala-Lys-Arg-Lys-Leu-Phe-Gly]
5,8-cyclo-[H-His-Ala-Lys-Arg-Orn-Leu-Phe-Gly]

With particular reference to SEQ ID No. 3, a variant peptide may additionally involve the replacement of an amino acid residue by an alanine residue, the deletion of X₁ or the reversal of X₅ and the terminal phenylalanine residue. These options, by way of example result in the peptides X₂KRRLX₅F and HX₂KRRLFX₅. Most preferably, the peptide is H A K R R L I F. Further variants those discussed below.

More preferably with respect to H X₂ K R R L X₅ F (SEQ ID No.3) preferred peptides and variants thereof may include any one of or optionally at least one or more of the following;
(a) His is unchanged, deleted or replaced by D-His, Ala, Thi, Hse, Phe, or Dab,
(b) X₂ is Ala unchanged or replaced by Ser, Abu Bug or Val,
(c) Lys is unchanged or replaced by Arg or Abu,
(d) Arg is unchanged or replaced by Lys, Cit, or Gln,
(e) Arg is unchanged or modified to form a cyclic peptide with the C-terminal residue, or replaced by Cit or Ser,
(f) Leu is unchanged or replaced by Ile,
(g) X₅ is Ile unchanged, replaced by Leu or Gly if reversed with Phe,
(h) Phe is unchanged or replaced by para-fluoroPhe, meta-fluoroPhe, L-Psa, 2-Nap or Dhp,
(i) the two C-terminal residue are reversed, or
(j) the peptide is in cyclic form by virtue of a linkage between the C-terminal residue and the residue 3 upstream to it.

Especially preferred are peptides wherein X₂ is Ala and X₅ is Ile, incorporating more than one of the above variations particularly where Phe is replaced by para-fluoro-Phe and His is replaced by Ala or is deleted. Of such peptides, especially preferred are those that include further modifications where:
(a) Lys is replaced by Abu,
(b) the first Arg residue is replaced by Gln and
(c) the second Arg residue is replaced by Cit or Ser and,
(d) Ile is replaced by Ala.

Thus, preferred peptides in accordance with the preferred sequence H A K R R L I F include;

| His152 | | | | | | | | | |
|---|---|---|---|---|---|---|---|---|---|
| H- | his- | Ala- | Lys- | Arg- | Arg- | Leu- | Ile- | Phe | -NH2 |
| H- | Ala- | Ala- | Lys- | Arg- | Arg- | Leu- | Ile- | Phe | -NH2 |
| | H- | Ala- | Lys- | Arg- | Arg- | Leu- | Ile- | Phe | -NH2 |
| H- | Thi- | Ala- | Lys- | Arg- | Arg- | Leu- | Ile- | Phe | -NH2 |
| H- | Hse- | Ala- | Lys- | Arg- | Arg- | Leu- | Ile- | Phe | -NH2 |
| H- | Phe- | Ala- | Lys- | Arg- | Arg- | Leu- | Ile- | Phe | -NH2 |
| H- | Dab- | Ala- | Lys- | Arg- | Arg- | Leu- | Ile- | Phe | -NH2 |

| Ala153 | | | | | | | | | |
|---|---|---|---|---|---|---|---|---|---|
| H- | His- | Abu- | Lys- | Arg- | Arg- | Leu- | Ile- | Phe | -NH2 |
| H- | His- | Val- | Lys- | Arg- | Arg- | Leu- | Ile- | Phe | -NH2 |

| Lys154 | | | | | | | | | |
|---|---|---|---|---|---|---|---|---|---|
| H- | His- | Ala- | Arg- | Arg- | Arg- | Leu- | Ile- | Phe | -NH2 |

| Leu157 | | | | | | | | | |
|---|---|---|---|---|---|---|---|---|---|
| H- | His- | Ala- | Lys- | Arg- | Arg- | Ile- | Ile- | Phe | -NH2 |

| Ile158 | | | | | | | | | |
|---|---|---|---|---|---|---|---|---|---|
| H- | His- | Ala- | Lys- | Arg- | Arg- | Leu- | Leu- | Phe | -NH2 |

| Phe159 | | | | | | | | | |
|---|---|---|---|---|---|---|---|---|---|
| H- | His- | Ala- | Lys- | Arg- | Arg- | Leu- | Ile- | pFPhe | -NH2 |
| H- | His- | Ala- | Lys- | Arg- | Arg- | Leu- | Ile- | 2Nap | -NH2 |
| H- | His | Ala | Lys | Arg | Arg | Leu | Ile | D-Psa | OH |
| H- | His | Ser | Lys | Arg | Arg | Leu | Ile | Dhp | OH |

| Multiples | | | | | | | | | |
|---|---|---|---|---|---|---|---|---|---|
| H- | Ala- | Ala- | Abu- | Arg- | Arg- | Leu- | Ile- | pFPhe | -NH2 |
| H- | Ala- | Ala- | Lys- | Arg- | Arg- | Leu- | Ile- | pFPhe | -NH2 |
| H- | Ala- | Ala- | Lys- | Arg- | Cit- | Leu- | Ile- | pFPhe | -NH2 |
| H- | Ala- | Ala- | Lys- | Arg- | Arg- | Leu- | Ala- | pFPhe | NH2 |
| H- | Ala- | Ala- | Abu- | Arg- | Ser- | Leu- | Ile- | pFPhe | -NH2 |
| H- | Ala- | Ala- | Lys- | Gln- | Arg- | Leu- | Ile- | pFPhe | -NH2 |
| | H- | Ala- | Lys- | Arg- | Arg- | Leu- | Ile- | pFPhe | -NH2 |

The three letter notations appearing above are in accordance with IUPAC convention. The structure of various unnatural amino acid derivatives are provided in the introduction to the Examples, further expansion on nomenclature being given above.

The peptides of the present invention may be subjected to a further modification that is beneficial in the context of the present invention being conversion of the free carboxyl group of the carboxy terminal amino acid residue, to a carboxamide group. By way of example, when the peptide is of SEQ ID No.1 the carboxy terminal phenylalanine residue may have its carboxyl group converted into a carboxamide group. This modification is believed to enhance the stability of the peptide. Thus, the C-terminal amino acid residue may be in the form -C(O)-NRR', wherein R and R' are each independently selected from hydrogen, C1-6 alkyl, C1-6 alkylene or C1-6 alkynyl (collectively referred to "alk"), aryl such as benzyl or alkaryl, each optionally substituted by heteroatoms such as O, S or N.

Preferably at least one of R or R' is hydrogen, most preferably, they are both hydrogen. Thus, the present invention therefore encompasses the peptides wherein the C-terminal amino acid residue is in the carboxyl or carboxamide form.

The present invention further encompasses the above described peptides of the first, second and third aspects, their use in the inhibition of CDK2, their use in the treatment of proliferative disorders such as cancers and leukaemias where inhibition of CDK2 would be beneficial and their use in the preparation of medicaments for such use. Such preparation including their use in assays for further candidate compound as described herein. The embodiments described as being preferred in the context of the peptides of the invention apply equally to their use.

### ASSAYS

A further embodiment of the present invention relates to assays for candidate substances that are capable of modifying the cyclin interaction with CDK's, especially CDK2 and CDK4. Such assays are based upon the observation that the peptides of the invention, despite not including the generally considered "cyclin binding motif' as discussed in example 9, have been shown to bind to cyclin. Furthermore, it has been shown that the peptides of the second and further aspects of the invention competitively inhibit the binding of a peptide of the first aspect of the invention. Thus, such assays may involve incubating a candidate substance with a cyclin and a peptide of the invention and detecting either the candidate-cyclin complex or free (unbound) peptide of the invention. An example of the latter would involve the peptide of the invention being labeled such as to emit a signal when bound to a CDK. The reduction in said signal being indicative of the candidate substance binding to, or inhibiting peptide-cyclin interaction.

Suitable candidate substances include peptides, especially of from about 5 to 30 or 10 to 25 amino acids in size, based on the sequence of the various domains of p21, or variants of such peptides in which one or more residues have been substituted. Peptides from panels of peptides comprising random sequences or sequences which have been varied consistently to provide a maximally diverse panel of peptides may be used.

Suitable candidate substances also include antibody products (for example, monoclonal and polyclonal antibodies, single chain antibodies, chimeric antibodies and CDR-grafted antibodies) which are specific for p21 or cyclin binding regions thereof. Furthermore, combinatorial libraries, single-compound collections of synthetic or natural organic molecules, peptide and peptide mimetics, defined chemical entities, oligonucleotides, and natural product libraries may be screened for activity as modulators of cyclin/CDK/regulatory protein complex interactions in assays such as those described below. The candidate substances may be used in an initial screen in batches of, for example, 10 substances per reaction, and the substances of those batches which show inhibition tested individually. Candidate substances which show activity in *in vitro* screens such as those described below can then be tested in whole cell systems, such as mammalian cells.

Thus the present invention further relates to an assay for the identification of compounds that interact with cyclin A, cyclin E or cyclin D (hereinafter "a cyclin") or these cyclins when complexed with the physiologically relevant CDK, comprising;
(a) incubating a candidate compound and a peptide of the formula X₁X₂X₃RX₄LX₅F (SEQ ID No. 2) or more preferably of formula HX₂KRRLX₅F (SEQ ID No.3) or variants thereof as defined above, and a cyclin or cyclin/CDK complex,
(b) detecting binding of either the candidate compound or the peptide of formula X₁X₂X₃RX₄LX₅F/ HX₂KRRLX₅F with the cyclin.

The assays of the present invention (discussed hereinafter with reference to cyclin A) encompass screening for candidate compounds that bind a cyclin "recruitment center" or "cyclin groove" discussed above in respect of the prior art but herein defined in greater detail with reference to the amino acid sequence of preferably human cyclin A or of partially homologous and functionally equivalent mammmalian cyclins. The substrate recruitment site from previously described cyclin A/peptide complexes consists mainly of residues of the α1 (particularly residues 207-225) and α3 (particularly residues 250-269) helices, which form a shallow groove on the surface, comprised predominantly of hydrophobic residues . This is discussed in greater details in Russo AA et al. (Nature (1996) 382, 325-331) with respect to p27/cyclin A. From the X-ray structure assigned to the p27/cyclin A/CDK2 provided therein it is possible to conclude that the sequence SACRNLFG of p27 that interacts with cyclin A does so through the following interactions cyclin A:

| p27 residue | Cyclin A residues |
|---|---|
| S | E220,E224 |
| A | W217, E220, V221, E224, I281 |
| C | Y280, I281, D283 |
| R | D216, W217, E220, Q254 |
| N | Q254, T-285, Y286 |
| L | I213, L214, W217, Q254 |
| F | M210, I213, R250, G251, K252, L253, Q254 |
| G | T285 |

These residues are largely conserved in the A, B, E and D1 cyclins.

Through analysis of the interaction of the p21 peptides of the present invention with cyclin A, further distinct amino acid residues of cyclin A have been identified as being important in the interaction between cyclin A and p21, especially with respect to the inhibitory activity the peptides of the present invention display against CDK2.

The cyclin A amino acids believed to be important for interaction with the p21 derived peptides of the present invention include:

| p21 residue | Cyclin A residues | | |
|---|---|---|---|
| | Major Interaction | Intermediate Interaction | Minor Interaction |
| H | E223, E224 | W217, V219, V221 | G222, Y225, I281 |
| | | S408, E411 | |
| A | | Y225 | E223 |
| K | D284 | | E220, V279 |
| R | | I213 | A212, V215, L218 |
| | | | Q406, S408 |
| R | D283 | I213, L214 | M210, L253 |
| L | L253 | G257 | L218, I239, V256 |
| I | | R250, Q254 | |
| F | 1206, R211 | T207, L214 | M200 |

The present invention therefore includes assays for candidate compounds that interact with cyclin A by virtue of forming associations with at least two of the amino acid residues L253, I206 and R211 of cyclin A or the corresponding homologous amino acids of cyclin D or cyclin E.

In a further preferred assay, the candidate compound may form associations with at least E223, E224, D284, D283, L253, I206 and R211 of cyclin A or the corresponding homologous amino acids of cyclin D or cyclin E.

In a preferred assay, the candidate compound may form further associations with W217, V219, V221, S408, E411, Y225, I213, L214, G257, R250, Q254, T207 and L214 of cyclin A or the corresponding homologous amino acids of cyclin D or cyclin E.

In a more preferred assay, the candidate compound may form further associations with G222, Y225, I281, E223, E220, V279, A212, V215, L218, Q406, S408, M210, L253, L218, 1239, V256 and M200 of cyclin A or the corresponding homologous amino acids of cyclin D or cyclin E.

As used in this context the phrase "forming associations" is used to include any form of interaction a binding peptide may make with a peptide ligand. These include electrostatic interactions, hydrogen bonds, or hydrophobic/lipophilic interactions through Van der Waals's forces or aromatic stacking, *etc*.

Also, as used herein in the context of assays of the present invention, the term "cyclin" is used to refer to cyclin A, cyclin D or cyclin E, or regions thereof that incorporate the "cyclin groove" as hereinbefore described. Thus, an assay may be performed in accordance with the present invention if it utilises the a full length cyclin protein or a region sufficient to allow the cyclin groove to exist, for example amino acids 173-432 or 199-306 of human cyclin A.

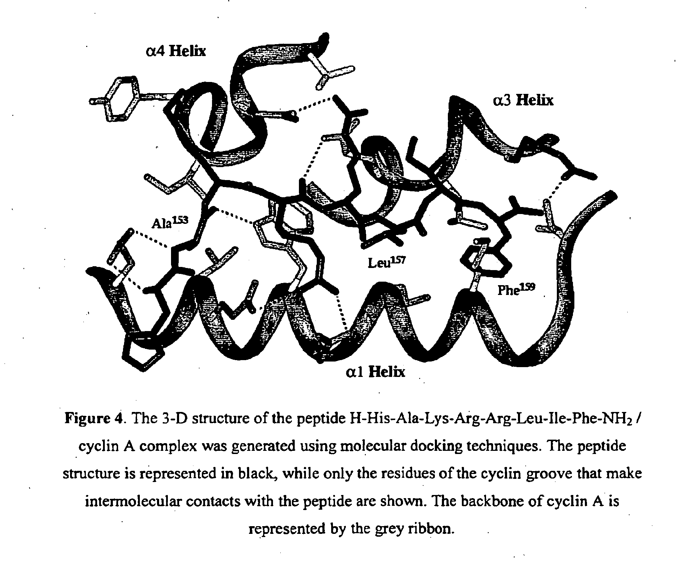

Thus, by utilising the peptides of the present invention especially those of the preferred embodiments in competitive binding assays with candidate compounds, further compounds that interact at this site may be identified and assigned utility in the control of the cell cycle by virtue of controlling, preferably inhibiting CDK2 and/or CDK4 activity. Such assays may be performed in vitro or virtually i.e. by using a three dimensional model or preferably, a computer generated model of a complex of a peptide of the present invention and cyclin A. Using such a model, candidate compounds may be designed based upon the specific interactions between the peptides of the present invention and cyclin A, the relevant bond angles and orientation between those components of the peptides of the preset invention that interact both directly and indirectly with the cyclin groove. By way of example, Figure 4 shows the interaction between the peptide HAKRRLIF and Cyclin A. From using the three dimensional model computer generated by this interaction it has been possible to identify the cyclin A amino acid residues that interact with the peptides of the present invention, particularly with HAKRRLIF as outlined above and discussed in greater detail in the examples.

As used herein the term "three dimensional model" includes both crystal structures as determined by X-ray diffraction analysis, solution structures determined by nuclear magnetic resonance spectroscopy as well as computer generated models. Such computer generated models may be created on the basis of a physically determined structure of a peptide of the present invention bound to cyclin A or on the basis of the known crystal structure of cyclin A, modified (by the constraints provided by the software) to accommodate a peptide of formula I. Suitable software suitable of the generation of such computer generated three dimensional models include AFFINITY, CATALYST and LUDI (Molecular Simulations, Inc.).

Such three dimensional models may be used in a program of rational drug design to generate further candidate compounds that will bind to cyclin A. As used herein the term "rational drug design" is used to signify the process wherein structural information about a ligand-receptor interaction is used to design and propose modified ligand candidate compounds possessing improved fit with the receptor site in terms of geometry and chemical complementarity and hence improved biological and pharmaceutical properties, such properties including, *e.g*., increased receptor affinity (potency) and simplified chemical structure. Such candidate compounds may be further compounds or synthetic organic molecules. The preferred peptides for use in these aspects of the invention are identical to those designated as preferred with respect to the first and second aspects of the invention, most especially those of the formula HX₂KRRLX₅F and of those particularly the peptide HAKRRLIF. In a preferred embodiment, rational drug design is focussed upon the four C-terminal amino acids RLX₅F or RLFX₅ or variants thereof as discussed above with respect to SEQ ID No. 3.

Using techniques known in the art, crystal or solution structures of cyclin A bound to a peptide of the present invention may be generated, these too may be used in a programme of rational drug design as discussed above.

Crystals of the p21 derived peptides of the present invention complexed with cyclin A can be grown by a number of techniques including batch crystallization, vapor diffusion (either by sitting drop or hanging drop) and by microdialysis. Seeding of the crystals in some instances is required to obtain X-ray quality crystals. Standard micro and/or macro seeding of crystals may therefore be used.

Once a crystal of the present invention is grown, X-ray diffraction data can be collected. Crystals can be characterized by using X-rays produced in a conventional source (such as a sealed tube or a rotating anode) or using a synchrotron source. Methods of characterization include, but are not limited to, precision photography, oscillation photography and diffractometer data collection. Se-Met multiwavelength anamalous dispersion data.

Once the three-dimensional structure of a protein-ligand complex formed between a p21 derived peptide of the present invention and cyclin A is determined, a candidate compound may be examined through the use of computer modeling using a docking program such as GRAM, DOCK or AUTODOCK [Dunbrack et al., 1997, Folding & Design 2:R27-42]. This procedure can include computer fitting of candidate compounds to the ligand binding site to ascertain how well the shape and the chemical structure of the candidate compound will complement the binding site. [Bugg et al., Scientific American, December:92-98 (1993); West et al;1 TIPS, 16:67-74 (1995)]. Computer programs can also be employed to estimate the attraction, repulsion and steric hindrance of the two binding partners (i.e. the ligand-binding site and the candidate compound). Generally the tighter the fit, the lower the steric hindrances, and the greater the attractive forces, the more potent the potential drug since these properties are consistent with a tighter binding constant. Furthermore, the more specificity in the design of a potential drug the more likely that the drug will not interact as well with other proteins. This will minimize potential side-effects due to unwanted interactions with other proteins.

Initially candidate compounds can be selected for their structural similarity to a p21 derived peptide of the present invention such as HAKRRLIF, the four C-terminal amino acids thereof RLX₅F or RLFX₅; or variants or a region thereof. The structural analog can then be systematically modified by computer modeling programs or by inspection until one or more promising candidate compounds are identified. A candidate compound could be obtained by initially screening a random peptide library produced by recombinant bacteriophage for example [Scott and Smith, Science, 249:386-390 (1990); Cwirla et al., Proc. Natl. Acad Sci., 87:6378-6382 (1990); Devlin et al., Science, 249:404-406 (1990)]. A peptide selected in this manner would then be systematically modified by computer modeling programs as described above, and then treated analogously to a structural analog as described below.

Once a candidate compound is identified it can be either selected from a library of chemicals as are commercially available or alternatively the candidate compound or antagonist may be synthesized de novo. As mentioned above, the de novo synthesis of one or even a relatively small group of specific compounds is reasonable in the art of drug design. The candidate compound can be placed into a standard binding assay with cyclin A together with a peptide of the present invention and its relative activity assessed.

In such an assay, cyclin A may be attached to a solid support. Methods for placing such a binding domain on the solid support are well known in the art and include such things as linking biotin to the ligand binding domain and linking avidin to the solid support. The solid support can be washed to remove unreacted species. A solution of a labeled candidate compound alone or together with a peptide of the present invention can be contacted with the solid support. The solid support is washed again to remove the candidate compound/peptide not bound to the support. The amount of labeled candidate compound remaining with the solid support and thereby bound to the ligand binding domain may be determined. Alternatively, or in addition, the dissociation constant between the labeled candidate compound and cyclin A can be determined. Alternatively, if a peptide of the present invention is used, it may be labeled and the decrease in bound labeled peptide used an indication of the relative activity of the candidate compound. Suitable labels are exemplified in our WO00/50896 (the contents of which are hereby incorporated by reference) which describes suitable fluorescent labels for use in fluorescent polarisation assays for protein/protein and protein/non-protein binding reactions. Such assay techniques are of use in the assays and methods of the present invention.

When suitable candidate compounds are identified, a supplemental crystal may be grown comprising a protein-candidate complex formed between cyclin A and the potential drug. Preferably the crystal effectively diffracts X-rays for the determination of the atomic coordinates of the protein-candidate complex to a resolution of greater than 5.0 Angstroms, more preferably greater than 3.0 Angstroms, and even more preferably greater than 2.0 Angstroms. The three-dimensional structure of the supplemental crystal may be determined by Molecular Replacement Analysis. Molecular replacement involves using a known three-dimensional structure as a search model to determine the structure of a closely related molecule or protein-candidate complex in a new crystal form. The measured X-ray diffraction properties of the new crystal are compared with the search model structure to compute the position and orientation of the protein in the new crystal. Computer programs that can be used include: X-PLOR (Bruger X-PLOR v.3.1 Manual, New Haven: Yale University (1993B)) and AMORE [J. Navaza, Acta Crystallographics ASO, 157-163 (1994)]. Once the position and orientation are known an electron density map can be calculated using the search model to provide X-ray phases. Thereafter, the electron density is inspected for structural differences and the search model is modified to conform to the new structure.

Candidates whose cyclin A binding capability has thus been verified biochemically can then form the basis for additional rounds of drug design through structure determination, model refinement, synthesis, and biochemical screening all as discussed above, until lead compounds of the desired potency and selectivity are identified. The candidate drug is then contacted with a cell that expresses cyclin A. A candidate drug is identified as a drug when it inhibits CDK2 and/or CDK4 in the cell. The cell can either by isolated from an animal, including a transformed cultured cell; or alternatively, in a living non-human animal. In such assays, and as alternative embodiments of the herein described assays, a functional endpoint may be monitored as an indications of efficacy in preference to the detection of cyclin binding. Such end-points include; G0 and/or G1/S cell cycle arrest (using flow cytometry), cell cycle-related apoptosis (sub-G0 population by fluorescence-activated cell sorting, FACS; or TUNEL assay), suppression of E2F transcription factor activity (e.g. using a cellular E2F reporter gene assay), hypophosphorylation of cellular pRb (using Western blot analysis of cell lysates with relevant phospho-specific antibodies), or generally in vitro anti-proliferative effects.

Thus, a further related aspect of the present invention relates to a three dimensional model of a peptide of the formula X₁X₂X₃RX₄LX₅F (SEQ ID No. 2) or preferably HX₂KRRLX₅F (SEQ ID No. 3): or variants thereof as defined above and cyclin A.

The invention further includes a method of using a three-dimensional model of cyclin A and a peptide of the present invention in a drug screening assay comprising;
(a) selecting a candidate compound by performing rational drug design with the three-dimensional model, wherein said selecting is performed in conjunction with computer modeling;
(b) contacting said candidate compound with cyclin A, and
(c) detecting the binding of the candidate compound; wherein a potential drug is selected on the basis of the candidate compound having a similar or greater affinity for cyclin A than that of a standard p21 derived peptide.

In a preferred embodiment the standard p21 derived peptide has the general formula HX₂KRRLX₅F as defined above. Preferably, the three dimensional model is a computer generated model.

The peptides of the invention and substances identified or identifiable by the assay methods of the invention may preferably be combined with various components to produce compositions of the invention. Preferably the compositions are combined with a pharmaceutically acceptable carrier or diluent to produce a pharmaceutical composition (which may be for human or animal use). Suitable carriers and diluents include isotonic saline solutions, for example phosphate-buffered saline. The composition of the invention may be administered by direct injection. The composition may be formulated for parenteral, intramuscular, intravenous, subcutaneous, intraocular or transdermal administration. Typically, each protein may be administered at a dose of from 0.01 to 30 mg/kg body weight, preferably from 0.1 to 10 mg/kg, more preferably from 0.1 to mg/kg body weight.

Pharmaceutically acceptable salts of the peptides of the invention include the acid addition salts (formed with free amino groups of the peptide) and which are formed with inorganic acids such as, for example, hydrochloric or phosphoric acids, or such organic acids such as acetic, oxalic, tartaric and maleic. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine and procaine.

Additional formulations which are suitable for other modes of administration include suppositories and, in some cases, oral formulations. For suppositories, traditional binders and carriers may include, for example, polyalkylene glycols or triglycerides; such suppositories may be formed from mixtures containing the active ingredient in the range of 0.5% to 10%, preferably 1% to 2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10% to 95% of active ingredient, preferably 25% to 70%. Where the vaccine composition is lyophilised, the lyophilised material may be reconstituted prior to administration, e.g. as a suspension. Reconstitution is preferably effected in buffer

Capsules, tablets and pills for oral administration to a patient may be provided with an enteric coating comprising, for example, Eudragit "S", Eudragit "L", cellulose acetate, cellulose acetate phthalate or hydroxypropylmethyl cellulose.

### Brief Description of the Figures:

Figure 1 shows the effect of p21 (149-160) on CDK2-Cyclin E induced phopshorylation of different concentrations Histone 1.
Figure 2 shows that p21 (141-160)153A is a strong inhibitor of GST-Rb phopshorylation but not of Histone **1** phosphorylation induced by CDK2-Cyclin E kinase complex.
Figure 3 shows: **a**: Interactions of p27(²⁷Ser-Ala-Cys-Arg-Asn-Leu-Phe-Gly³⁴) segment with cyclin A and **b**: conformation of the same segment (top) compared with modelled cyclic Ser-Ala-Cys-Arg-Lys-Leu-Phe-Gly peptide (bottom).
Figure 4 shows the 3-D structure of the peptide H-His-Ala-Lys-Arg-Arg-Leu-Ile-Phe-NH₂ / cyclin A complex.
Figure 5 shows a comparison of the conformation of cyclin A-complexed structures of the p21- and p27-derived peptides H-His-Ala-Lys-Arg-Arg-Leu-Ile-Phe and H-Ser-Ala-Cys-Arg-Asn-Leu-Phe-Gly-NH₂.
Figure 6 shows a comparison of modelled cyclin A groove-bound conformations of the p21(152-159)Ser153Ala peptides containing either Phe¹⁵⁹ (top) or pFPhe¹⁵⁹ (bottom).

### Examples

### Abbreviations

The nomenclature for amino acid and peptide derivatives conforms with IUPAC-IUB rules (*J. Peptide Sci.***1999***, 5*, 465 -471). D-amino acids are indicated by lower-case abbreviations, *e.g*. Ala for L-alanine, ala for D-alanine. Non-standard abbreviations for amino-acid residues are as follows:

| | | |
|---|---|---|
| Abu | 2-Aminobutyric acid | 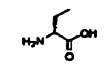 |
| Aib | Aminoisobutyric acid | 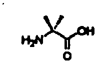 |
| Ahx | ε-Aminohexanoic acid | 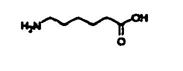 |
| hArg | Homoarginine | 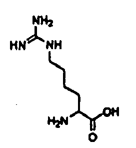 |
| Bug | *t*-Butylglycine | 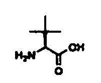 |
| oCIPhe | *o*-Chlorophenylalanine | 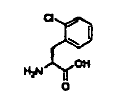 |
| mClPhe | *m*-Chlorophenylalanine | 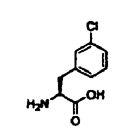 |
| pClPhe | *p*-Chlorophenylalanine | 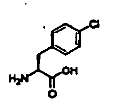 |
| Cha | Cyclohexylalanine | 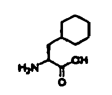 |
| DiClPhe | *m,p*-Dichlorophenylalanine | 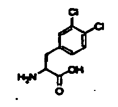 |
| Cit | Citrulline | 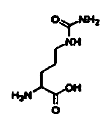 |
| Dhp | Dehydrophenylalanine | 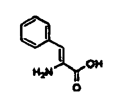 |
| Dab | 1,3-Diaminobutyric acid | 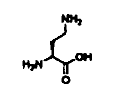 |
| mFPhe | *m*-Fluorophenylalanine | 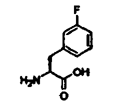 |
| pFPhe | *p*-Fluorophenylalanine | 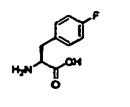 |
| Hof | Homophenylalanine | 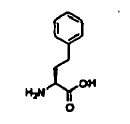 |
| Hse | Homoserine | 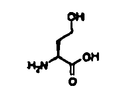 |
| aIle | *allo*-Isoleucine | |
| | | |
| Inc | 2-Indolecarboxylic acid | 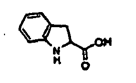 |
| pIPhe | *p*-Iodophenylalanine | 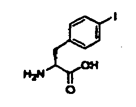 |
| 1Nap | 1-Naphthylalanine | 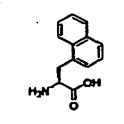 |
| 2Nap | 2-Naphthylalanine | 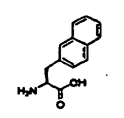 |
| Nle | Norleucine | 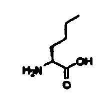 |
| Nva | Norvaline | 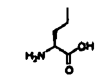 |
| Pheol | Phenylalaninol | 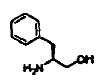 |
| Phg | Phenylglycine | 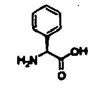 |
| Psa | *O*-Acetylphenylserine | 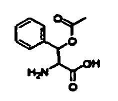 |
| Pse | Phenylserine | 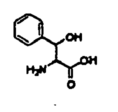 |
| Pya | 3-Pyridylalanine | 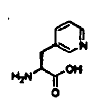 |
| Sar | Sarcosine | 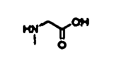 |
| Thi | 2-Thienylalanine | 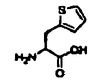 |
| Tic | 1,2,3,4-Tetrahydroisoquinoline-3-carboxylic acid | 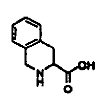 |
| Tyr(Me) | *O*-Methyltyrosine | 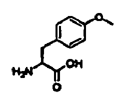 |

Other abbreviations used:

| | |
|---|---|
| Boc | *t*-Butyloxycarbonyl |
| BSA | Bovine serum albumin |
| CDK | Cyclin-dependent kinase |
| DE MALDI-TOF MS | Delayed extraction matrix-assisted laser desorption ionisation time-of-flight mass spectrometry |
| DMF | Dimethylformamide |
| ES-MS | Electrospray ionisation mass spectrometry |
| FAB-MS | Fast atom bombardment mass spectrometry |
| Fmoc | Fluoren-9-ylmethoxycarbonyl |
| Fmoc-ONSu | Fmoc *N*-hydroxysuccinimidyl ester |
| HBTU | 2-(1H-Benzotriazole-1-yl)-1,1,3,3-tetramethyluronium hexafluorophosphate |
| HOBt | 1-Hydroxybenzotriazole |
| IC₅₀ | Concentration at which 50 % inhibition is observed |
| Mtt | 4-Methyltrityl- |
| NMP | N-methylpyrrolidinone |
| Pbf | 2,2,4,6,7- Pentamethyldihydrobenzofuran-5-sulfonyl |
| Pmc | 2,2,5,7,8-Pentamethylchroman-6-sulfonyl |
| PyBOP | Benzotriazole-1-yl-oxy-tris-pyrrolidino-phosphonium hexafluorophosphate |
| RP-HPLC | Reversed-phase high-performance liquid chromatography |
| TBTU | 2-(1H-Benzatriazole-1-yl)-1,1,3,3-tetramethyluronium tetrafluoroborate |
| TFA | Trifluoroacetic acid |
| THF | Tetrahydrofuran |
| TLC | Thin layer chromatography |
| Trt | Trityl |

### Example 1: Peptide inhibitors of Rb Phosphorylation by G1 CDKs

### Experimental Procedures

Unless otherwise indicated, the peptides in the examples below were assembled using a Multipin Peptide Synthesis Kit (Chiron Technologies, Clayton, VIC, Australia; Valerio, R. M.; Bray, A. M.; Maeji, N. J. Intl. J. Peptide Protein Res. 1994, 44, 158-165 & Valerio et al., 1993) or an automated peptide synthesiser (ABI 433A). In either case, the solid-phase linker was 4-(2',4-dimethoxyphenyl-Fmoc-aminomethyl)phenoxyacetamido (Rink amide linker; Rink, H. Tetrahedron Lett. 1987, 28, 3787-3790 & Fields et al. 1990). Standard solid-phase chemistry based on the Fmoc protecting group (Atherton, E.; Sheppard, R. C. Solid phase peptide synthesis: a practical approach; IRL Press at Oxford University Press: Oxford, 1989) was employed using PyBOP- HBTU- or TBTU-mediated acylation chemistry in the presence of HOBt and Pr₂ⁱNEt, in either NMP or DMF. Repetitive Fmoc-deprotection was achieved with piperidine. The following amino acid side-chain protecting groups were used: Asp(OBu^{t}), Glu(OBu^{t}), His(Trt), Lys(Boc), Arg(Pmc), Hse(Bu^{t}), Ser(Bu^{t}), Dab(Boc), Asn (Trt), Gln(Trt), Trp(Boc). Peptides were side-chain deprotected and cleaved from the synthesis support using either of the following acidolysis mixtures: a) 2.5 : 2.5 : 95 (v/v/v) Pr₃ⁱSiH, H₂O, CF₃COOH, b) 0.75 : 0.5 : 0.5 : 0.25 :10 (w/v/v/v/v) PhOH, PhSMe, H₂O, HSCH₂CH₂SH, CF₃COOH (King et al., 1990).

Cleavage/deprotection was allowed to proceed for 2.5 h under N₂, before evaporation *in vacuo,* precipitation from Et₂O, and drying. All peptides were purified by preparative RP-HPLC or solid phase extraction (on octadecylsilane cartridges), isolated by lyophilisation, and were analyzed by analytical RP-HPLC and mass spectrometry (Dynamo DE MALDI-TOF spectrometer, ThermoBioAnalysis).

***Peptide synthesis.*** Peptides were assembled using a Multipin Peptide Synthesis Kit (Chiron Technologies, Clayton, VIC, Australia) (Valerio et al., 1993). Standard solid-phase chemistry based on the Fmoc protecting group was employed (Fields et al., 1990). Peptides were side-chain deprotected and cleaved from the synthesis support using methods as described (King et al., 1990). All peptides were purified by preparative reversed-phase HPLC or solid phase extraction, isolated by lyophilisation, and were analyzed by analytical HPLC and mass spectrometry (Dynamo DE MALDI-TOF spectrometer, ThermoBioAnalysis).

### Example 2: Production of Recombinant Proteins:

**PKCα - 6 x His, CDK4 - 6 x His, CDK2-6 x His/Cyclin E-6 x His, CDK1-6 x His/Cyclin B-6 x His** - His - tagged CDK2/Cyclin E and CDK1/Cyclin B were co-expressed and PKCα, and CDK4 were singularly expressed in Sf 9 insect cells infected with the appropriate baculovirus constructs. The cells were harvested two days after infection by low speed centrifugation and the proteins were purified from the insect cell pellets by Metal-chelate chromatography. Briefly, the insect cell pellet was lysed in Buffer A (10 mM Tris-HCl, pH 8.0, 150 mM NaCl, 0.02% NP40 and 5 mM β-marcaptoethanol, 1 mM NaF. 1 mM Na3VO4 and Protease Inhibitors Coctail (Sigma) containing AEBSF, pepstatin A, E 64, bestatin, leupeptin) by sonication. The soluble fraction was cleared by centrifugation and loaded onto Ni-NTA-Agarose (Quiagen). Non bound proteins were washed off with 300 mM NaCl, 5-15 mM Imidazole in Buffer A and the bound proteins were eluted with 250 mM Imidazole in Buffer A. The purified proteins were extensively dialyzed against Storage buffer (20 mM HEPES pH 7.4, 50 mM NaCl, 2 mM DTT, 1mM EDTA, 1 mM EGTA, 0.02% NP40, 10% v/v Glycerol) aliquoted and stored at -70°C.

PKC-α - 6 x His was purified the same way but using different buffers- 50 mM NaH2PO4, pH 8.0 and 0.05% Triton X-100 instead of Tris and NP40 respectively.

**Cyclin D1 and p21** were expressed in E coli BL21 (DE3) using PET expression vectors. BL21 (DE3) was grown at 37°C with shaking (200 rpm) to mid-log phase (OD600 nm=0.6). Expression was induced by the addition of IPTG at a final concentration of 1 mM, and the culture was incubated for a further 3h. The bacteria were than harvested by centrifugation, and the cell pellet was resuspended in 50 mM Tris-HCl, pH 7.5, 10% sucrose. Both proteins were purified from inclusion bodes. Briefly, the bacterial cells were lysed by treatment with lysosyme and sonication. The insoluble fraction was pelleted by centrifugation. The inclusion bodies were purified by repetitive washing of the insoluble fraction with 50 mM Tris-Hcl pH 8.0, 2 mM EDTA, 100 mM NaCl and 0.5% Triton X-100. Purified inclusion bodies were solubilized with the same buffer, containing 6M urea. The proteins were refolded by slow dilution with 25 mM Tris-HCl pH 8.0, 100 mM NaCl, 2 mM DTT, 1 mM EDTA, 0.2% NP40. After concentration by ultrafiltration (Amicon concentration unit) the purified proteins were aliquoted and stored at -70°C.

**GST-Rb** - An E coli expression construct containing the hyperphosphorylation domain of pRb (amino acids 773-924) was purified on a Glutathione-Sepharose column according to the manufacturers instructions (Pharmacia). For the 96-well format "in vitro" kinase assay GST-Rb was used immobilized on Glutathione-Sepharose beads.

### Example 3: Enzyme Assays

### CDK4/Cyclin D1, CDK2/Cyclin E, CDIK1/Cyclin B kinase assays

### Phosphorylation of GST Rb

GST-Rb phosphorylation, induced by CDK4/Cyclin D1, CDK2/Cyclin E or CDK1/Cyclin B was determined by incorporation of radio-labeled phosphate in GST-Rb(772-928) using radiolabelled ATP in 96-well format *in vitro* kinase assay. The phosphorylation reaction mixture (total volume 40 µl) consisted of 50 mM HEPES pH 7.4, 20 mM MgCl2, 5 mM EGTA, 2-mM DTT, 20 mM β-glycerophosphate, 2 mM NaF, 1 mM Na3VO4, Protease Inhibitors Cocktail (Sigma, see above), BSA 0.5mg/ml, 1 µg purified enzyme complex, 10 µl of GST-Rb-Sepharose beads, 100 µM ATP, 0.2pCi ³²P-ATP. The reaction was carried out for 30 min at 30°C at constant shaking. At the end of this period 100 µl of 50 mM HEPES, pH 7.4 arid 1 mM ATP were added to each well and the total volume was transferred onto GFC filtered plate. The plate was washed 5 times with 200 µl of 50 mM HEPES, pH 7.4 and 1 mM ATP. To each well were added 50 µl scintillant liquid and the radioactivity of the samples was measured on Scintilation counter (Topcount, HP). The IC50 values of different peptides were calculated using GraFit software.

### Phosphorylation of Histone

Histone 1 phosphorylation induced by CDK2/Cyclin E and CDK1/Cyclin B was measured using similar method. The concentration of Histone 1 in the kinase reaction was 1mg/ml (unless different stated). The kinase reaction was stopped by 75 mM Phosphoric acid (100 µl per well) and the reaction mixture was transferred onto P81 plates. The plates were washed 3 times with 200 µl 75 mM orthophosphoric acid.

### Protein Kinase C (PKC) α Assay

PKCα kinase activity was measured by the incorporation of radio-labeled phosphate in Histone 3. The reaction mixture (total volume 65 µl) consist of 50 mM Tris-HCl, 1 mM Calcium acetate, 3 mM DTT, 0.03 mg/ml Phosphatidylserine, 2.4 µg/ml PMA, 0.04% NP40, 12 mM Mg/Cl, purified PKCα -100 ng, Histone 3, 0.2mg/ml, 100 µM ATP, 0.2 µCi [γ-³²P]-ATP. The reaction was carried over 15 min at 37°C in microplate shaker and was stopped by adding 10 µl 75 mM orthophosphoric acid and placing the plate on ice. 50 µl of the reaction mixture was transferred onto P81 filterplate and after washing off the free radioactive phosphate (3 times with 200 µl 75 mM orthophosphoric acid per well) 50 µl of scintillation liquid (Microscint 40) were added to each well and the radioactivity was measured on Scintillation counter (Topcount, HP).

### ERK-2 (MAP Kinase) Assay

ERK-2 kinase activity was measured by the incorporation of radio-labeled phosphate into Myelin Basic Protein (MBP), catalyzed by purified mouse ERK2 (Upstate Biotecnoligies). The reaction mixture (total volume 50 µl) consisted of 20 mM MOPS, pH 7.0, 25 mM β-glycerophosphate, 5 mM EGTA, 1 mM DTT, 1 mM Na₃VO₄, 10 mM MgCl, 100 µM ATP, 0.2 pCi [γ-³²P]-ATP.

### CDK2/Cyclin A

CDK2/cyclin A kinase assays were performed in 96-well plates using recombinant CDK2/cyclin A. Assay buffer consisted of 25 mM β-glycerophosphate, 20 mM MOPS, 5 mM EGTA, 1 mM DTT, 1mM NaVO₃, pH 7.4, into which was added 2 - 4 µg of CDK2/cyclin A with substrate pRb(773-928). The reaction was initiated by addition of Mg/ATP mix (15mM MgCl₂, 100 µM ATP with 30-50 kBq per well of [γ-³²P]-ATP) and mixtures incubated for 10 - 30 min, as required, at 30 °C. Reactions were stopped on ice, followed by filtration through p81 filterplates (Whatman Polyfiltronics, Kent, UK). After washing 3 times with 75 mM orthophosphoric acid, plates were dried, scintillant added and incorporated radioactivity measured in a scintillation counter (TopCount , Packard Instruments, Pangbourne, Berks, UK).

### Competitive Cyclin D1/Cyclin A binding assay (ELISA).

Biotinylated p21 (149-159) - DFYHSKRRLIF was immobilized on Streptavidin coated 96-well plates (PIERCE). Different amounts of a competitor peptide were mixed with Cyclin D1/Cyclin A and then loaded onto the plate with immobilized biotinylated p21 (149-159). The amount of bound Cyclin D1/Cyclin A was immunodetected and quantified by Turbo-ELISA reagent (PIERCE). The IC 50 values (a concentration of the competitor peptide which inhibits 50 % of Biotin-p21 (149-159) - Cyclin D1/Cyclin A binding) were calculated using GraFit software.

### Cyclin A binding assay

Streptavidin-coated plates (Reacti-Bind^{™}, Pierce) were washed three times with TBSBSA buffer (25 mM Tris•HCl, 150 mM NaCl pH 7.5, 0.05 % Tween-20, 0.1% BSA; 200 µL) for 2 min each. A 10 mM stock solution of biotinyl-Ahx-His-Ala-Lys-Arg-Arg-Leu-Ile-Phe-NH₂ was diluted to 0.5 µM with TBS/BSA buffer. This was added to each well (100 µL). The plate was incubated for 1 h at room temperature with constant shaking. The plate was washed once quickly with TBS/BSA buffer (200 µL), followed by three more washes with TBS/BSA buffer (200 µL) for 5 min each. Serial dilutions of test peptides were prepared in a new plate (50 µL in each well). Cyclin A was diluted to 5 µg/50 µL with TBS/BSA buffer and this was then added to each well (50 µL). The solutions were mixed thoroughly with a pipette (5-6 times), before being incubated for 30 min at room temperature. This reaction mixture was then transferred to the biotinylated peptide:streptavidin-coated plate and incubated for 1 h at room temperature with constant shaking. The plate was washed once quickly with TBS/BSA buffer (200 µL), followed by three more washes with TBS/BSA buffer (200 µL) for 5 min each. The cyclin A antibody (Santa Cruz polyclonal) solution was diluted 1:200 with TBS/BSA buffer and this was then added to each well of the plate (100 µL. The plate was incubated for 1 h at room temperature with constant shaking. The plate was washed once quickly with TBS/BSA buffer (200 µl), followed by three more washes with TBS/BSA buffer (200 µL) for 5 min each. The anti-rabbit secondary antibody (goat anti-rabbit IgG peroxidase conjugate) was diluted 1:10,000 with TBS/BSA and this was then added to each well of the plate (100 µL). The plate was incubated for 1 h at room temperature with constant shaking. The plate was washed once quickly with TBS/BSA buffer (200 µL), followed by three more washes with TBS/BSA buffer (200 µL) for 5 min each. To each well was added the TMB-ELISA reagent (Pierce 1-Step^{™} Turbo TMB-ELISA; 100 µL) and the plate incubated for 1 min with constant shaking. The reaction was then quenched by the addition of 2 M aqueous H₂SO₄ (100 µL, each well). The UV absorbance of the each solution was measured spectrophotometrically at 450 nm. IC₅₀ values were calculated from dose-response curves.

### Example 4: Molecular modelling

The structure co-ordinates of the ternary complex of CDK2/cyclin A/p27^{KIP1} were obtained from the RCSB (accession code 1JSU) and used as the starting point for generating a bound complex of H-His-Ala-Lys-Arg-Arg-Leu-Ile-Phe-NH₂. The peptide was modelled by replacing the residues of the corresponding p27 peptide and manipulating the torsion angles of the Leu-Ile-Phe hydrophobic motif to approximate the bound positioning of the Leu and Phe residues. This structure was then docked into the cyclin groove using the Affinity program (Molecular Simulations, San Diego, CA). This molecular docking routine, which incorporates a full molecular mechanics approach, allows for flexibility both in the ligand and in the side chains and backbone of the receptor. For these calculations the side chains and non-a carbons of the cyclin groove were allowed to sample a range of conformational space during optimisation of the peptide/ protein complex. The calculation was performed using the CVFF force field, in a two-step process using an implicitly derived solvation model and geometric hydrogen bond restraints. For the initial phase of the calculation, the peptide was minimised into the groove using a simple non-bonded method where the Coulombic and Van der Waals terms are scaled to zero and 0.1, respectively. The subsequent refinement phase involved conformational sampling using molecular dynamics calculated over 5 ps in 100 fs stages, where the temperature is scaled from 500 K to 300 K. The calculation was completed by a final minimisation over 1,000 steps using the Polak-Ribiere Conjugate Gradient method.

### Example 5: Structure-activity relationships of p21(145-164) peptides with respect to inhibition of cyclin E/CDK2 and cyclin D1/CDK4

Previous studies have shown that a 20-residue peptide, derived from the C-terminus of p21^{WAF1} (residues 141-160) binds to CDK4 and cyclin D1 and is able to inhibit *in vitro* kinase activity of the CDK4/cyclin D1 complex (Ball, K. L.; Lain, S.; Fåhraeus, R.; Smythe, C.; Lane, D. P. Curr. Biol. 1996, 7, 71-80). In order to define the pharmacophore region of the p21^{WAF1} *C*-terminus, we synthesised 12mer overlapping peptides covering the region of p21(145-164). The *in vitro* effect of these peptides on CDK4/cyclin D1 and CDK2/cyclin E kinase activity in terms of inhibition of phosphorylation of GST-pRb was investigated.

We have demonstrated that a shorter sequence being a 12 amino acid peptide DFYHSKRRLIFS - p21 (149-160) appeared to have very similar activity as the original 20-mer peptide of Ball et al.. with respect to in vitro inhibitory activity in vitro CDK4-Cyclin D 1 kinase.

A detailed SAR analysis of p21 (149-160) was done in 96-well format CDK4-Cyclin D1 kinase assay using different peptide derivatives - truncations and alanine substitutions. In order to determine the relative importance of each position of the 12 amino acid peptide which contained the binding domain, we synthesised p21(149-160) derivatives, where each residue was sequentially substituted with Ala. The effect of the peptide mutations on their kinase inhibitory activity was then tested. Ala substitution of Phe¹⁵⁰, Tyr¹⁵¹, His¹⁵² Ile¹⁵⁸, and Ser¹⁶⁰ did not change significantly the CDK2/cyclin E inhibitory activity of p21(149-160). Substitution of Ser¹⁵³ with Ala increased 100-fold the inhibitory potency of p21 (149-160) towards CDK2/cyclin E: The results are shown in Table 1.

### SAR of p21 (149-160) in-CDK2/Cyclin E kinase assay.

P21 (141-160) peptide was shown to inhibit CDK2-Cyclin E induced phosphorylation of GST-Rb (Ball et al., 1995) at concentration 40 times its IC50 of CDK4/cyclin D1. The results herein show that a truncated form- p21 (149-160) and variants thereof, retain very good potency to inhibit the CDK2-Cyclin E induced phosphorylation of GST-Rb and in many cases the peptides were shown to be preferentially inhibitory of CDK2 as opposed to CDK4. Detailed SAR of p21 (149-160) were determined in CDK2-Cyclin E *in vitro* kinase assay. The data are shown in Table 1.

A comparison between the SAR of p21 (149-160) in CDK2-Cyclin E and CDK4-Cyclin D1 kinase assays shows a higher inhibitory activity towards CDK2-Cyclin E than to CDK4-Cyclin D1. Alanine mutation of Ser153 increases 100 fold the potency of the peptide to inhibit the CDK2-Cyclin E but has little effect on CDK4-Cyclin D1 induced phosphorylation of GST-Rb. For both inhibitory activities of p21 (149-160) the most important residues are Arg155, Leu 157 and Phe 159. The CDK4-Cyclin D1 inhibitory activity of p21 (149-160) tolerates less changes than the CDK2-Cyclin E one.

Using identical assays, the sequence p21(148-159) was shown to be active against both CDK2/cyclin E and CDK4/cyclin D1.

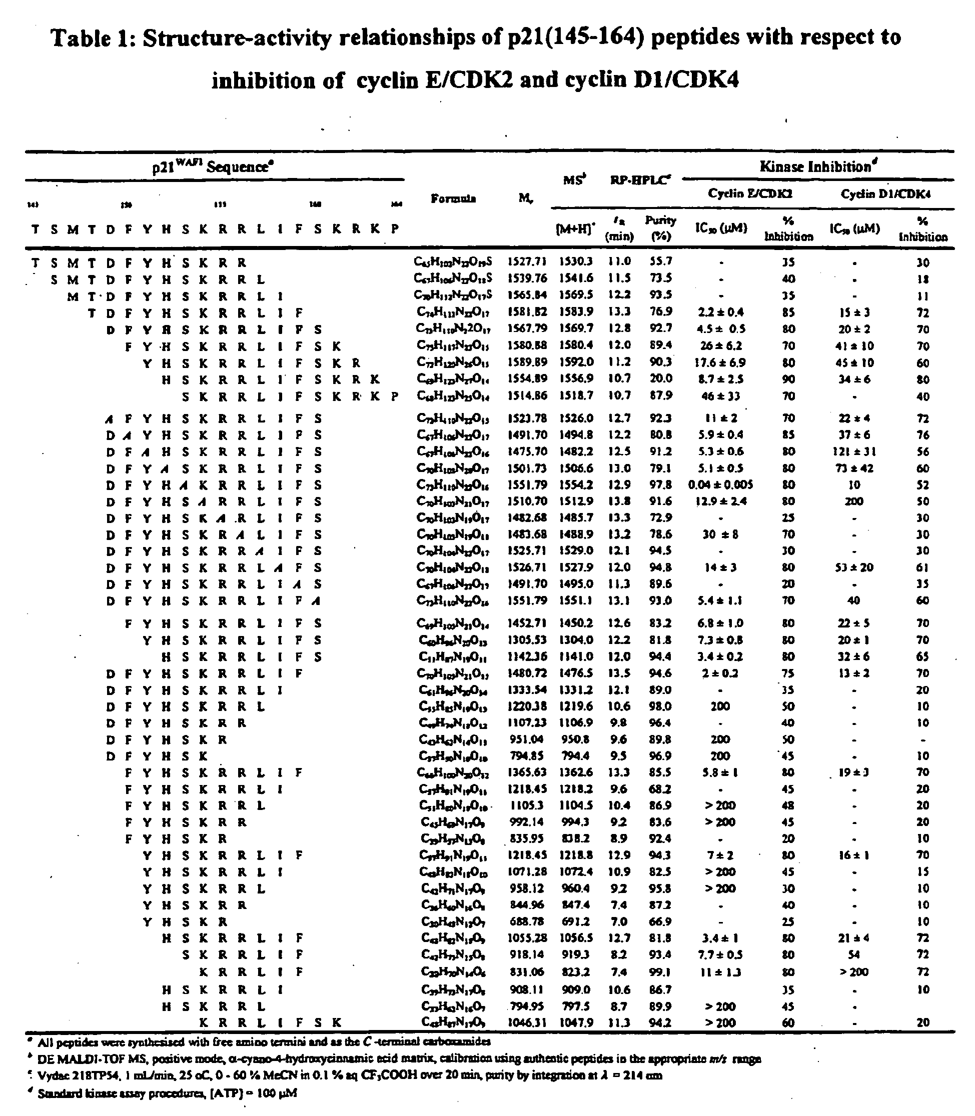

### Example 6: Specificity of Enzyme inhibition

### Effect of p21 (149-160) on CDK2-Cyclin E induced phosphorylation of Histone 1.

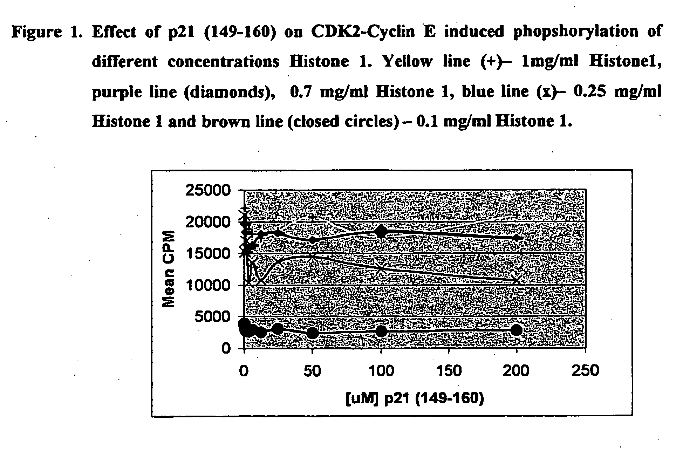

p21 (149-160) was tested for inhibitory activity in a CDK2/cyclin E kinase assay with histone H1 as a substrate. The peptide was completely inactive as an inhibitor of CDK2/cyclin E-induced phosphorylation of histone H1 *(**Figure 1**).*

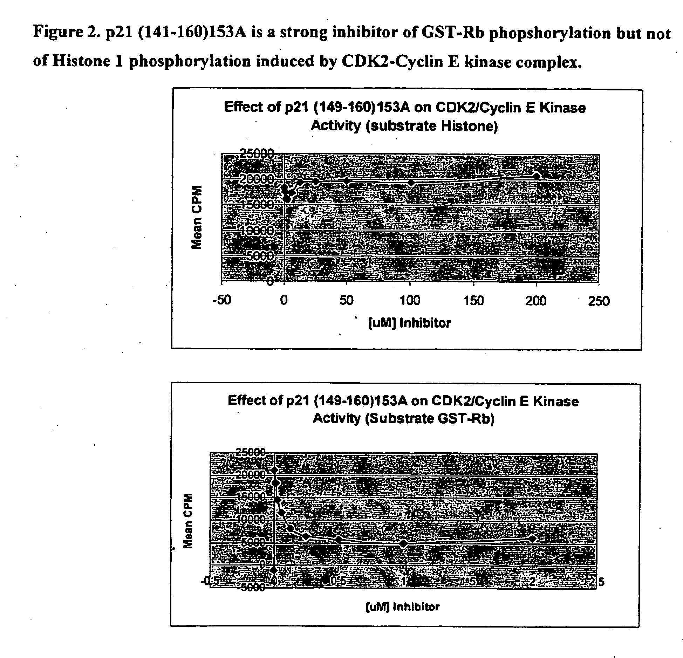

One possible mechanism for inhibitory action is competition of the peptide with the substrate for binding to the kinase complex. If this is so, the peptide inhibitory activity will depend on the substrate concentration. We determined the IC₅₀ of p21(149-160) in the presence of different concentrations of histone H1 but p21(149-160) did not inhibit CDK2/cyclin E-induced phosphorylation of histone H1 at any of the substrate concentrations used. The most potent inhibitor of CDK2/cyclin E phosphorylation of GST-pRb, i.e. p21(149-160)Ser153A1a was also tested for its ability to inhibit histone H1 phosphorylation induced by the same kinase complex *(**Figure 2*). Even this powerful inhibitor of the GST-pRb phosphorylation was completely inactive in inhibition of the phosphorylation of histone H1 induced by CDK2/cyclin E kinase complex. Full-length p21 ^{WAF1}, on the other hand, inhibited strongly both the CDK2/yclin E- and CDK4/cyclin D1-induced phosphorylation of GST-pRb and histone H1. The substrate-specific effect of p21(149-160) and its derivatives strongly suggests a mechanism of competitive binding of the peptide inhibitors and pRb to CDK2/cyclin E and CDK4/cyclin D1. The fact that p21(149-160) and its derivatives did not inhibit significantly the CDK1/cyclin B-induced phosphorylation of GST-pRb (see below) excludes a possibility of direct binding of the peptide to the substrate.

### Effect of p21 (149-160) and its derivatives on CDK1-Cyclin B kinase activity.

p21(149-160) and its derivatives were tested for ability to inhibit CDK1/cyclin B kinase activity in phosphorylating histone H1 or GST-pRb *(Table* 2). p21(149-160) and its Ala mutant p21(149-160) Ser153A1a did not have any significant effect on the CDK1/cyclin B-induced phosphorylation of histone H1. None of the tested peptides were able to inhibit significantly the CDK1/cyclin B-induced phosphorylation of GST-pRb and only the highest peptide concentrations used (200 µM) had a marginal inhibitory effect on CDK1/cyclin B kinase activity. When tested in the "pull-down" assay, immobilised p21(149-160) was unable to precipitate cyclin B either as a monomer, or as a complex with CDK1. These data coincide with the very poor inhibitory activity of the original 20mer p21(141-160) peptide (Ball, K. L.; Lain, S.; Fåhraeus, R.; Smythe, C.; Lane, D. P. Curr. Biol. 1996, 7, 71-80) and the full-length p21 ^{WAF1} protein towards CDK1/cyclin B complex (Harper, J. W.; Elledge, S. J.; Keyomarsi, K.; Dynlacht, B.; Tsai, L. H.; Zhang, P.; Dobrowolski, S.; Bai, C.; Connell-Crowley, L.; Swindell, E.; et al. Molec. Biol. Cell 1995, 6, 387-400) and show that p21(149-160) and its derivatives retain the selectivity of the full-length protein.

**Table 2 Inhibition of CDK1-Cyclin B induced phosphorylation of Histone 1 and GST-Rb by p21 derived peptides.**

| Peptide | Sequence | Histone | GST-Rb |
|---|---|---|---|
| | | IC50 [µM] | IC50 [µM] |
| P21 (149-160) | DFYHSKRRLIFS | > 200 | 200 |
| P21 (149-160)153A | DFYHAKRRLIFS | 200 | >200 |
| P21 (149-159) | DFYHSKRRLIF | Not tested | >200 |

### Effect of purified P21 ^{WAF1} on CDK4-Cyclin D1 and CDK2-Cyclin E kinase activity

In order to evaluate the selectivity, specificity and potency of p21 (149-160) and its derivatives we compared their effect with the one of purified p21 on kinase activity of CDK2-Cyclin E and CDK4-Cyclin D1. The IC 50 values characterizing the inhibition of CDK4-Cyclin D1 and CDK2-Cyclin E induced phosphorylation of GST-Rb and CDK2-Cyclin E induced phosphorylation of Histone1 by purified p21 ^{WAF1} are shown in Table 3. The IC 50 of the most active peptide - p21 (149-160) 153A for CDK2-Cyclin E induced phosphorylation of GST-Rb was 40 nM which is approximately 50 fold higher than the IC 50 value for p21^{WAF1}. Purified p21 though, inhibited strongly the CDK2-Cyclin E induced phosphorylation of GST-Rb as well as Histone 1. The peptides derived from p21 ^{WAF1}. p21 (149-160) and p21 (149-160)153A peptides specifically inhibit the GST-Rb phosphorylation, but do not inhibit the Histone 1 phosphorylation induced by CDK2-Cyclin E. This substrate specific effect of p21 (149-160) and its derivatives strongly suggest a mechanism of competitive binding of the peptide inhibitors and Rb to CDK2-Cyclin E or CDK4-Cyclin D1. The fact that p21 (149-160) and its derivatives did not inhibit significantly the CDK1-Cyclin B induced phosphorylation of GST- Rb excludes a possibility for direct binding of the peptide to the substrate (see Table 2).

**Table 3 Inhibition of CDK4-Cyclin D1 and CDK2-Cyclin E kinase activity by purified p21 ^{WAF1}**

| **Kinase complex** | **Substrate** | **Inhibition by p21^{WAF1}** |
|---|---|---|
| | | **IC50 [nM]** |
| CDK4-Cyclin D1 | GST-Rb(772-928) | 6.5 ± 0.8 |
| CDK2-Cyclin E | GST-Rb(772-928) | 0.7 ± 0.2 |
| CDK2-Cyclin E | Histone 1 | 1.8 ± 0.4 |

### Example 7: P21 (149-160) and its derivatives do not inhibit PKCα and ERK2 kinase activity in vitro

To investigate further the specificity of p21 (149-160) and its derivatives we investigated the effect of the strongest inhibitors of CDK2-Cyclin E and CDK4-Cyclin D1 complexes on PKC α and ERK2 kinase activity (Table 4). None of the tested peptides (at concentrations up to 100 µM) had any inhibitory effect on PKCα phosphorylation of Histone 3 or ERK2 phosphorylation of Myelin Basic Protein. These results demonstrate further the selectivity of the inhibitory effect of the peptides derived from p21 C-terminus.

**Table 4: Effect of p21^{WAF1}-derived peptides on PKCα and ERK2 kinase activity (activities against CDK2/cyclin E and CDK4/cyclin D1 included for comparison)**

| **Peptide** | **Sequence^{a}** | **IC₅₀ (mM)** | | | |
|---|---|---|---|---|---|
| | | **CDK4-D1** | **CDK2-E** | **PKCA** | **ERK2** |
| p21(148-159) | TDFYHSKRRLIF | 15 | 2.2 | > 100 | > 100 |
| p21(149-160) | DFYHSKRRLIFS | 20 | 4.5 | > 100 | > 100 |
| p21(149-160)S153A | DFYHAKRRLIFS | 10 | 0.04 | > 100 | > 100 |
| p21(149-159) | DFYHSKRRLIF | 13 | 2 | > 100 | > 100 |
| p21(150-159) | FYHSKRRLIF | 19 | 5.8 | > 100 | > 100 |
| p21(151-159) | YHSKRRLIF | 16 | 7 | > 100 | > 100 |
| p21(152-159) | HSKRRLIF | 21 | 3.4 | > 100 | > 100 |

| | | | | | |
|---|---|---|---|---|---|
| *^{a}* All peptides were synthesised with free amino termini and as the C-terminal carboxamides | | | | | |

### Example 8: P21 (149-159) binds to the Cyclin, but does not bind to the CDK sub-unit of CDK/Cvclin complex. Blinding of the peptide to the Cyclin does not disrupt the complex.

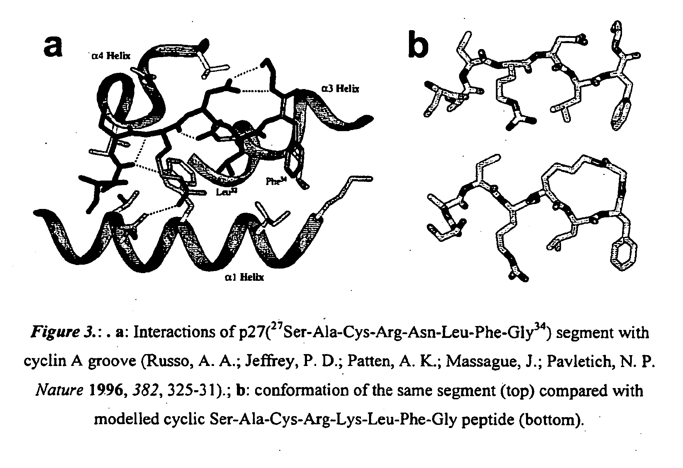

A biotinylated version of p21(149-159) was used in "pull-down" experiments with the purified CDK sub-units, CDK2, CDK4, cyclin A, cyclin D1, and with the complexes of CDK2/cyclin A or CDK4/cyclin D 1 kinases, in order to determine the binding partner of the peptide. The biotinylated peptide was pre-immobilised on streptavidin-agarose beads. Figure 3 shows the profiles of the "pulled down" proteins, after SDS-PAGE , Western blotting and immunodetection.-It was found that p21(149-159) bound to cyclin A and cyclin D1, but failed to interact with CDK2 or CDK4 in the absence of their respective cyclin partners. Both CDK2 and CDK4 were "pulled down", however, with biotinylated p21(149-159) - streptavidin-agarose beads when they were in a complex with cyclin A or cyclin D1, respectively. Similar results were obtained with cyclin E and CDK2/cyclin E complex. These results suggest that binding of biotinylated p21(149-160) to the cyclin subunit does not disrupt the CDK/cyclin complex. Such a method may be utilised either alone or together with a candidate substance to identify cyclin binding moities and/or inhibitors of cyclin-CDK interaction.

### Example 9: Comparison between peptides, containing ZRXL substrate recognition motif.

Adams et al., (1996) identified a motif - **ZXRL** which is present in many CDK2/Cyclin A (E) substrates -E2F family transcription factors and pRb family proteins; the same motif is present in p21 (N- and C -terminus), p27 and p57 kinase inhibitors (see Fig 2 in Adams et al.). When the substrate recognition motif was mutated in p107 (Rb related protein) or E2F1 their phosphorylation by CDK2-Cyclin A was prevented (Adams et al., 1996).

Our p21 (149-160) SAR data clearly show though that two amino acids outside of ZXRL motif are very important for the kinase inhibitory activity of p21 (C-terminus) derived peptide - A153 (which increases the potency approximately 100 fold) and F159 (which is vial for the kinase inhibition). To evaluate the importance of these flanking the ZXRL motif regions we designed peptides, hybrids between p21 (152-159) and LDL motif (derived from E2F family transcription factors) or LFG motif (derived from p21 N-terminus, p27 and p57 kinase inhibitors), between p21 (16 - 23) and LIF motif (derived from p21 C-terminus) and between p21 (152-159)A153 and LFG motif. Their ability to inhibit CDK2/Cyclin E, CDK2/Cyclin A or CDK4/Cyclin D1 phosphorylation of pRb was compared with the one of the original peptides derived form p21- N and C-terminus, p27, E2F1 and p107 (Table 5).

The main results as presented in Table 5 below, are:
1. All peptides inhibited CDK2-Cyclin and were much less potent toward CDK4/Cyclin D 1 kinase activity.
2. CDK2/Cyclin A and CDK2/Cyclin E were inhibited with similar potency by the 8-mers with the exception of HAKRRLIF and KAURRLIF which were 10 fold more potent toward CDK2/Cyclin A than to CDK2/Cyclin E kinase activity.
3. In the context of eight amino acid peptides alanine substitution of Ser153 led two significant increase of the kinase inhibitory potency of p21 (152-159) - 100, 10 and 4 fold toward CDK2/Cyclin A, CDK2/Cyclin E and CDK4/Cyclin D1 phosphorylation of pRb respectively.
4. The most potent inhibitors of pRb phosphorylation contain Ala on the second position and LIF motif; they are followed by the peptides containing Ala on the second position and LFG motif (with the exception of the p27 derived peptide which contain Gln instead of Arg on the 5^{th} position ), Ser and LFG, and Ser and LIF containing peptides.

The least potent were LDL containing peptides.

These results manifest the importance of Ala and LIF motif for the kinase inhibitory potency of the peptides.

### Competitive binding of peptides, containing different motifs (LIF, LFG, LDL) to Cyclin A or Cyclin D1.

The next important question was if these peptides share the same kinase inhibitory mechanism (bind to the same Cyclin docking site). To answer this question we developed a competitive binding assay where the influence of the 8-mers on Cyclin A (D1) - p21 (149-159) binding was studied (See Materials and Methods for more details).

The results from Cyclin D1 competitive binding assay are summarized on Table 6. For easy comparison, the data for CDK4/Cyclin D1 kinase inhibitory activity of the peptides are given in the same table.

**Table 5: Kinase inhibitory activity of LDL, LIF and LFG containing peptides, derived from E2F, p107, p21 N- and C-terminus and p27.**

| **Peptide** | **Sequence*^{a}*** | **Kinase Inhibition** | | | | | | | | **Competitive binding^{b}** | |
|---|---|---|---|---|---|---|---|---|---|---|---|
| | | **Cyclin** | **A/CDK2** | **Cyclin** | **E/CDK2** | **Cyclin** | **D1/CDK4** | **Cyclin** | **D1/CDK6** | **Cyclin A** | **Cyclin D1** |
| | | **IC₅₀** | **%** | **IC₅₀** | **%** | **IC₅₀** | **%** | **IC₅₀** | **%** | **IC₅₀ (µM)** | **IC₅₀ (µM)** |
| | | **(µM)** | **Inhibition** | **(µM)** | **Inhibition** | **(µM)** | **inhibition** | **(µM)** | **Inhibition** | | |
| p21 *C*-terminus | HSKRRLIF | 3.4 | 80 | 3.4 | 80 | 21 | 72 | n/d | n/d | n/d | 48 |
| p21 *C*-terminus(S153A) | HAKRRLIF | 0.021 | 88 | 0.35 | 81 | 6 | 82 | 5.8 | 100 | 0.3 | 13 |
| p21 *C*-terminus -LFG hybrid | HSKRRLFG | 1.4 | 78 | 1.6 | 82 | n/a | 42 | n/d | n/d | 4.4 | > 200 |
| p21 C-taminus - LDL hybrid | HSKRRLDL | 5.4 | 78 | 39 | 74 | n/a | 24 | n/d | n/d | 5.8 | > 200 |
| p21 *C*-terminus (S153A) LFG | HAKRRLFG | 0.67 | 78 | 0.9 | 82 | 30 | 70 | n/d | n/d | 0.35 | 33 |
| E2F1 | PVKRRLDL | t.2 | 80 | 2.1 | 74 | 99 | 58 | n/d | n/d | 1.2 | > 100 |
| p27 | SAURNLFG | 6.1 | 80 | 2 | 82 | a/a | 46 | n/d | n/d | 3.8 | > 200 |
| p107 | SAKRRLFG | 0.73 | 75 | 0.5 | 86 | 17 | 78 | n/d | n/d | 0.51 | 24 |
| p21 *N*-terminus | KAURRLFG | 0.54 | 80 | 0.074 | 86 | 42 | 66 | n/d | n/d | 0.73 | 134 |
| p21 *N*-terminus-LIF hybrid | KAURRLIF | 0.062 | 70 | 1.2 | 78 | 13 | 83 | n/d | n/d | 0.3 | 20 |

| | | | | | | | | | | | |
|---|---|---|---|---|---|---|---|---|---|---|---|
| ^{a} All peptides were synthesised with free amino termini and as the C-terminal carboxamides ^{b} Using the immobilised p21(149-159) peptide biotinyl-Ahx-Asp-Phe-Tyr-His-Ser-Lys-Arg-Arg-Leu-Ile-Phe-NH | | | | | | | | | | | |

We have demonstrated a very good agreement between the CDK4/Cyclin D1 kinase inhibition and Cyclin D1 competitive binding capabilities of the tested peptides.

The highest potency to inhibit CDK4/Cyclin D1 phosphorylation of pRb and to compete with Biotinylated p21 - (149-159) for binding to Cyclin D1 has HAKRRLIF peptide. These results suggest a mode of kinase inhibition via binding to the cyclin and coincide well with our previous results from 'pull down' experiments showing that the p21 (C-terminus) peptides bind to the Cyclins but not to the CDKs.

Thus, peptides containing the LDL motif (HSKRRLDL and PVKRRLDL) were not able to inhibit CDK4/Cyclin D1 or to compete with Biotin-DFYHSKRRLIF for binding to Cyclin D1. However, peptides, containing LFG motif and Ala on second position were able to inhibit CDK4/Cyclin D1 and to compete with Biotin-DFYHSKRRLIF for binding to Cyclin D1. The only exception of this rule is p27 derived peptide - SAURNLFG, where one of the important Arg residues is replaced with Asn. These results suggest that LFG and LIF peptides bind to the same site of Cyclin D1.

The results for Cyclin A competitive binding and CDK2/Cyclin A kinase inhibition of the peptides, containing LIF, LFG and LDL motifs are also shown in Table 5. There is a very good correlation between the CDK2/Cyclin A inhibition and Cyclin A binding capabilities of the tested peptides. The most potent inhibitor and strongest binding competitor was HAKRRLIF peptide.

### Specificity and selectivity of HAKRRLIF kinase inhibitory activity.

Similarly to p21 (149-160) its derivative p21 (152-159)S153A was not able to inhibit Histone phosphorylation by CDK2/Cyclin A(E) complexes (data not shown). HAKRRLIF was not effective as an inhibitor in CDK1/Cyclin B in vitro kinase assay with Histone or Rb as substrates. HAKRRLIF did not inhibit PKCα induced phosphorylation of Histones.

Thus, we have defined a 8- amino acid peptide derived for p21 (C-terminus) with a single point mutation - S153A which has significantly higher kinase inhibitory activity than the original sequence. HAKRRLIF inhibited most strongly CDK2/Cyclin A phosphorylation of pRb - with IC 50 of 20 nM. The inhibitory activity of the peptide correlates with its ability to bind the cyclin sub-unit. HAKRRLIF is very selective and specific kinase inhibitor - it inhibits specificly only the pRb phosphorylation activity of G1 CDK/Cyclins and does not inhibit the mitotic CDK/Cyclins - CDK1/Cyclin B (or A), or PKC α. HAKRRLIF has much higher specificity and selectivity than the full length p21 protein, which inhibits the Histone phosphorylation of CDK2/Cyclin kinases complexes and has some activity toward CDK1/Cyclin B.

### Example 10: Competitive cyclin A binding of p21- and pRb(866-880)/pRb(870-877) peptides

It has been shown (Adams, P. D.; Li, X.; Sellers, W. R.; Baker, K. B.; Leng, X.; Harper, J. W.; Taya, Y.; Kaelin, W. G. J. Molec. Cell. Biol. 1999,19, 1068-1080) that pRb contains a cyclin-binding motif in its *C*-terminus and that this motif is required for the protein's phosphorylation. To test if the mechanism of kinase inhibition of the p21(152-159)Ser153Ala peptide was indeed *via* competition with pRb for binding to the cyclin subunit, we compared two synthetic pRb-derived peptides - pRb(866-880) and pRb(870-877), as well as the recombinant GST-pRb(772-928) used in our kinase assays (all containing the cyclin-binding motif) with p21-derived peptides for binding to cyclin A. Table 6 shows that all three pRb-derived peptides were able to compete with the p21-derived peptides for binding to cyclin A, and *vice versa.* Interestingly, the longer synthetic peptide pRb(866-880) was less effective than its truncated version pRb(870-877). Probably the conformation of the latter peptide is more favourable for cyclin binding. This peptide contains a *C*-terminal Phe, which case was found considerably to enhance the kinase inhibitory and cyclin-binding activity in the case of the p21-derived peptides.

**Table 6: Competitive cyclin A binding of p21- and pRb(866-880)/pRb(870-877) peptides**

| **Compound** | **Formula** | M*ᵣ* | **MS*** | **RP-HPLC*^{b}*** | | **Competitive cyclin A binding IC₅₀ (µM)** | |
|---|---|---|---|---|---|---|---|
| | | | [M+H]⁺ | *t*ₐ (min) | Purity (%) | immobilised pRb peptide^{a} | immobilised p21 peptide*^{d}* |
| H- His- Ala- Lys-Arg-Arg- Leu- Ile- Phe -NH₂, | | | | | | 0.2 | 0.02 |
| H- Asp- Phe- Tyr- His- Ala- Lys- Arg-Arg- Leu- Ile- Phe -NH₂ | C₇₀H₁₀₅N₂₁O₁₄ | 1464.7 | 1466.0 | 15.8*ⁱ* | > 95 | 0.1 | n/d |
| H- Ser- Asn- Pro-Pro- Lys- Pro- Leu- Lys-Lys- Leu- Arg- Phe- Asp- Ile- Glu -NH₂ | C₈₂H₁₃₇N₂₃O₂₁ | 1781.1 | 1780.0 | 18.1*ⁱⁱ* | > 95 | 35 | 48 |
| H- Lys- Pro- Leu- Lys-Lys- Leu- Arg- Phe -NH₂ | C₃₀H₈₉N₁₅O₂ | 1028.3 | 1026.0 | 17.0*ⁱⁱⁱ* | > 95 | 0.6 | 24 |
| GST -pRb(772-928) | | | | | | n/d | 9 |

| | | | | | | | |
|---|---|---|---|---|---|---|---|
| *^{a}* DEMALDI-TOF MS, +ve mode, α-cyano-4-hydroxycinnamic acid matrix, calibration on authentic H-His-Ala-Lys-Arg-Arg-Leu-Ile-Phe-NH₂ ^{b}Vydac 218TP54, 1 mL/min, 25°C, λ= 214 nm;*ⁱ* 0-40%*ⁱⁱ* 15 - 25% *ⁱⁱⁱ* 10.5 - 20.5 % MeCN in 0.1 % aq CF₃COOH over 20 min *^{c}* Competitive cyclin A binding assay using immobilised biotinyl-Ahx-Lys-Pro-Leu-Lys-Lys-Leu-Arg-Phe-NH₃ *^{d}* Competitive cyclin A binding assay using immobilised biotinyl-Ahx-His-Ala-Arg-Arg-Leu-Ile-Phe-NH₂ *^{e}* Recombinant protein | | | | | | | |

### Example 11: Competitive binding of p21^{WAF1} and H-His-Ala-Lys-Arg-Arg-Leu-Ile-Phe-NH₂ to cyclin A in the presence and absence of CDK2

p21^{WAF1} contains two cyclin-binding sites, one each in its *N-* and *C*-terminus [(p21(19-23) and p21(154-159)], as well as a CDK2-binding site [p21(46-65)]. The cyclin A-binding affinities of full-length p21^{WAF1} and the peptide containing only the C-terminal cyclin-binding motif were compared in the presence and absence of CDK2. This showed (Table 7) that recombinant p21^{WAF1} had *ca.* 27-fold lower affinity than H-His-Ala-Lys-Arg-Arg-Leu-Ile-Phe-NH₂ for cyclin A alone. When cyclin A was pre-complexed with CDK2, on the other hand, the apparent binding affinity of p21^{WAF1} increased and was comparable to that of the octapeptide. The increased ability of p21^{WAF1} to compete with the octapeptide for binding to CDK2-complexed cyclin A is most probably due to the contribution of the CDK-binding motif present in the former. On the other hand, the presence of CDK2 slightly decreased the apparent binding affinity of H-His-Ala-Lys-Arg-Arg-Leu-Ile-Phe-NH₂ for cyclin A, which could be due to some conformational changes of the substrate recognition site on the cyclin sub-unit upon binding of CDK2.

**Table 7: Competitive binding of p21^{WAF1} and H-His-Ala-Lys-Arg-Arg-Leu-Ile-Phe-NH₂ to cyclin A in the presence and absence of CDK2**

| **Protein** | **Test ligand in solution** | **Competitive binding IC₅₀ (nM)*^{a}*** |
|---|---|---|
| Cyclin A | H-His-Ala-Lys-Arg-Arg-Leu-Ile-Phe-NH₂ | 14 |
| Cyclin A | human recombinant p21 ^{WAF1} | 289 |
| Cyclin A / CDK2 complex | H-His-Ala-Lys-Arg-Arg-Leu-Ile-Phe-NH₂ | 28 |
| Cyclin A / CDK2 complex | human recombinant p21 ^{WAF1} | 11 |

| | | |
|---|---|---|
| *^{a}* Competitive binding of cyclin A or cyclin A / CDK2 complex using immobilised peptide biotinyl-Ahx-His-Ala-Lys-Arg-Arg-Leu-Ile-Phe-NH₂ | | |

### Examples 12-22: Structure-activity relationships of the p21(152-159)Ser153Ala peptide (H-His-Ala-Lys-Arg-Arg-Leu-Ile-Phe-NH₂)

For the purposes of the following examples, the reference peptide of the invention has been taken as HAKRRLIF i.e. a preferred peptide of the invention in accordance with the third aspect. As such, the relative activity is expressed against this peptide and all relative activities approaching ( over about 0.7) or greater than unity indicate peptides that may be classified as preferred. The comments provided in these Examples are made with this comparator in mind. It should however be borne in mind that even a peptide having a relative activity or <0.1, remains within the scope of the present invention by virtue of still being active in the context of the invention, such variants are variants upon the first or second embodiments as described above.

### Example 12: Sensitivity to chiral changes

Each residue in turn was substituted by its chiral antipode and the resulting peptide analogues were tested for both CDK2/cyclin A kinase inhibition and competitive cyclin A binding in the presence of immobilised p21(152-159)Ser153Ala peptide. It was found that inversion of configuration at the *C*^{α} atoms was only tolerated (in terms of retention of biological activity) at the peptide's termini. Thus His¹⁵² could be present as either the L- or D-amino acid without loss of potency. Some potency was lost for the corresponding change at position Ala¹⁵³ . Lys¹⁵⁴-Ile¹⁵⁸ could not be substituted by the corresponding D-amino acids without near-complete loss of activity. Some activity was retained when Phe¹⁵⁹ was inverted. These results confirm the highly selective and specific binding mode of the lead peptide. The effects seen for the terminal residues probably reflect the fact that these residues are conformationally more flexible in solution than sequence-internal groups and can be brought into a productive binding mode upon binding.

### Example 12: D-Amino acid substitutions based on p21(152-159)Ser153Ala

| **Compound** | | | | | | | | | | **Formula** | **M*ᵣ*** | **MS^{a}** | **RP-HPLC^{b}** | | **Relative activity** | |
|---|---|---|---|---|---|---|---|---|---|---|---|---|---|---|---|---|
| | | | | | | | | | | | | [M+H]⁺ | H] *^{t}*R(min) | Purity (%) | Kinase Inhibition*^{c}* | Cyclin A Binding*^{d}* |
| H- | His- | Ala- | Lys- | Arg- | Arg- | Leu- | Ile- | Phe | -NN₂ | C₄₈H₈₂N₁₈O₈ | | | | | 1 | 1 |
| H- | *his-* | Ala- | Lys- | Arg- | Arg- | Leu- | Ile- | Phe | -NH₂ | C₄₈H₈₂N₁₈O₈ | 1039.3 | 1039.1 | 15.4 | 96 | 1.7 | 0.9 |
| H- | His*-* | *ala -* | Lys- | Arg- | Arg- | Leu- | Ile- | Phe | -NH₂ | C₄₈H₈₂N₁₈O₈ | 1039.3 | 1042.5 | 15.3 | 98 | 0.3 | 0.6 |
| H- | His- | Ala- | *lys -* | Arg- | Arg- | Leu- | Ile- | Phe | -NH₂ | C₄₈H₈₂N₁₈O₈ | 1039.3 | 1042.9 | 15.6 | 100 | <0.1 | < 0.1 |
| H- | His- | Ala- | Lys- | *arg*- | Arg- | Leu- | Ile- | Phe | -NH₂ | C₄₈H₈₂N₁₈O₈ | 1039.3 | 1041.6 | 15.2 | 99 | <0.1 | < 0.1 |
| H- | His- | Ala- | Lys- | Arg- | *arg*- | Leu- | Ile- | Phe | -NH₂ | C₄₈H₈₂N₁₈O₈ | 1039.3 | 1041.1 | 15.2 | 99 | <0.1 | < 0.1 |
| H- | His- | Ala- | Lys- | Arg- | Arg*-* | *leu-* | Ile- | Phe | -NH₂ | C₄₈H₈₂N₁₈O₈ | 1039.3 | 1041.0 | 17.6 | 100 | <0.1 | < 0.1 |
| H- | His- | Ala- | Lys- | Arg- | Arg- | Leu- | *ile* - | Phe | -NH₂ | C₄₈H₈₂N₁₈O₈ | 1039.3 | 1040.5 | 18.1 | 100 | <0.1 | < 0.1 |
| H- | His- | Ala- | Lys- | Arg- | Arg- | Leu- | Ile- | *phe* | -NH₂ | C₄₈H₈₂N₁₈O₈ | 1039.3 | 1039.7 | 17.1 | 100 | 0.1 | 0.2 |

| | | | | | | | | | | | | | | | | |
|---|---|---|---|---|---|---|---|---|---|---|---|---|---|---|---|---|
| *^{a}*DE MALDI-TOF MS, +ve mode, α-cyano-4-hydroxycinnamic acid matrix, calibration on authentic H-His-Ala-Lys-Arg-Arg-Leu-Ile-Phe-NH₂ *^{b}*Vydac218TP54, 1 mL/min, 25°C, 0 - 40 % MeCN in 0.1 % aq TFA over 20 min, λ = 214 nm *^{c}* CDK2 / cyclin A kinase assay, pRb substrate, [ATP] = 100 µM *^{d}* Competitive cyclin A binding assay using immobilised biotinyl-Ahx-His-Ala-Lys-Arg-Arg-Leu-Ile-Phe-NH₂ | | | | | | | | | | | | | | | | |

### Residue substitutions

### Example 13: His¹⁵²

This residue is comparatively insensitive to substitution. With the exception of Pya, all residue substitutions were either tolerated or even lead to enhanced binding and/or kinase inhibition potency. Furthermore, this residue can be truncated without significant loss in biological activity.

### Example 13: Substitutions of His¹⁵² residue in p21(152-159)Ser153Ala

| **Compound** | | | | | | | | | | **Formula** | **M*ᵣ*** | **MS*^{a}*** | **RP-HPLC*^{b}*** | | **Relative activity** | |
|---|---|---|---|---|---|---|---|---|---|---|---|---|---|---|---|---|
| | | | | | | | | | | | | [M + H]⁺ | *t*_{R} (min) | Purity (%) | Kinase Inhibition*^{c}* | Cyclin A Binding*^{d}* |
| H- | His- | Ala- | Lys- | Arg- | Arg- | Leu- | Ile- | Phe | -NH₂ | | | | | | 1 | 1 |
| H*-* | *Ala-* | Ala- | Lys- | Arg- | Arg- | Leu- | Ile- | Phe | -NH₂ | C₄₅H₈₀N₁₆O₈ | 973.2 | 975.4 | 15.4 | 98 | 1.8 | 2.5 |
| | H- | Ala- | Lys- | Arg- | Arg- | Leu- | Ile- | Phe | -NH₂ | C₄₂H₇₅N₁₅O₇ | 902.1 | 901.0 | 15.5 | 100 | 1 | 0.3 |
| H*-* | *Pya-* | Ala- | Lys- | Arg- | Arg- | Leu- | Ile- | Phe | -NH₂ | C₅₁H₈₄N₁₆O₈ | 1049.3 | 1050.6 | 15.4 | 98 | <0.1 | 0.2 |
| H- | *Thi-* | Ala- | Lys- | Arg- | Arg- | Leu- | Ile- | Phe | -NH₂ | C₄₉H₈₂N₁₆O₈S | 1055.3 | 1055.5 | 16.3 | 100 | 2 | 0.4 |
| H*-* | *Hse-* | Ala- | Lys- | Arg- | Arg- | Leu- | Ile- | Phe | -NH₂ | C₄₆H₈₂N₁₆O₉ | 1003.3 | 1002.9 | 15.7 | 82 | 2 | 2 |
| H*-* | *Phe-* | Ala- | Lys- | Arg- | Arg- | Leu- | Ile- | Phe | -NH₂ | C₅₁H₈₄N₁₆O₈ | 1049.3 | 1052.3 | 16.3 | 100 | 3 | 1 |
| H*-* | *Dab* | Ala- | Lys- | Arg- | Arg- | Leu- | Ile- | Phe | -NH₂ | C₄₆H₈₃N₁₇O₈ | 1002.3 | 1004.7 | 15.5 | 100 | 5 | 0.4 |

| | | | | | | | | | | | | | | | | |
|---|---|---|---|---|---|---|---|---|---|---|---|---|---|---|---|---|
| *^{a}* DE MALDI-TOF MS, +ve mode, α-cyano-4-hydroxycinnamic acid matrix, calibration on authentic H-His-Ala-Lys-Arg-Arg-Leu-Ile-Phc-NH₂ *^{b}* Vydac218TP54, 1 mL/min, 25 °C, 0 - 40 % MeCN in 0.1 % aq TFA over 20 min, λ = 214 nm *^{c}* CDK2 / cyclin A kinase assay, pRb substrate, [ATP] = 100 µM *^{d}* Competitive cyclin A binding assay using immobilised biotinyl-Ahx-His-Ala-Lys-Arg-Arg-Leu-Ile-Phe-NH₂ | | | | | | | | | | | | | | | | |

### Example 14: Ala¹⁵³

This is the residue position where replacement of the native Ser with Ala resulted in a dramatic potency increase. Further potency enhancements are observed when short, straight-chain (Abu) or β-branched (Val, Bug) residues are introduced. Side chains containing more than three saturated carbon atoms in a straight chain are poorly tolerated.

### Examples 14: Substitutions of Ala¹⁵³ residue in p21(152-159)Ser153Ala

| **Compound** | | | | | | | | | | Formula | **M*ᵣ*** | **MS^{a}** | **RP-HPLC^{b}** | | **Relative activity** | |
|---|---|---|---|---|---|---|---|---|---|---|---|---|---|---|---|---|
| | | | | | | | | | | | | [M+H]⁺ | *t*_{R} (min) | Purity (%) | Kinase Inhibition*^{c}* | Cyclin A Binding*^{a}* |
| H- | His- | Ala- | Lys- | Arg- | Arg- | Leu- | Ile- | Phe | -NH₂ | | | | | | 1 | 1 |
| H- | His- | *Gly-* | Lys- | Arg- | Arg- | Leu- | Ile- | Phe | -NH₂ | C₄₇H₈₀N₁₈O₈ | 1025.3 | 1026.8 | 15.2 | 98 | 0.1 | 0.1 |
| H- | His- | *Abu-* | Lys- | Arg- | Arg- | Leu- | Ile- | Phe | -NH₂ | C₄₉H₈₄N₁₈O₈ | 1053.3 | 1055.2 | 15.8 | 100 | 5 | 1.3 |
| H- | His- | *Nva-* | Lys- | Arg- | Arg- | Leu- | Ile- | Phe | -NH₂ | C₅₀M₈₆N₁₈O₈ | 1067.3 | 1069.1 | 16.0 | 100 | <0.1 | <0.1 |
| H- | His- | *Bug*- | Lys- | Arg- | Arg- | Leu- | Ile- | Phe | -NH₂ | C₅₁H₈₈N₁₈O₈ | 1081.4 | 1082.7 | 15.9 | 100 | 0.2 | 1.2 |
| H- | His- | *Val-* | Lys- | Arg- | Arg- | Leu- | Ile- | Phe - | NH₂ | C₅₀H₈₆N₁₈O₈ | 1067.3 | 1068.5 | 15.9 | 100 | 2 | 1.7 |
| H- | His- | *Ile-* | Lys- | Arg- | Arg- | Leu- | Ile- | Phe | -NH₂ | C₅₁H₈₈N₁₈O₈ | 1081.4 | 1081.9 | 16.1 | 100 | 0.5 | 0.2 |
| H- | His- | *Phg-* | Lys- | Arg- | Arg- | Leu- | Ile- | Phe | -NH₂ | C₅₃H₈₄N₁₈O₈ | 1101.4 | 1101.8 | 15.8. 16.1*^{c}* | 100 | <0.1 | <0.1 |
| H- | His- | *Phe*- | Lys- | Arg- | Arg- | Leu- | Ile- | Phe | -NH₂ | C₅₄H₈₆N₁₈O₈ | 1115.4 | 1115.8 | 16.5 | 100 | 0.5 | 0.2 |

| | | | | | | | | | | | | | | | | |
|---|---|---|---|---|---|---|---|---|---|---|---|---|---|---|---|---|
| *^{a}* DE MALDI-TOF MS, +ve mode, α-cyano-4-hydroxycinnamic acid matrix, calibration on authentic H-His-Ala-Lys-Arg-Arg-Leu-Ile-Phe-NH₂ *^{b}* Vydac218TP54, 1 mL/min, 25 °C, 0 - 40 % MeCN in 0.1 % aq TFA over 20 min, λ = 214 nm *^{c}* CDK2 / cyclin A kinase assay, pRb substrate, [ATP] = 100 µM *^{d}* Competitive cyclin A binding assay using immobilised biotinyl-Ahx-His-Ala-Lys-Arg-Arg-Leu-Ile-Phe-NH₂ *^{c}* Mixture of diastereomers (racemic Fmoc-Phg-OH used) | | | | | | | | | | | | | | | | |

### Example 15: Lys¹⁵⁴

Various non-isosteric replacements are tolerated to some extent. A significant potency increase is observed when the conservative Lys-to-Arg replacement is made.

### Example 15: Substitutions of Lys¹⁵⁴ residue in p21(152-159)Ser153Ala

| **Compound** | | | | | | | | | | **Formula** | M, | **MS*^{a}*** | **RP-HPLC*^{b}*** | | **Relative activity** | |
|---|---|---|---|---|---|---|---|---|---|---|---|---|---|---|---|---|
| | | | | | | | | | | | | [M + H]⁺ | *t*_{R} (min) | Purity (%) | Kinase Inhibition*^{c}* | Cyclin A Binding*^{d}* |
| H- | His- | Ala- | *Lys*- | Arg- | Arg- | Leu- | Ile- | Phe | -NH₂ | C₄₈H₈₂N₁₈O₈ | | | | | 1 | 1 |
| H- | His- | Ala- | *Ala-* | Arg- | Arg- | Leu- | Ile- | Phe | -NH₂ | C₄₅H₇₅N₁₇O₈ | 982.2 | 983.6 | 15.6 | 99 | < 0.1 | 0.5 |
| H- | His- | Ala- | *Nle-* | Arg- | Arg- | Leu- | Ile- | Phe | -NH₂ | C₄₈H₈₁N₁₇O₈ | 1024.3 | 1022.9 | 16.8 | 97 | 0.3 | 0.2 |
| H- | His- | Ala- | *Abu-* | Arg- | Arg- | Leu- | Ile- | Phe | -NH₂ | C₄₆H₇₇N₁₇O8 | 996.2 | 997.4 | 16.1 | 100 | 0.8 | 0.2 |
| H- | His- | Ala- | *Leu*- | Arg- | Arg- | Leu- | Ile**-** | Phe | -NH₂ | C₄₈H₈₁N₁₇O₈ | 1024.3 | 1025.5 | 16.8 | 97 | 0.1 | 1.4 |
| H- | His- | Ala- | *Arg*- | Arg- | Arg- | Leu- | Ile- | Phe | -NH₂ | C₄₈H₈₂N₂₀O₈ | 1067.3 | 1067.9 | 15.5 | 94 | 5.7 | 1.5 |

| | | | | | | | | | | | | | | | | |
|---|---|---|---|---|---|---|---|---|---|---|---|---|---|---|---|---|
| *^{a}* DE MALDI-TOF MS, +ve mode, α-cyano-4-hydroxycinnamic acid matrix, calibration on authentic H-His-Ala-Lys-Arg-Arg-Leu-Ile-Phe-NH₂ *^{b}* Vydac218TP54, 1 mL/min, 25 °C, 0 - 40 % MeCN in 0.1 % aq TFA over 20 min, λ = 214 nm *^{c}* CDK2 / cyclin A kinase assay, pRb substrate, [ATP] =100 µM *^{d}* Competitive cyclin A binding assay using immobilised biotinyl-Ahx-His-Ala-Lys-Arg-Arg-Leu-Ile-Phe-NH₂ | | | | | | | | | | | | | | | | |

### Example 16: Arg¹⁵⁵

Only the conservative replacements with Cit and Lys are tolerated to some extent.

### Example 16: Substitutions of Arg¹⁵⁵ residue in p21(152-159)Ser153Ala

| **Compound** | | | | | | | | | | **Formula** | **M*_{F}*** | **MS*^{a}*** | **RP-HPLC*^{b}*** | | **Relative activity** | |
|---|---|---|---|---|---|---|---|---|---|---|---|---|---|---|---|---|
| | | | | | | | | | | | | [M+H]⁺ | *t_{R}* (min) | Purity (%) | Kinase Inhibition*^{c}* | Cyclin A Binding*^{d}* |
| H- | His- | Ala- | Lys- | Arg- | Arg- | Leu- | Ile- | Phe | -NH₂ | C₄₈H₈₂N₁₈O₈ | | | | | 1 | 1 |
| H- | His- | Ala- | Lys- | *Ala*- | Arg- | Leu- | Ile- | Phe | -NH₂ | C₄₅H₇₅N₁₅O₈ | 954.2 | 954.9 | 16.0 | 95 | <0.1 | <0.1 |
| H- | His- | Ala- | Lys- | *Cit*- | Arg- | Leu- | Ile- | Phe | -NH₂ | C₄₈H₈₁N₁₇O₉ | 1040.3 | 1053.5 | 12.5 | 94 | 0.2 | 0.2 |
| H- | His- | Ala- | Lys- | *Hse*- | Arg- | Leu- | Ile- | Phe | -NH₂ | C₄₆H₇₇N₁₅O₉ | 984.2 | 985.9 | 15.8 | 100 | <0.1 | <0.1 |
| H- | His- | Ala- | Lys- | *His*- | Arg- | Leu- | Ile- | Phe | -NH₂ | C₄₈H₇₇N₁₇O₈ | 1020.2 | 1022.1 | 15.4 | 98 | <0.1 | <0.1 |
| H- | His- | Ala- | Lys- | *Nle*- | Arg- | Leu- | Ile- | Phe | -NH₂ | C₄₈H₈₁N₁₅O₈ | 996.3 | 998.4 | 18.1 | 86 | <0.1 | <0.1 |
| H- | His- | Ala- | Lys- | *Gln*- | Arg- | Leu- | Ile- | Phe | -NH₂ | C₄₇H₇₈N₁₆O₉ | 1011.2 | 1012.9 | 15.6 | 98 | <0.1 | <0.1 |
| H- | His- | Ala- | Lys- | *Lys*- | Arg- | Leu- | Ile- | Phe | -NH₂ | C₄₈H₈₂N₁₆O₈ | 1011.3 | 1011.8 | 15.3 | 100 | 0.8 | 0.1 |

| | | | | | | | | | | | | | | | | |
|---|---|---|---|---|---|---|---|---|---|---|---|---|---|---|---|---|
| *^{a}* DE MALDI-TOF MS, +ve mode, α-cyano-4-hydroxycinnamic acid matrix, calibration on authentic H-His-Ala-Lys-Arg-Arg-Leu-Ile-Phe-NH₂ *^{b}* Vydac218TP54, 1 mL/min, 25 °C, 0 - 40 % MeCN in 0.1 % aq TFA over 20 min, λ = 214 nm *^{c}*CDK2 / cyclin A kinase assay, pRb substrate, [ATP] = 100 µM *^{d}* competitive cyclin A binding assay using immobilised biotinyl-Ahx-His-Ala-Lys-Arg-Arg-Leu-Ile-Phe-NH₂ | | | | | | | | | | | | | | | | |

### Example 17: Arg¹⁵⁶

This residue was probed with replacements constraining the backbone dihedral angles in different ways (Ala, Pro, Aib, Sar), none of which were tolerated. Partially tolerated replacements with Cit or Ser indicate involvment in H-bonding.

### Example 17: Substitutions of Arg¹⁵⁶ residue in p21(152-159)Ser153Ala

| **Compound** | | | | | | | | | | **Formula** | **M*ᵣ*** | **MS^{a}** | **RP-HPLC*^{b}*** | | **Relative activity** | |
|---|---|---|---|---|---|---|---|---|---|---|---|---|---|---|---|---|
| | | | | | | | | | | | | [M+H]⁺ | *t*_{R} (min) | Purity (%) | Kinase Inhibition*^{c}* | Cyclin A Binding*^{d}* |
| H- | His- | Ala- | Lys- | Arg- | Arg- | Leu- | Ile- | Phe | -NH₂ | C₄₈H₈₂N₁₈O₈ | | | | | 1 | 1 |
| H- | His- | Ala- | Lys- | Arg- | *Ala*- | Leu- | Ile- | Phe | -NH₂ | C₄₈H₇₅N₁₅O₈ | 954.2 | 954.5 | 16.1 | 100 | <0.1 | < 0.1 |
| H- | His- | Ala- | Lys- | Arg*-* | *Asn-* | Leu- | Ile- | Phe | -NH₂ | C₄₆H₇₆N₁₆O₉ | 997.2 | 997.5 | 15.5 | 99 | <0.1 | < 0.1 |
| H- | His- | Ala- | Lys- | Arg- | *Pro*- | Leu- | Ile- | Phe | -NH₂ | C₄₇H₇₇N₁₅O₈ | 980.2 | 980.1 | 16.3 | 100 | <0.1 | <0.1 |
| H- | His- | Ala- | Lys- | Arg- | *Ser*- | Leu- | Ile- | Phe | -NH₂ | C₄₅H₇₅N₁₅O₉ | 970.2 | 970.2 | 16.1 | 100 | 0.7 | 0.2 |
| H- | His- | Ala- | Lys- | Arg- | *Aib*- | Leu- | Ile- | Phe | -NH₂ | C₄₆H₇₇N₁₅O₈ | 968.2 | 968.1 | 16.7 | 73 | <0.1 | <0.1 |
| H- | His- | Ala- | Lys- | Arg- | *Sar*- | Leu- | Ile- | Phe | -NH₂ | C₄₅H₇₅N₁₅O₈ | 954.2 | 955.4 | 16.5 | 100 | <0.1 | <0.1 |
| H- | His- | Ala- | Lys- | Arg- | *Cit-* | Leu- | Ile- | Phe | -NH₂ | C₄₈H₈₁N₁₇O₉ | 1040.3 | 1041.42 | 15.67 | 100 | 0.3 | n/d |

| | | | | | | | | | | | | | | | | |
|---|---|---|---|---|---|---|---|---|---|---|---|---|---|---|---|---|
| *^{a}* DE MALDI-TOF MS, +ve mode, α-cyano-4-hydroxycinnamic acid matrix, calibration on authentic H-His-Ala-Lys-Arg-Arg-Leu-Ile-Phe-NH₂ *^{b}* Vydac218TP54, 1 mL/min, 25 °C, 0 - 40 % MeCN in 0.1 % aq TFA over 20 min, λ = 214 mm *^{c}* CDK2 / cyclin A kinase assay, pRb substrate, [ATP] = 100 µM *^{d}* Competitive cyclin A binding assay using immobilised biotinyl-Ahx-His-Ala-Lys-Arg-Arg-Leu-Ile-Phe-NH₂ | | | | | | | | | | | | | | | | |

### Example 18: Leu¹⁵⁷

This residue is very sensitive to replacement, even with nearly isosteric groups. Only the very conservative Leu-to-Ile replacement was tolerated somewhat.

### Example 18: Substitutions of Leu¹⁵⁷ residue in p21(152-159)Ser153Ala

| **Compound** | | | | | | | | | | **Formula** | **M*ᵣ*** | **MS*^{a}*** | **RP-HPLC*^{b}*** | | **Relative activity** | |
|---|---|---|---|---|---|---|---|---|---|---|---|---|---|---|---|---|
| | | | | | | | | | | | | [M + H]⁺ | *t*_{R} (min) | Purity (%) | Kinase Inhibition*^{c}* | Cyclin A Binding*^{d}* |
| H- | His- | Ala- | Lys- | Arg- | Arg- | Leu- | Ile- | Phe | -NH₂ | C₄₈H₈₂N₁₈O₈ | | | | | 1 | 1 |
| H- | His- | Ala- | Lys- | Arg- | Arg- | *Ala-* | Ile- | Phe | -NH₂ | C₄₅H₇₆N₁₈O₈ | 997.2 | 996.9 | 13.9 | 100 | <0.1 | <0.1 |
| H- | His- | Ala- | Lys- | Arg- | Arg- | *leu-* | Ile- | Phe | -NH₂ | C₄₈H₈₁N₁O₈ | 10393 | 1041.0 | 15.1 | 100 | <0.1 | 0.1 |
| H- | His- | Ala- | Lys- | Arg- | Arg- | *Ile-* | Ile- | Phe | -NH₂ | C₄₈H₈₂N₁₈O₈ | 1039.3 | 1041.1 | 14.4 | 100 | 1.5 | 0.2 |
| H- | His- | Ala- | Lys- | Arg- | Arg- | *Val-* | Ile- | Phe | -NH₂ | C₄₇H₈₀N₁₈O₈ | 1025.3 | 1026.2 | 15.8 | 100 | <0.1 | <0.1 |
| H- | His- | Ala- | Lys- | Arg- | Arg- | *Nle-* | Ile- | Phe | -NH₂ | C₄₈H₈₂N₁₈O₈ | 1039.3 | 1040.2 | 15.8 | 100 | <0.1 | <0.1 |
| H- | His- | Ala- | Lys- | Arg- | Arg- | *Nva-* | Ile- | Phe | -NH₂ | C₄₇H₈₀N₁₈O₈ | 1025.3 | 1025.0 | 14.9 | 100 | <0.1 | <0.1 |
| H- | His- | Ala- | Lys- | Arg- | Arg- | *Cha*- | Ile- | Phe | -NH₂ | C₅₁H₈₆N₁₈O₈ | 1079.4 | 1079.2 | 17.5 | 100 | <0.1 | <0.1 |
| H- | His- | Ala- | Lys- | Arg- | Arg- | *Phe-* | Ile- | Phe | -NH₂ | C₅₁H₈₀N₁₈O₈ | 1073.3 | 1072.7 | 16.4 | 100 | <0.1 | <0.1 |
| H- | His- | Ala- | Lys- | Arg- | Arg- | *INap-* | Ile- | Phe | -NH₂ | C₅₂H₈₂N₁₈O₈ | 1123.4 | 1122.5 | 17.9 | 100 | <0.1 | <0.1 |

| | | | | | | | | | | | | | | | | |
|---|---|---|---|---|---|---|---|---|---|---|---|---|---|---|---|---|
| *^{a}* DE MALDI-TOF MS, +ve mode, α-cyano-4-hydroxycinnamic acid matrix, calibration on authentic H-His-Ala-Lys-Arg-Arg-Leu-Ile-PhL,NH, *^{b}* Vydac218TP54,1 mL/min, 25 °C, 0 - 40 % MeCN in 0.1 % aq TFA over 20 min, λ = 214 nm *^{c}* CDK2 / cyclin A kinase assay, pRb substrate, [ATP] = 100 µM *^{d}* Competitive cyclin A binding assay using immobilised biotinyl-Ahx-His-Ala-Lys-Arg-Arg-Leu-Ile-Phe-NH₂ | | | | | | | | | | | | | | | | |

### Example 19: Ile¹⁵⁸

All substitutions with aliphatic and aromatic residues were tolerated to some extent. However, excision of the Ile residue abolished activity. These results indicate that this residue is not crucial for activity but may be important as a spacer group between the flanking Leu and Phe groups.

### Example 19: Substitutions of Ile¹⁵⁸ residue in p21(152-159)Ser153Ala

| **Compound** | | | | | | | | | | **Formula** | **M*ᵣ*** | **MS*^{a}*** | **RP-HPLC*^{b}*** | | **Relative activity** | |
|---|---|---|---|---|---|---|---|---|---|---|---|---|---|---|---|---|
| | | | | | | | | | | | | [M + H]⁺ | *t*_{R} (min) | Purity (%) | Kinase Inhibition*^{c}* | Cyclin A Binding*^{d}* |
| H- | His- | Ala- | Lys- | Arg- | Arg- | Leu- | Ile- | Phe | -NH₂ | C₄₈H₈₂N₁₈O₈ | | | | | 1 | 1 |
| H- | His- | Ala- | Lys- | Arg- | Arg- | Leu- | *Ala-* | Phe | -NH₂ | C₄₅H₇₆N₁₈O₈ | 997.2 | 996.5 | 13.8 | 100 | 0.3 | 0.8 |
| H- | His- | Ala- | Lys- | Arg- | Arg- | Leu- | *Leu-* | Phe | -NH₂ | C₄₈H₈₂N₁₈O₈ | 1039.3 | 1038.4 | 16.1 | 100 | 1.2 | 0.6 |
| H- | His- | Ala- | Lys- | Arg- | Arg- | Leu- | *Val-* | Phe | -NH₂ | C₄₇H₈₀N₁₈O₈ | 1025.3 | 1024.7 | 14.9 | 100 | 0.8 | 1.5 |
| H- | His- | Ala- | Lys- | Arg- | Arg- | Leu- | *Nle-* | Phe | -NH₂ | C₄₈H₈₂H₁₈O₈ | 1039.3 | 1040.3 | 16.3 | 100 | 0.4 | 0.3 |
| H- | His- | Ala- | Lys- | Arg- | Arg- | Leu- | *Nva-* | Phe | -NH₂ | C₄₇H₈₀N₁₈O₈ | 1025.3 | 1025.7 | 15.2 | 100 | 0.2 | 0.6 |
| H- | His- | Ala- | Lys- | Arg- | Arg- | Leu- | *Cha*- | Phe | -NH₂ | C₅₁H₈₆N₁₈O₈ | 1079.4 | 1080.2 | 18.4 | 100 | 0.3 | 0.5 |
| H- | His- | Ala- | Lys- | Arg- | Arg- | Leu- | *Phe-* | Phe | -NH₂ | C₅₁H₈₀N₁₈O₈ | 1073.3 | 1073.9 | 16.3 | 100 | 0.4 | 0.4 |
| H- | His- | Ala- | Lys- | Arg- | Arg- | Leu- | *INap-* | Phe | -NH₂ | C₅₅H₈₂N₁₈O₈ | 1123.4 | 1122.9 | 18.2 | 100 | 0.5 | 0.5 |
| H- | His- | Ala- | Lys- | Arg- | Arg- | Leu- | Phe | -NH₂ | | C₄₂H₇₁N₁₇O₇ | 926.1 | 924.8 | 13.8 | 100 | < 0.1 | < 0.1 |

| | | | | | | | | | | | | | | | | |
|---|---|---|---|---|---|---|---|---|---|---|---|---|---|---|---|---|
| *^{a}*DE MALDI-TOF MS, +ve mode, α-cyano-4-hydroxycinnamic acid matrix, calibration on authentic H-His-Ala-Lys-Arg-Arg-Leu-Ile-Phe-NH₂ *^{b}* Vydac218TP54, 1 mL/min, 25 °C, 0 - 40 % MeCN in 0.1 % aq TFA over 20 min, λ = 214 nm *^{c}* CDK2 / cyclin A kinase assay, pRb substrate, [ATP] = 100 µM *^{d}* Competitive cyclin A binding assay using immobilised biotinyl-Ahx-His-Ala-Lys-Arg-Arg-Leu-Ile-Phe-NH₂ | | | | | | | | | | | | | | | | |

### Example 20: Phe¹⁵⁹

Only certain replacements with aromatic residues were tolerated. Notably pFPhe substitution resulted in an analogue with enhanced cyclin A-binding affinity.

### Example 20: Substitutions of Phe¹⁵⁹ residue in p21(152-159)Ser153Ala

| **Compound** | | | | | | | | | | **Formula** | **M*ᵣ*** | **MS*^{a}*** | **RP-HPLC*^{b}*** | | **Relative activity** | |
|---|---|---|---|---|---|---|---|---|---|---|---|---|---|---|---|---|
| | | | | | | | | | | | | [M+H]⁺ | *t_{R} t_{R}* (min) | Purity (%) | Kinase Inhibition*^{c}* | Cyclin A Binding*^{d}* |
| H- | His- | Ala- | Lys- | Arg- | Arg- | Leu- | Ile- | Phe | -NH₂ | C₄₈H₈₂N₁₈O₈ | | | | | 1 | 1 |
| H- | His- | Ala- | Lys- | Arg- | Arg- | Leu- | Ile- | *Leu* | -NH₂ | C₄₅H₈₄N₁₈O₈ | 1005.3 | 1005.7 | 14.2 | 97 | 0.3 | < 0.1 |
| H- | His- | Ala- | Lys- | Arg- | Arg- | Leu- | Ile- | *Cha* | -NH₂ | C₄₈H₈₃N₁₈O₈ | 1045.3 | 1045.5 | 16.9 | 100 | < 0.1 | 0.1 |
| H- | His- | Ala- | Lys- | Arg- | Arg- | Leu- | Ile- | *Hof* | -NH₂ | C₄₉H₈₄N₁₈O₈ | 1053.3 | 1052.8 | 15.8 | 96 | < 0.1 | < 0.1 |
| H- | His- | Ala- | Lys- | Arg- | Arg- | Leu- | Ile- | *Tyr* | -NH₂ | C₄₅H₈₂N₁₈O₉ | 1055.3 | 1054.6 | 13.3 | 100 | 0.3 | 0.2 |
| H- | His- | Ala- | Lys- | Arg- | Arg- | Leu- | Ile- | *pFPhe* | -NH₂ | C₄₈H₈₁N₁₈O₈ | 1057.3 | 1055.8 | 16.0 | 100 | 1 | 5 |
| H- | His- | Ala- | Lys- | Arg- | Arg- | Leu- | Ile- | *mFPhe* | -NH₂ | C₄₈H₈₁N₁₈O₈ | 1057.3 | 1055.5 | 16.2 | 100 | 0.8 | 0.8 |
| H- | His- | Ala- | Lys- | Arg- | Arg- | Leu- | Ile- | *Trp* | -NH₂ | C₅₀H₈₃N₁₈O₈ | 1078.3 | 1076.1 | 15.6 | 98 | 0.3 | 0.1 |
| H- | His- | Ala- | Lys- | Arg- | Arg- | Leu- | Ile- | *1Nap* | -NH₂ | C₅₂H₈₄N₁₈O₈ | 1089.3 | 1090.7 | 17.8 | 100 | 0.2 | < 0.1 |
| H- | His- | Ala- | Lys- | Arg- | Arg- | Leu- | Ile- | *2Nap* | -NH₂ | C₅₂H₈₄N₁₈O₈ | 1089.3 | 1090.6 | 18.0 | 100 | 1.2 | 0.7 |
| H- | His- | Ala- | Lys- | Arg- | Arg- | Leu- | Ile- | *Lys* | -NH₂ | C₄₅H₈₅N₁₉O₈ | 1020.3 | 1021.5 | 11.6 | 66 | < 0.1 | < 0.1 |
| H- | His- | Ala- | Lys- | Arg- | Arg- | Leu- | Ile- | *Tic* | -NH₂ | C₄₉H₈₂N₁₈O₈ | 1051.3 | 1052.3 | 15.6 | 91 | 0.3 | < 0.1 |

| | | | | | | | | | | | | | | | | |
|---|---|---|---|---|---|---|---|---|---|---|---|---|---|---|---|---|
| *^{a}* DE MALDI-TOF MS, +ve mode, α-cyano-4-hydroxycinnamic acid matrix, calibration on authentic H-His-Ala-Lys-Arg-Arg-Leu-Ile-Phe-NH₂ *^{b}* Vydac218TP54, 1 mL/min, 25 °C. 0 - 40 % MeCN in 0.1 % aq TFA over 20 min, λ = 214 nm *^{c}* CDK2 / cyclin A kinase assay, pRb substrate, [ATP] = 100 µM *^{d}* Competitive cyclin A binding assay using immobilised biotinyl-Ahx-His-Ala-Lys-Arg-Arg-Leu-Ile-Phe-NH₂ | | | | | | | | | | | | | | | | |

### Example 21: Substitutions of Phe¹⁵⁹ residue in p21(152-159)Ser153Ala with conformationally defined residues

### Fmoc-DL-threo-Pse-OH

To a solution of H-DL-*threo*-Pse-OH (1 g, 5.5 mmol) in 5 % aq Na₂CO₃ (13 mL, 6 mmol), was added a solution of Fmoc-ONSu (1.7 g, 5 mmol) in THF (13 mL) over a period of 30 min. The mixture was stirred vigorously for 5 h. The solvent was evaporated to dryness *in vacuo.* The residual white solid was dissolved in H₂O (150 mL) and was washed with Et₂O (2 × 100 mL). The aqueous phase was acidified to pH 2 with 0.2 M aq HCl and a precipitate was obtained, which was extracted into EtOAc (2 × 100 mL). The combined extracts were washed with aq KHSO₄ and brine, dried (MgSO₄) and concentrated *in vacuo* to afford a crude product (1.32 g, 65 %). This was dissolved in the minimum volume of EtOAc and dripped into vigorously stirred hexane to afford, after filtration and drying, the title compound (1.27 g, 63 %). M.p. 107 - 108°C. TLC (EtOAc/AcOH, 99:1): *R_{f}*= 0.27. RP-HPLC (Vydac 218TP54, 1 mL/min, 50 - 100 % MeCN in 0.1 % aq CF₃COOH over 20 min): *t*_{R} = 7.2 min. ¹H-NMR (CDCl₃, 250 MHz), δ 7.75 (2H, d, *J* = 7.6 Hz, Fmoc aromatic *H*), 7.42-7.49 (2h, M, Fmoc aromatic *H*), 7.27-7.39 (9H, m, aromatic *H*), 5.67 (1H, d, *J* = 9.0 Hz, N*H*), 5.45 (1H, d, *J* = 2.4 Hz, C^{β}*H*), 4.68 (1H, dd, *J* = 2.5, 8.8 Hz, C^{α}*H*), 4.27 (2H, m, Fmoc C*H*₂), 4.14 (1H, t, *J*= 7.1 Hz, Fmoc C*H*); ¹³C-NMR (CDCl₃:*d*₆-DMSO, 62.9 MHz) δ: 172.28 (carbonyl *C*, acid), 155.98 (carbonyl *C*, urethane), 143.60, 143.54, 140.80 (quaternary *C*). 127.81, 127.28, 127.16, 126.71, 125.73, 124.99, 124.88, 119.53, 72.68 (*C*H), 66.45 (*C*H₂), 59.62, 46.69 (*C*H). HR-MS (FAB) calc. For C₂₄H₂₂NO₅ (MH⁺): 404.149798, found 404.148369.

### Ac-DL-threo-Pse-OH

To a cold solution (5 °C) of H-DL-*threo*-Pse-OH (1 g, 5.5 mmol) and NaHCO₃ (1.85 g, 22.1 mmol) in H₂O (30 mL) was added Ac₂O (1.6 mL, 16.6 mmol) dropwise over a period of 15 min. The mixture was stirred vigorously at room temperature overnight. It was extracted with EtOAc (100 mL). The aqueous phase was acidified to pH 2 with aq KHSO₄ and the product was extracted into EtOAc (3 × 100 mL), and NaCl was added to aid the process. The organic extracts were combined and washed with aq KHSO₄, brine, dried (MgSO₄) and concentrated *in vacuo* to afford the title compound as a white solid (0.9 g, 73 %). M.p. 142-143 °C; ES-MS⁺ *m*/*z* 224.2 (MH⁺), calc. 224.2; TLC (MeOH/CH₂Cl₂/AcOH, 20:79:1): *R_{f}*= 0.41; RP-HPLC (Vydac 218TP54, 1 mL/min, 20 - 60 % MeCN in 0.1 % aq CF₃COOH over 25 min): *t*_{R} = 3.6 min. ¹H-NMR (*d*₆-DMSO, 250 MHz) δ. 12.49-12.60 (1H, br. S, CO₂*H*), 7.99 (1H, d, *J* = 9.1 Hz, N*H*), 7.07-7.39 (5H, m, Ar*H*), 5.76-5.90 (1H, br. S, O*H*), 5.14 (1H, d, *J =* 2.9 Hz, C^{β}*H*), 4.48 (1H, dd, *J =* 3.0, 9.1 Hz, C^{α}*H*), 1.75 (3H, s, C*H*₃).

### H-DL-threo-Pse-OMe.HCl

A stream of HCl gas was passed through a stirred suspension of H-DL-*threo*-Pse-OH (1 g, 5.5 mmol) in MeOH (30 mL) at 0°C. After *ca.* 30 min, dissolution was complete. Gas addition was continued for 2 h. The mixture was allowed to reach room temeperature, sealed, and left to stand overnight. Solvent was removed *in vacuo* to afford the title compound as an off-white solid (1.07 g, 83 %). M.p. 154-156 °C (dec.); ES-MS⁺ *m*/*z* 195.9 (MH⁺), calc. 196.2; RP-HPLC (Vydac 218TP54, 1 mL/min, 20 - 60 % MeCN in 0.1 % aq CF₃COOH over 25 min): *t_{R}* = 3.2 min; ¹H-NMR (d₆-DMSO, 250 MHz) δ 8.54, (3H, br. S, N*H*₃⁺), 7.29-7.40 (5H, m, Ar*H*), 5.03 (1H, d, *J =* 5.6 Hz, C^{β}*H*), 4.16 (1H, m, C^{α}*H*), 3.61 (3H, s, C*H*₃).

### Ac-DL-threo-Pse-OMe

To a vigorously stirred solution of H-DL-*threo*-Pse-OMe.HCl (0.5 g. 2.15 mmol) and NaOAc.(H₂O)₃ (1.17 g, 8.6 mmol) in H₂O (10 mL) at 5 °C was added Ac₂O (0.6 mL, 6.45 mmol) dropwise over 15 min. A white precipitate was formed within 10 min, and stirring was continued for 16 h at room temperature. The mixture was extracted with EtOAc (2 × 100 mL), and the organic phase was separated and washed with aq NaHCO₃ (2 × 50 mL) and brine (100 mL). The organic phase was dried (MgSO₄), and evaporated to dryness *in vacuo* to afford th title compound as a white solid (0.36 g, 71 %). M.p. 176-179 °C; ES-MS⁺ *m*/*z* 238.1 (MH⁺), calcd. 238.2; TLC (MeOH/CH₂Cl₂, 1:5): *R_{f}* = 0.69; RP-HPLC (Vydac 218TP54, 1 mL/min, 20 - 50 % MeCN in 0.1 % aq CF₃COOH over 25 min): *t_{R}* = 5.2 min. ¹H-NMR (*d*₆-DMSO, 250 MHz) δ. 8.20 (1H, d, *J* = 8.8 Hz, N*H*), 7.20-7.39 (5H, m, Ar*H*), 5.88 (1H, d, *J =* 4.6 Hz, O*H*), 5.09 (1H, m, C^{β}*H*), 4.54 (1H, dd, *J =* 3,7 8.8 Hz, C^{α}*H*), 3.61 (3H, s, CO₂C*H*₃), 1.76 (3H, s, NHCOC*H*₃).

### Ac-L-threo-Pse-OH

To a suspension of Ac-DL-*threo*-Pse-OMe (100 mg, 0.42 mmol) in 0.05 M aq potassium phosphate buffer (14 mL) was added α-chymotrypsin (10 mg, 400 units). The pH was maintained at its initial value (pH 7 - 8) by the manual addition of 0.5 M phosphate buffer. The mixture was stirred vigorously overnight. It was extracted with EtOAc (3 × 50 mL) to remove Ac-D-*threo*-Pse-OMe. The aqueous phase was separated, acidified to pH 2 with 2 M aq HCl and extracted into EtOAc (3 × 100 mL). The organic extract was washed with brine, dried (MgSO₄), and evaporated to dryness *in vacuo* to afford a colourless oil (25 mg, 53 %). The title compound was obtained as a white solid after lyophilisation from H₂O. M.p. 160-163; [α]_{D}²⁶ +25.1° (*c* = 1.0, AcOH); ES-MS⁺ *m*/*z* 224.1 (MH⁺), calcd. 224.2; RP-HPLC (Vydac 218TP54, 1 mL/min, 20 - 60 % MeCN in 0.1 % aq CF₃COOH over 25 min): *t_{R}* = 3.6 min; ¹H-NMR (*d*₆-DMSO, 250 MHz) δ 7.99 (1H, D, *J*= 9.1 Hz, N*H*), 7.18-7.39 (5H, m, Ar*H*), 5.14 (1H, d, *J* = 3.0 Hz, C^{β}*H*), 4.48 (1H, dd, *J* = 3.0, 9.1 Hz, C^{α}*H*), 1.74 (3H, s, C*H*₃).

### Ac-D-threo-Pse-OMe

From the above reaction, the initial EtOAc extract (150 mL) was washed with aq NaHCO₃ (2 × 50 mL) and brine (2 × 50 mL), dried and evaporated to dryness *in vacuo* to afford the title compound as a white solid (48 mg, 96 %). RP-HPLC (Vydac 218TP54, 1 mL/min, 20 - 60 % MeCN in 0.1 % aq CF₃COOH over 25 min): *t*_{R} = 5.2 min).

### Fmoc-L-threo-Pse-OH

A solution of Ac-L-*threo*-Pse-OH (40 mg, 0.18 mmol) in 6 M aqueous HCl (5 mL) was refluxed at 100°C for 5 h. The solution was allowed to attain room temperature and the solvent was removed *in vacuo.* The residue was then dissolved in H₂O and lyophilised to afford H-L-*threo*-Pse-OH.HCl as a white foam. To this was added a solution of 5 % aq Na₂CO₃ (0.5 mL, 0.22 mmol). Effervescence occurred, and the solution was adjusted to pH 9 using a further equivalent of 5 % aq Na₂CO₃ (0.5 mL). A solution of Fmoc-ONSu (60 mg, 0.18 mmol) in THF (1 mL) was then added over a period of 10 min. The mixture was stirred vigorously at room temperature for a further 5 h. The solvent was evaporated *in vacuo,* and the resulting white solid was dissolved in H₂O (100 mL) and washed with diethyl ether (2 × 50 ml). The aqueous extract was acidified to pH 2 with 2 M aq HCl and a precipitate was obtained, which was extracted into EtOAc (3 × 60 ml). The organic extract was washed with aq KHSO₄ (100 mL) and brine (100 mL), dried (MgSO₄) and concentrated *in vacuo* to afford a crude product as a yellow oil (84 mg, quantitative). This was dissolved in the minimum volume of EtOAc and dripped into vigorously stirred hexane (120 mL) to afford, after filtration and drying, the title compound (47 mg, 65 %) as a white crystalline solid. M.p. 127-129 °C; [α]_{D}²² +27.9° (*c* = 1.0, MeOH); ES-MS⁺ *m*/*z* 404.3 (MH⁺), calcd. 404.4; TLC (EtOAc/AcOH, 99:1); *R_{f}* = 0.27; RP-HPLC (Vydac 218TP54, 1 mL/min, 50 - 100 % MeCN in 0.1 % aq CF₃COOH over 20 min): *t_{R}* 7.2 min; ¹H-NMR (*d*₆-DMSO, 250 MHz) δ: 7.88 (2H, d, *J* = 7.5 Hz, Fmoc Ar*H*), 7.67 (1H, d, *J*= 7.2 Hz, Fmoc Ar*H*), 7.63 (1H, d, *J* = 7.5 Hz, Fmoc Ar*H*), 7.31-7.43 (9H, m, Ar*H*), 5.80 (1H, br. s, O*H*), 5.16 (1H, d, *J* 3.3 Hz, C^{β}*H*), 4.29 (1H, dd, *J* 3.3, 9.4 Hz, C^{α}*H*), 4.01-4.16 (3H, m, Fmoc C*H*, C*H*₂). HR-MS (FAB) calcd, for C₂₄H₂₂NO₅ (MH⁺): 404.149798, found: 404.149850.

### Fmoc-D-threo-Pse-OH

A solution of Ac-D-*threo*-Pse-OMe (150 mg, 0.63 mmol) in 6 M aq HCl (10 mL) was refluxed at 100°C for 5 h. The solution was allowed to attain room temperature and the solvent removed *in vacuo.* The residual material was dissolved in H₂O and lyophilised to afford H-D-*threo*-Pse-OH.HCl as a pale yellow solid, which was immediately carried forward to the next step. In a similar manner to that described for the preparation of Fmoc-L-*threo*-Pse-OH, the crude title product was obtained as a yellow oil (229 mg, 90 %). The crude product was dissolved in the minimum volume of EtOAc and dripped into vigorously stirred hexane (120 mL) to afford the title compound (174 mg, 68 % overall) as an off-white crystalline solid. M.p.: 127-129 °C; [α]_{D}²² -29.0° (*c* = 1.0, methanol); APcI-MS⁺ *m*/*z* 404.0 (MH⁺), calcd. 404.4; TLC (EtOAc/AcOH, 99:1); *R_{f}* = 0.27; RP-HPLC (Vydac218TP54, 1 ml/min, 50 - 100 % MeCN in 0.1 % aq CF₃COOH over 20 min): *t_{R}* = 7.2 min; ¹H-NMR (*d*₆-DMSO, 250 MHz) δ: 7.88 (2H, d, *J* = 7.2 Hz, Fmoc Ar*H*), 7.68 (1H, d, *J* = 7.2 Hz, Fmoc Ar*H*), 7.63 (1H, d, *J* = 7.5 Hz, Fmoc Ar*H*), 7.22-7.41 (9H, m, Ar*H*), 5.17 (1H, d, *J =* 3.2 Hz, C^{β}*H*), 4.30 (1H, dd, *J =* 3.3, 9.5 Hz, C^{α}*H*), 4.01-4.15 (3H, m, Fmoc C*H*, C*H*₂). HR-MS (FAB) calcd. for C₂₄H₂₂NO₅ (MH⁺): 404.149798, found: 404.149722.

### Addition of Fmoc-protected amino acids to 5-[4-(4-tolyl(chloro)methyl)phenoxy]pentanoyl amino-methylated polystyrene

5-[4-(4-Tolyl(chloro)methyl)phenoxy]pentanoyl aminomethylated polystyrene (0.064 mmol, theoretical loading 0.64 mmol g⁻¹; Atkinson, G. E.; Fischer, P. M.; Chan, W. C. J. Org. Chem. 2000, 65, 5048-5056) and Fmoc-protected amino acid (0.192 mmol) were suspended in CH₂Cl₂ (2 mL). Following the addition of Pr₂ⁱNEt (0.128 mmol), the resultant mixture was stirred gently at room temperature for 24 h. The resin was filtered, washed successively with DMF, CH₂Cl₂ and MeOH, and dried *in vacuo.*

### Addition of Fmoc-amino alcohols to 5-[4-(4-tolyl(chloro)methyl)-phenoxy]pentanoyl aminomethylated polystyrene

To a mixture of 5-[4-(4-tolyl(chloro)methyl)phenoxy]pentanoyl aminomethylated polystyrene (0.06 mmol, theoretical loading 0.64 mmol g⁻¹; ) and Fmoc-amino alcohol (0.19 mmol) in ClCH₂CH₂Cl (3 mL) and THF (1 mL) was added Pr₂ⁱNEt (0.10 mmol). The suspension was then gently agitated at room temperature for 48-72 h. The resin was filtered, washed successively with DMF, CH₂Cl₂ and MeOH, and dried *in vacuo.*

### Synthesis of peptides

Amino acyl or peptidyl resin (0.026 mmol) was placed in a reaction column, swollen in DMF for 18 h, and Fmoc-deprotected using 20 % piperidine in DMF. The resin was then washed with DMF (10 min, 2.5 mL/min), and the sequence Boc-His(Trt)-Ala/Ser(Bu^{t})-Lys(Boc)-Arg(Pbf)-Arg(Pbf)-Leu-Ile was assembled using an automated PepSynthesizer 9050 (MilliGen). Sequential acylation reactions were carried out at ambient temperature for 2 h using appropriate Fmoc-protected amino acids [Fmoc-Ile-OH, 141 mg; Fmoc-Leu-OH, 141 mg; Fmoc-Arg(Pbf)-OH, 260 mg; Fmoc-Lys(Boc)-OH, 187 mg; Fmoc-Ala-OH, 125 mg; Fmoc-Ser(t-Bu)-OH, 153 mg, Fmoc-His(Trt)-OH, 248 mg; 0.40 mmol] and carboxyl-activated using TBTU (128 mg, 0.40 mmol), HOBt (31 mg, 0.20 mmol) and Pr₂ⁱNEt (1.31 mL, 10 % in DMF). Repetitive Fmoc-deprotection was achieved using 20 % piperidine in DMF (6 min, 2.5 mL/min). After the final Fmoc-deprotection, the terminal amine group was Boc-protected with di-*tert*-butyl dicarbonate (87 mg, 0.40 mmol). The assembled *N*-Boc-protected peptidyl-resin was filtered, washed successively with DMF, CH₂Cl₂ and MeOH, and dried *in vacuo.* The resin product was suspended in a mixture of Pr₃ⁱSiH (0.1 mL) and H₂O (0.4 mL), followed by the addition of CF₃COOH (4.5 mL). The reaction mixture was gently stirred at room temperature for 2 h. The suspension was filtered, washed with CF₃COOH (5 mL) and the filtrate evaporated to dryness *in vacuo.* The residual material was triturated with Et₂O (15 mL) to yield a white solid. The desired synthetic peptide was lyophilised from H₂O overnight, and purified by preparative RP-HPLC.

### Dehydration reaction of peptides

The *N*-Boc-protected peptidyl-resin containing a *C*-terminal Pse residue (0.02 mmol) was swelled in CH₂Cl₂ (0.5 mL) and THF (0.5 mL) in a 2-necked round-bottomed flask for 1 h under N₂. The solution was cooled to -78 °C, and Et₃N (84 µL, 0.60 mmol) followed by SOCl₂ (10 µL, 0.08 mmol) were carefully added to the resin suspension. The mixture was stirred at -78°C for 3.5 h, after which a further quantity of SOCl₂ (10 µL, 0.08 mmol) was added, and the stirred mixture was gradually warmed to -10°C over a period of 2.5 h. The mixture was then stirred at 5 °C overnight. The resin was filtered, washed successively with DMF, CH₂Cl₂ and MeOH, and dried *in vacuo.* The resin product was suspended in a mixture of ethyl methyl sulfide (0.1 mL), Pr3ⁱSiH (0.1 mL) and H₂O (0.4 mL), followed by the addition of CF₃COOH (4.4 mL). The suspension was gently stirred at ambient temperature for 2 h. The suspension was filtered, washed with CF₃COOH (5 mL) and the filtrate evaporated to dryness *in vacuo.* The residual material was then triturated with Et₂O to yield a yellow solid, which was lyophilised from water (5-10 mL) overnight and purified by preparative RP-HPLC.

### Psa-containing peptides

A portion of the corresponding Pse-containing peptidyl resin (50 mg, 0.015 mmol, theoretical loading 0.293 mmol/g) was suspended in DMF (1 mL) and treated with Ac₂O (14 µL, 0.15 mmol), Pr₂ⁱNEt (5 µL, 0.02 mmol) and 4-(*N*,*N*-dimethylamino)pyridine (0.18 mg, 0.0015 mmol). The mixture was gently stirred at room temperature for 24 h. The resin was filtered, washed successively with DMF, CH₂Cl₂ and MeOH, and *dried in vacuo.* The resin product (50 mg) upon acidolytic treatment gave the crude product. Pure peptides were obtained after purification by preparative RP-HPLC.

| **Compound** | | | | | | | | | | **Formula** | **M*ᵣ*** | **MS*^{a}*** | **RP-HPLC*^{b}*** | | **Relative activity** | |
|---|---|---|---|---|---|---|---|---|---|---|---|---|---|---|---|---|
| | | | | | | | | | | | | [M+H]⁺ | *t_{R}* (min) | Purity (%) | Kinase Inhibition*^{c}* | Cyclin A Binding*^{d}* |
| H- | His- | Ala- | Lys- | Arg- | Arg- | Leu- | Ile- | Phe | -NH₂ | C₄₈H₈₂N₁₈O₈ | | | | | 1 | 1 |
| H- | His | Ala | Lys | Arg | Arg | Leu | Ile | *L-Pse* | OH | C₄₈H₈₂N₁₈O₉ | 1055.9 | 1055.7 | 8.8 | 99 | < 0.1 | 0.2 |
| H- | His | Ala | Lys | Arg | Arg | Leu | Ile | *D-Pse* | OH | C₄₈H₈₂N₁₈O₈ | 1055.9 | 1056.0 | 6.8 | 99 | < 0.1 | < 0.1 |
| H- | His | *Ser* | Lys | Arg | Arg | Leu | Ile | *L-Pse* | OH | C₄₈H₈₂N₁₈O₁₀ | 1071.3 | 1074.1 | 8.8 | 99 | < 0.1 | < 0.1 |
| H- | His | *Ser* | Lys | Arg | Arg | Leu | Ile | *D-Pse* | OH | C₄₈H₈₂N₁₈O₁₀ | 1071.3 | 1073.0 | 6.8 | 99 | < 0.1 | < 0.1 |
| H- | His | Ala | Lys | Arg | Arg | Leu | Ile | *L-Psa* | OH | C₅₀H₈₄N₁₈O₁₀ | 1097.3 | 1098.0 | 11.2 | 99 | 22 | n/d |
| H- | His | Ala | Lys | Arg | Arg | Leu | Ile | *D-Psa* | OH | C₅₀H₈₄N₁₈O₁₀ | 1097.3 | 1098.0 | 8.4 | 99 | < 0.1 | n/d |
| H- | His | *Ser* | Lys | Arg | Arg | Leu | Ile | *L*-*Psa* | OH | C₅₀H₈₄N₁₈O₁₁ | 1113.3 | 1114.9 | 10.8 | 99 | < 0.1 | n/d |
| H- | His | *Ser* | Lys | Arg | Arg | Leu | Ile | *D-Psa* | OH | C₅₀H₈₄N₁₈O₁₁ | 1113.3 | 1114.4 | 8 | 99 | < 0.1 | n/d |
| H- | His | Ala | Lys | Arg | Arg | Leu | Ile | *Dhp* | OH | C₄₈H₈₀N₁₈O₈ | 1037.3 | 1038.4 | 8.8 | 99 | 3.3 | 0.2 |
| H- | His | *Ser* | Lys | Arg | Arg | Leu | Ile | *Dhp* | OH | C₄₈H₈₀N₁₈O₉ | 1053.3 | 1054.6 | 8.8 | 99 | 0.4 | n/d |
| H- | His | Ala | Lys | Arg | Arg | Leu | Ile | *Pheol* | | C₄₈H₈₃N₁₇O₈ | 1026.3 | 1026.2 | 8.4 | 99 | 0.6 | 1.0 |
| H- | His | *Ser* | Lys | Arg | Arg | Leu | Ile | *Pheol* | | C₄₈H₈₃N₁₇O₉ | 1042.3 | 1041.6 | 8.4 | 95 | 0.2 | < 0.1 |

| | | | | | | | | | | | | | | | | |
|---|---|---|---|---|---|---|---|---|---|---|---|---|---|---|---|---|
| *^{a}* DE MALDI-TOF MS, +ve mode, α-cyano-4-hydroxycinnamic acid matrix, calibration on authentic H-His-Ala-Lys-Arg-Arg-Leu Ile-Phe-NH₂ *^{b}* Vydac218TP54, 1 mL/min, 25 °C, 0 - 40 % MeCN in 0.1 % aq TFA over 20 min, λ = 214 nm *^{c}* CDK2 / cyclin A kinase assay, pRb substrate, [ATP] = 100 µM *^{d}* Competitive cyclin A binding assay using immobilised biotinyl-Ahx-His-Ala-Lys-Arg-Arg-Leu-Ile-Phe-NH₂ | | | | | | | | | | | | | | | | |

As is clear from the results presented above, the Phe¹⁵⁹ residue represents a key determinant in the p21(152-159) pharmacophore: its truncation abolishes activity and certain well-defined substitutions lead to enhanced potency. For this reason, further constriction of the Phe aromatic side chain may lock it into a bio-active conformation and further potency gains may be expected. Such conformational definition can be introduced in many different ways, *e.g.* through further substitution at *C*^{β} (as in Psa and Pse), introduction of unsaturation, particularly between *C*^{α} and *C*^{β} (as in Dhp), or by tethering of the aromatic system to the peptide backbone (*C*^{α} and NH), as *e.g.* in Tic (refer structures below).

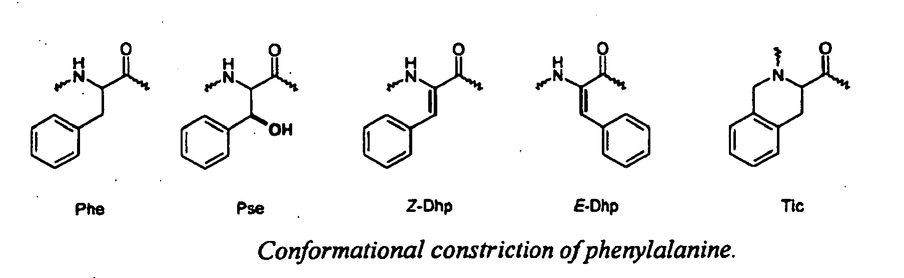

The resolution of β-hydroxy-α-amino acids by the action of proteases on a range of *N-*acyl methyl esters has been described (Chênevert, R.; Létourneau, M.; Thiboutot, S. Can. J. Chem. 1990, 68, 960-963). Using a similar method, we resolved *N*-acetyl-DL-*threo-*phenylserine methyl ester into enantiomers of high optical purity by α-chymotrypsin-mediated enzymatic hydrolysis. Chymotrypsin is specific for the 2*S*-enantiomer that is typically found in natural amino acids. *N*^{α}-Fmoc-L-*threo*-β-phenylserine and *N*^{α}-Fmoc-D-*threo*-β-phenylserine were then synthesised from the resolved enantiomers. In principle the same transformations are applicable in the case of *erythro*-phenylserine (refer *Fig. 3* for stereochemistry of Pse). The protected amino acids were immobilised for standard solid-phase peptide synthesis on a novel synthesis linker (Atkinson, G. E.; Fischer, P. M.; Chan, W. C. J. Org. Chem. 2000, 65, 5048-5056). It was found that the hydroxyl function in Pse did not require temporary protection under the reaction conditions applied (Fischer, P. M.; Retson, K. V.; Tyler, M. I.; Howden, M. E. H. Intl. J. Peptide Protein Res. 1991, 38, 491-493).

The peptides with a C-terminal Dhp residue were obtained directly from the corresponding Pse-containing peptides Thus, protected peptidyl resins were treated with thionyl chloride and triethylamine (Stohlmeyer, M. M.; Tanaka, H.; Wandless, T. J. J. Am. Chem. Soc. 1999, 121, 6100-7101), which led to selective dehydration, *via* a cyclic sulphamidite intermediate, of the hydroxyethylene function in the Pse residue, thus furnishing upon release from the linker-resin the corresponding Dhp peptides. The nature of the reaction mechanism ensured that the intermediate cyclic sulphamidite formed from the *threo-*configuration of phenylserine, under basic conditions, eliminated SO₂ stereospecifically to yield the corresponding Z-Dhp isomer. Peptides H-His-Ser(Ala)-Lys-Arg-Arg-Leu-Ile-Dhp-OH were typically obtained in > 30 % purity when analysed by RP-HPLC and purified yields of 20-30 %. Conversely, *E*-Dhp peptides would be obtained by analogous dehydration of *erythro*-Pse peptides. Protected Pse peptidyl resins were acetylated selectively at the free hydroxyl of the Pse residue to afford the corresponding *O-*acetylphenylserine (Psa) peptides.

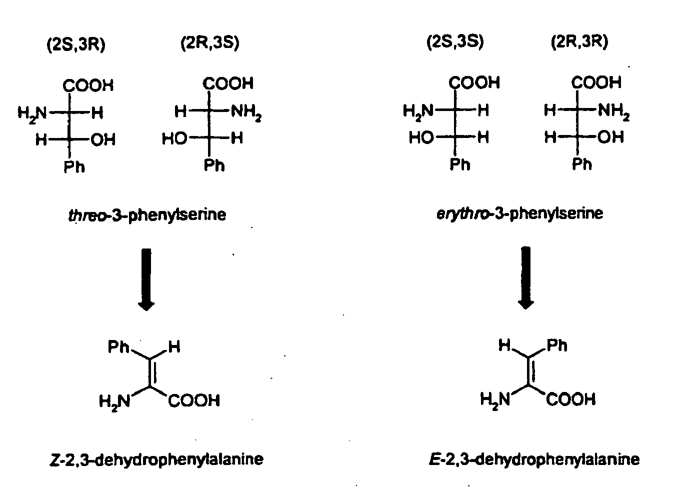

*Stereochemistry of 3-phenylserine.* The *cis* (*Z*) and *trans* (*E*) isomers of dehydrophenylalanine are derived from *threo-* and *erythro*-phenylserine, respectively, by dehydration.

As far as biological activity is concerned, only the L-Pse/Psa p21(152-159) peptides were able to inhibit CDK2/cyclin A and/or to bind efficiently to cyclin A. Of these, H-His-Ala-Lys-Arg-Arg-Leu-IIe-[L-*Psa*]-OH was particularly potent. Both Z-Dhp peptides were biologically active; the Ala¹⁵³ analogue being more potent then the corresponding Ser¹⁵³ peptide. Furthermore, the terminal Phe residue in the p21(152-159) peptides was also replaced with phenylalaninol (Pheol). This substitution was comparatively well-tolerated, showing that the terminal peptide carboxamide (or carboxylate) is not essential in terms of biological activity.

### Example 22: Multiple substitutions in p21(152-159)Ser153Ala,Phe159pFPhe

It was seen above that certain residue substitution in the p21(152-159) peptides were in fact tolerated, and, in some cases, led to increased potency. Some of these single substitutions were then combined in order to test if combinatorial modifications at various positions in the peptide would be additive and/or synergistic. The results suggest that some synergysm is obtained. *E.g*., combination of His152Ala and Phe159pFPhe replacements yielded a peptide analogue with about 80-fold increased potency, whereas the same substitutions individually lead to 2.5- and 5-fold potency increase (in terms of cyclin A binding) only. Thus, combination of the His152Ala, Ser153Ala, and Phe159pFPhe modifications permitted introduction of *e.g*. Lys154Abu, Arg155Gln, Arg156Cit, Arg156Ser, and Ile158Ala.

### Example 22: Multiple substitutions in p21(152-159)Ser153Ala,Phe159pFPhe

| **Compound** | | | | | | | | | | **Formula** | **M,** | **MS°** | **RP-HPLC*^{b}*** | | **Relative activity** | |
|---|---|---|---|---|---|---|---|---|---|---|---|---|---|---|---|---|
| | | | | | | | | | | | | **[M+H]⁺** | ***t*_{R} (min)** | **Purity (%)** | **Kinase inhibition*^{c}*** | **Cyclin A Binding*^{d}*** |
| H- | His- | Ala- | Lys- | Arg- | Arg- | Leu- | Ile- | Phe | -NH₂ | C₄₈H₈₂N₁₈O₈ | | | | | 1 | 1 |
| H- | *Ala-* | Ala*-* | *Abu-* | Arg- | Arg- | Leu- | Ile- | *pFPhe* | -NH₂ | C₄₃H₇₄N₁₅O₈F | 948.15 | 948.16 | 18.88 | 99 | 60 | n/d |
| H*-* | *Ala-* | Ala- | Lys- | Arg- | Arg- | Leu- | Ile- | *pFPhe* | -NH₂ | C₄₁H₇₂N₁₃O₉F | 922.11 | 922.11 | 17.82 | 99 | 80 | n/d |
| H- | *Ala*- | Ala- | Lys- | Arg- | *Cit-* | Leu- | Ile- | *pFPhe* | -NH₂ | C₄₅H₁₈N₁₅O₉F | 992.2 | 922.2 | 16.94 | 99 | 10 | n/d |
| H- | *Ala*- | Ala- | Lys- | Arg- | Arg- | Leu- | *Ala*- | *pFPhe* | -NH₂ | C₄₂H₇₃N₁₆O₈F | 949.14 | 949.69 | 17.89 | 99 | 20 | n/d |
| H- | *Ala*- | Ala- | *Abu-* | Arg*-* | *Ser-* | Leu- | Ile- | *pFPhe* | -NH₂ | C₄₀H₄₇N₁₂O₉F | 879.04 | 879.05 | 16.56 | 99 | 14 | n/d |
| *H-* | *Ala-* | Ala- | Lys- | *Gln*- | Arg- | Leu- | Ile- | *pFPhe* | *-*NH₂ | C₄₄H₇₃N₁₄O₉F | 963.16 | 963.17 | 20.16 | 99 | 4 | n/d |
| | H- | Ala- | Lys- | Arg- | Arg- | Leu- | Ile- | *pFPhe* | -NH₂ | C₄₂H₇₅N₁₅O₇F | 902.15 | 920.14 | 16.6 | 99 | 4 | n/d |

| | | | | | | | | | | | | | | | | |
|---|---|---|---|---|---|---|---|---|---|---|---|---|---|---|---|---|
| *^{a}*DE MALDI-TOF MS, +ve mode, α-cyano-4-bydroxycinnamic acid matrix, calibration on authentic H-His-Ala-Lys-Arg-Arg-Leu-He-Phe-NH₂ *^{b}*Vydac218TP54, 1 mL/min, 25 °C, 0 - 40 % MeCN in 0.1 % aq TFA over 20 min, λ = 214 nm *^{c}*CDK2 / cyclin A kinase assay, pRb substrate, [ATP] = 100 µM *^{d}* Competitive cyclin A binding assay using immobilised biotinyl-Ahx-His-Ala-Lys-Arg-Arg-Leu-Ile-Phe-NH₂ | | | | | | | | | | | | | | | | |

### Example 23: Cyclic peptides

Inspection of the appropriate contacts in the complex structure of cyclin A with a p27^{KIP1} fragment (Russo, A. A.; Jeffrey, P. D.; Patten, A. K.; Massague, J.; Pavletich, N. P. Nature 1996, 382, 325-31).; suggested a starting point for the design of such conformationally constrained peptides. Asn³¹ of the p27 sequence apparently participates in H-bonds not only to the cyclin groove, but also in intra-molecular H-bonding to Gly³⁴. It was therefore plausible that peptide analogues containing macrocyclic constraints approximating this situation may be bio-active. One such cyclic peptide, in which Asn was replaced with Lys and an amide bond patched between its ε-amino group and the carboxyl group of Gly, was designed and modelled ***(******Fig. 3******).***

While molecular modelling suggested that this approach may work, the question remained whether a synthetic peptide containing the same constraint would indeed be bio-active. For this reason a convenient synthetic route based on an alkanesulfonamide safety-catch linker (Backes, B. J.; Ellman, J. A. J. Org. Chem. 1999, 64, 2322-2330) was developed for the synthesis of the desired 'side chain-to-tail' cyclic peptides as set out below;

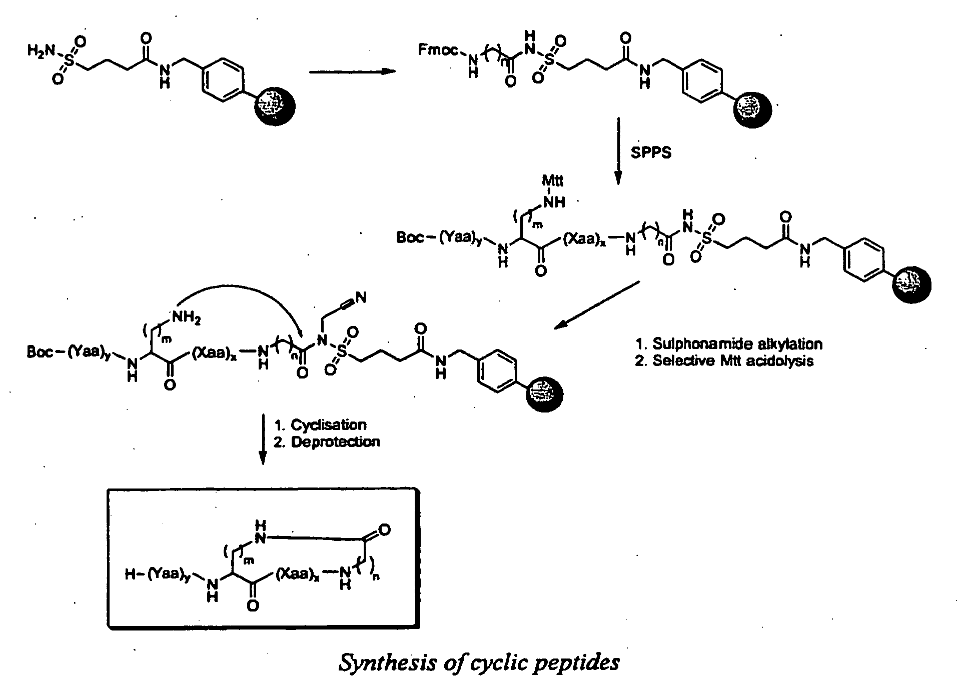

In this method, the immobilised alkanesulfonamide linker is acylated with the desired Fmoc-amino acid, peptide chain assembly is then continued using standard solid-phase peptide synthesis methods. The diamino acid residue which is to participate in the prospective cyclic lactam bond is introduced in an orthogonally protected form, *e.g*. using an Fmoc-diamino acid derivative bearing a side-chain Mtt amino protecting group. After complete chain assembly, the sulfonamide linker is activated through alkylation with iodoacetonitrile. The Mtt protecting group is then removed under mild acidolytic conditions. Intramolecular attack of the liberated amino group on the activated acyl sulfonamide function then results in liberation of the protected cyclic peptide from the solid phase. It is isolated, fully deprotected using strong acidolysis, and purified. A similar approach has recently been reported for the synthesis of 'head-to-tail' cyclic peptides (Zhang, Z.; Van Aerschot, A.; Hendrix, C.; Busson, R.; David, F.; Sandra, P.; Herdewijn, P. Tetrahedron 2000, 56, 2513-2522). 'Side chain-to-tail' cyclic peptides can be obtained through various known methods, using either solid phase- (refer, *e.g.,* Mihara, H.; Yamabe, S.; Niidome, T.; Aoyagi, H. Tetrahedron Lett. 1995, 36, 4837-4840) or solution methods (refer, *e.g.,* He, J. X.; Cody, W. L.; Doherty, A. M. Lett.Peptide Sci. 1994, 1, 25-30).

Using the above method, the peptides 5,8-*cyclo*-[H-His-Ala-Lys-Arg-Lys-Leu-Phe-Gly] and 5,8-*cyclo*-[H-His-Ala-Lys-Arg-Orn-Leu-Phe-Gly] were then synthesised and characterised. The results clearly show that the cyclic constraint introduced is relevant to the peptide's bioactive conformation. Whereas the analogue containing Lys in position 5 was approximately 2 orders of magnitude less potent than the lead peptide H-His-Ala-Lys-Arg-Arg-Leu-Ile-Phe-NH₂, the corresponding Orn analogue was nearly equipotent with the lead peptide.

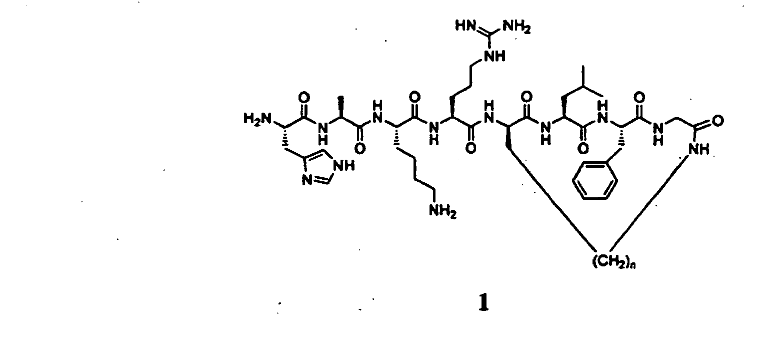

### 5,8-cyclo-[H-His-Ala-Lys-Arg-Lys-Leu-Phe-Gly] (1, n = 3)

Fmoc-Gly-OH (0.64 g, 2.16 mmol) and 4-sulfamylbutyryl aminomethylpolystyrene resin (Novabiochem; 0.50 g, nominally 0.54 mmol) were suspended in DMF (4.25 mL), and Prⁱ₂NEt (0.56 mL, 3.24 mmol) was added. The mixture was stirred for 20 min. After this time, it was cooled to -23 °C, and PyBOP (1.13 g, 2.16 mmol) was added in one portion. Stirring was continued overnight, and the reaction was allowed to warm to room temperature during that period. The resin was then washed thoroughly with DMF, drained, and treated with 50 % acetic anhydride in CH₂Cl₂ (10 mL) for 1 h. After completion, the resin was washed successively with CH₂Cl₂, DMF, and Et₂O, and was dried.

The linear peptide sequence Boc-His(Boc)-Ala-Lys(Boc)-Arg(Pmc)-Lys(Mtt)-Leu-Phe-Gly was then assembled using an ABI 433A peptide synthesiser, employing standard Fmoc protection strategy chemistry. The final peptidyl resin was washed successively with CH₂Cl₂, DMF, and Et₂O, and was dried. An aliquot (0.49 g) was swelled in NMP (4 mL) and treated with iodoacetonitrile (0.37 mL, 5.0 mmol) and Prⁱ₂NEt (0.24 mL, 1.25 mmol) under N₂, for 24 h. After this time, the resin was washed thoroughly with NMP (4 x 5 min), DMF, CH₂Cl₂, and Et₂O, before drying. The Lys⁵ Mtt side-chain protecting group was then removed by treatment with 1.5 % CF₃COOH, 3% MeOH in 1,2-dichloroethane (3×5 mL, 5 min each), and the resin was then washed with further 1,2-dichloroethane, followed by 20 % Prⁱ₂NEt in CH₂Cl₂ and Et₂O. The resin was then dried *in vacuo.*

The activated and Lys⁵ side chain-deprotected peptidyl resin (100 mg) was swelled in 1,4-dioxane (2 mL; dried over sodium-benzophenone), and dimethylaminopyridine (10 mg) was added. The mixture was then heated at reflux for 14 h, followed by filtering of the resin, and washing with DMF (2 × 5 mL, 5 min). The combined filtrate and washings were evaporated, and the residue was treated with 2.5% Pr₃ⁱSiH in CF₃COOH solution for 1 h. The peptide product was collected by precipitation in ice-cold Et₂O, and after washing was dried and fractionated by preparative RP-HPLC (Vydac 218TP1022, 9 mL/min, 13 - 23 % MeCN in 0.1 % aq CF₃COOH over 60 min). Fractions containing pure cyclised peptide were pooled and lyophilised to afford title compound (2.2 mg, 2.34 µmol, 6.5 % w.r.t. initial resin loading). Anal. RP-HPLC: *t*_{R} = 15.4 min (Vydac 218TP54, 1 mL/min, 25 °C, 13 - 23 % MeCN in 0.1 % aq CF₃COOH over 20 min), purity > 99 % (λ = 214 nm). DE MALDI-TOF MS: [M + H]⁺ = 937.8, C₄₄H₇₁N₁₅O₈ requires 938.14 (positive mode, α-cyano-4-hydroxycinnamic acid matrix. The presence of the 5,8-cyclic structure was verified by inspection of appropriate through-space connectivities in the NMR ROESY spectrum of the peptide.

### 5,8-cyclo-[H-His-Als-Lys-Arg-Orn-Leu-Phe-Gly] (1, n = 2)

This compound was prepared in a manner analogous to that described above except that residue position 5 was Orn (Fmoc-Orn(Mtt)-OH was used during chain assembly). A portion of the resin (200mg) was then treated as above, to give the pure title compound (6.3 mg, 6.81 µmol, 8.9 % w.r.t. initial resin loading). Anal. RP-HPLC: *t*_{R} = 14.09 min (Vydac 218TP54, 1 mL/min, 25 °C, 15 - 25 % MeCN in 0.1 % aq CF₃COOH over 20 min), purity > 99 % (λ = 214 nm). DE MALDI-TOF MS: [M + H]⁺ = 926.4, C₄₃H₆₉N₁₅O₈ requires 924.11 (positive mode, α-cyano-4-hydroxycinnamic acid matrix. The presence of the 5,8-cyclic structure was verified by inspection of appropriate through-space connectivities in the NMR ROESY spectrum of the peptide.

| **Compound** | **[Cyclin A] (µg/mL)** | **Immobilised ligand** | **IC₅₀ (µM)** | **Relative activity** |
|---|---|---|---|---|
| H-His-Ala-Lys-Arg-Arg-Leu-Ile-Phe-NH₂ | 5 | HAKRRLIF | 0.3±0.1 | 1 |
| 5,8-*cyclo*-[H-His-Ala-Lys-Arg-*Lys*-Leu-Phe-*Gly*] | 5 | HAKRRLIF | 11.1±0.7 | 0.03 |
| 5,8-*cyclo*-[H-His-Ala-Lys-Arg-*Orn*-Leu-Phe-*Gly*] | 5 | HAKRRLIF | 0.7±0.5 | 0.5 |
| H-His-Ala-Lys-Arg-Arg-Leu-Ile-Phe-NH₂ | 10 | HAKRRLIF | 0.1±0.05 | 1 |
| 5,8-*cyclo*-[H-His-Ala-Lys-Arg-*Lys*-Leu-Phe-*Gly*] | 10 | HAKRRLIF | 16±5 | 0.06 |
| 5,8-*cyclo*-[H-His-Ala-Lys-Arg-*Orn*-Leu-Phe-*Gly*] | 10 | HAKRRLIF | 0.4 ± 0.2 | 0.25 |
| H-His-Ala-Lys-Arg-Arg-Leu-Ile-Phe-NH₂ | 5 | DFYHSKRRLIFS | 0.09±0.02 | 1 |
| 5,8-*cyclo*-[H-His-Ala-Lys-Arg-*Lys*-Leu-Phe-*Gly*] | 5 | DFYHSKRRLIFS | 8 ± 1 | 0.01 |
| 5,8-*cyclo*-[H-His-Ala-Lys-Arg-*Orn*-Leu-Phe-*Gly*] | 5 | DFYHSKRRLIFS | 0.3±0.2 | 0.3 |
| H-His-Ala-Lys-Arg-Arg-Leu-Ile-Phe-NH₂ | 10 | DFYHSKRRLIFS | 1.8 ± 0.9 | 1 |
| 5,8-*cyclo*-[H-His-Ala-*Lys*-Leu-Phe-*Gly*] | 10 | DFYHSKRRLIFS | 22 ± 8 | 0.08 |
| 5,8-*cyclo*-[H-His-Ala-Lys-Arg-*Orn*-Leu-Phe-*Gly*] | 10 | DFYHSKRRLIFS | 6 ± 7 | 0.3 |

### Example 24: Further truncated peptides

The following truncated peptides were prepared and screened for competitive cyclin A binding in accordance with the methods described above. The results demonstrate that *N-*terminally truncated analogues of the 8mer p21-derived peptide H-His-Ala-Lys-Arg-Arg-Leu-Ile-Phe-NH₂, and, to a lesser extent, the p27-derived peptide H-Ser-Ala-Abu-Arg-Arg-Asn-Leu-Phe-Gly-NH₂, retain appreciable cyclin A binding capacity at least down to the C-terminal 4mer sequences.

### Example 24: Further truncated peptides

| **Compound** | | | | | | | | | | **Formula** | **M,** | **MS*^{a}*** | **RP-HPLC*^{b}*** | | **Cyclin A Binding*^{c}*** | |
|---|---|---|---|---|---|---|---|---|---|---|---|---|---|---|---|---|
| | | | | | | | | | | | | [M+H]⁺ | *t_{R}*(min) | Purity (%) | IC₅₀ (µM) | Maximum Inhibition (%) |
| | | | | H- | Arg- | Leu- | Ile- | Phe | -NH₂ | C₂₇H₆N₈O₄ | 546.71 | 548.6 | 15.01‴ | 99 | - | 50 (at 100 µM) |
| | | | H- | Arg- | Arg- | Leu- | Ile- | Phe | -NH₂ | C₃₃H₅₈N₁₂O₅ | 702.9 | 704.7 | 13.35‴ | 9 | 5 | 100 |
| | | H- | Lys- | Arg- | Arg- | Leu- | Ile- | Phe | -NN₂ | C₃₉H₇₀N₁₄O₆ | 831.07 | 832.8 | 12.63‴ | 99 | 5 | 100 |
| | H- | Ala- | Lys- | Arg- | Arg- | Leu- | Ile- | Phe | -NH₂ | C₄₂H₇₃N₁₅O₇ | 902.15 | 903.9 | 12.82‴ | 99 | 2 | 100 |
| H- | His- | Ala- | Lys- | Arg- | Arg- | Leu- | Ile- | Phe | -NH₂ | C₄₈H₈₂N₁₈O₈ | 1039.3 | 1040.4 | 12.91‴ | 99 | 0.3 | 100 |
| | | | | H- | Asn- | Leu- | Phe- | Gly | -NH₂ | C₂₁H₃₂N₆O₅ | 448.52 | 449.6 | 18.14' | 99 | - | 80 (at 200 µM) |
| | | | H- | Arg- | Asn- | Leu- | Phe- | Gly | -NH₂ | C₂₇H₄₄N₁₀O₆ | 604.71 | 605.2 | 17.17' | 9 | - | 20 (at 200 µM) |
| | | H- | Abu- | Arg- | Asn- | Leu- | Phe- | Gly | -NH₂ | C₃₁H₅₁N₁₁O₇ | 689.81 | 690.9 | 12.87" | 99 | - | - |
| | H- | Ala- | Abu- | Arg- | Asn- | Leu- | Phe- | Gly | -NH₂ | C₃₄H₅₆N₁₂O₈ | 760.89 | 761.4 | 13.61" | 99 | 25 | 90 |
| H- | Ser- | Ala- | Abu- | Arg- | Asn- | Leu- | Phe- | Gly | -NH₂ | C₃₁H₆₁N₁₃O₁₀ | 847.97 | 849.1 | 14.90" | 99 | 15 | 100 |

| | | | | | | | | | | | | | | | | |
|---|---|---|---|---|---|---|---|---|---|---|---|---|---|---|---|---|
| *^{a}* DE MALDI-TOF MS, +ve mode, α-cyano-4-hydroxycinnamic acid matrix, calibration on authentic H-His·Ala-Lys-Arg-Arg-Leu Ile-Phe-NH₂ *^{b}* Vydac218TP54, 1 mL/min, 25°C, MeCN gradient in 0.1 % aq TFA over 20 min, λ = 214 nm; *'* 20 - 30 %, *"*23 - 33 %, ‴ 25 - 35 % *^{c}*Competitive cyclin A binding assay using immobilised biotinyl-Ahx-His-Ala-Lys-Arg-Arg-Leu-Ile-Phe-NH₂ | | | | | | | | | | | | | | | | |

### Example 25: Peptide analogues of H-Ala-Ala-Lys-Arg-Leu-Ile-pFPhe-NH₂

| **Compound** | | | | | | | | | | **Formula** | **M,** | **MS*^{a}*** | **RP-HPLC*^{b}*** | |
|---|---|---|---|---|---|---|---|---|---|---|---|---|---|---|
| | | | | | | | | | | | | [M + H]⁺ | *t_{R}* (min) | Purity (%) |
| H- | Ala- | Ala- | Lys- | Arg- | Arg- | Leu- | Ile- | pFPhe | -NH₂ | C₄₅H₇₉FN₁₆O₈ | 991.2 | 991.1 | 12.45 | 90 |
| H- | *Gly-* | Ala- | Lys- | Arg- | Arg- | Leu- | Ile- | pFPhe | -NH₂ | C₄₄H₇₇FN₁₆O₈ | 977.2 | 976.4 | 15.9 | 94 |
| H- | Ala- | Ala- | Lys- | *hArg-* | Arg- | Leu- | Ile- | pFPhe | -NH₂ | C₄₆H₈₁FN₁₆O₈ | 1005.3 | 1004.1 | 12.47 | 85 |
| H- | Ala- | Ala- | Lys- | *Ser-* | Arg- | Leu- | Ile- | pFPhe | -NH₂ | C₄₂H₇₂FN₁₃O₉ | 922.1 | 921.0 | 12.64 | 87 |
| H- | Ala- | Ala- | Lys- | *Hse-* | Arg- | Leu- | Ile- | pFPhe | -NH₂ | C₄₃H₇₄FN₁₃O₉ | 936.1 | 935.5 | 12.68 | 87 |
| H- | Ala- | Ala- | Lys- | Arg- | *Lys-* | Leu- | Ile- | pFPhe | -NH₂ | C₄₅H₇₉FN₁₄O₈ | 963.2 | 962.3 | 12.24 | 90 |
| H- | Ala- | Ala- | Lys- | Arg- | *Orn-* | Leu- | Ile- | pFPhe | -NH₂ | C₄₄H₇₇FN₁₄O₈ | 949.2 | 948.3 | 12.35 | 95 |
| H- | Ala- | Ala- | Lys- | Arg- | *Gln*- | Leu- | Ile- | pFPhe | -NH₂ | C₄₄H₇₅FN₁₄O₉ | 963.2 | 962.6 | 12.58 | 93 |
| H- | Ala- | Ala- | Lys- | Arg- | *Hse-* | Leu- | Ile- | pFPhe | -NH₂ | C₄₃H₇₄FN₁₃O₉ | 936.1 | 934.9 | 12.83 | 90 |
| H- | Ala- | Ala- | Lys- | Arg- | *Thr*- | Leu- | Ile- | pFPhe | -NH₂ | C₄₁H₇₄FN₁₃O₉ | 936.1 | 934.8 | 12.88 | 92 |
| H- | Ala- | Ala- | Lys- | Arg- | *Nva-* | Leu- | Ile- | pFPhe | -NH₂ | C₄₄H₇₆FN₁₃O₈ | 934.2 | 932.6 | 13.74 | 93 |
| H- | Ala- | Ala- | Lys- | Arg- | Arg- | *Phg-* | Ile- | pFPhe | NH₂ | C₄₇H₇₅FN₁₆O₈ | 934.2 | 1009.8 | 11.42 | 90 |
| H- | Ala- | Ala- | Lys- | Arg- | Arg- | *Met-* | Ile- | pFPhe | -NH₂ | C₄₄H₇₇FN₁₆O₈S | 1011.2 | 1009.9 | 12.04 | 80 |
| H- | Ala- | Ala- | Lys- | Arg- | Arg- | *Ala-* | Ile- | pFPhe | -NH₂ | C₄₂H₇₃FN₁₆O₈ | 1009.3 | 948.1 | 11.43 | 82 |
| H- | Ala- | Ala- | Lys- | Arg- | Arg- | *Hof-* | Ile- | pFPhe | -NH₂ | C₄₉H₇₉FN₁₆O₈ | 949.1 | 1038.0 | 13.37 | 88 |
| H- | Ala- | Ala- | Lys- | Arg- | Arg- | Leu- | Ile- | pFPhe | -NH₂ | C₄₆H₈₁FN₁₆O₈ | 1039.3 | 1003.1 | 13.2 | 86 |
| H- | Ala- | Ala- | Lys- | Arg- | Arg- | *alle*- | Ile- | pFPhe | -NH₂ | C₄₅H₇₉HN₁₆O₈ | 1005.3 | 989.5 | 12.32 | 75 |
| H- | Ala- | Ala- | Lys- | Arg- | Arg- | Leu- | *Gly-* | pFPhe | -NH₂ | C₄₁H₇₁FN₁₆O₈ | 991.2 | 934.6 | 11.25 | 84 |
| H- | Ala- | Ala- | Lys- | Arg- | Arg- | Leu- | *βAla-* | pFPhe | -NH₂ | C₄₂H₇₃FN₁₆O₈ | 935.1 | 947.9 | 14.3 | 94 |
| H- | Ala- | Ala- | Lys- | Arg- | Arg- | Leu- | *Phg-* | pFPhe | -NH₂ | C₄₇H₇₅FN₁₆O₈ | 949.1 | 1009.7 | 12.8,14.1 | 88 |
| H- | Ala- | Ala- | Lys- | Arg- | Arg- | Leu- | *Aib-* | pFPhe | -NH₂ | C₄₃H₇₅FN₁₆O₈ | 1011.2 | 961.7 | 15.7 | 95 |
| H- | Ala- | Ala- | Lys- | Arg- | Arg- | Leu- | *Sar-* | pFPhe | -NH₂ | C₄₂H₇₃FN₁₆O₈ | 963.2 | 947.8 | 11.4 | 87 |
| H- | Ala- | Ala- | Lys- | Arg- | Arg- | Leu- | *Pro-* | pFPhe | -NH₂ | C₄₄H₇₅FN₁₆O₈ | 949.1 | 973.8 | 11.9 | 90 |
| H- | Ala- | Ala- | Lys- | Arg- | Arg- | Leu- | *Bug-* | pFPhe | -NH₂ | C₄₅H₇₉FN₁₆O₈ | 975.2 | 990.2 | 15.6 | 90 |
| H- | Ala- | Ala- | Lys- | Arg- | Arg- | Leu- | *Ser-* | pFPhe | -NH₂ | C₄₂H₇₃FN₁₆O₉ | 965.1 | 964.4 | 14.1 | 85 |
| H- | Ala- | Ala- | Lys- | Arg- | Arg- | Leu- | *Asp*- | pFPhe | -NH₂ | C₄₃H₇₃FN₁₆O₁₀ | 993.2 | 992.4 | 14.2 | 95 |
| H- | Ala- | Ala- | Lys- | Arg- | Arg- | Leu- | *Asn-* | pFPhe | -NH₂ | C₄₁H₇₄FN₁₇O₉ | 992.2 | 990.5 | 13.8 | 94 |
| H- | Ala- | Ala- | Lys- | Arg- | Arg- | Leu- | *pFPhe-* | Phe | -NH₂ | C₄₈H₇₇FN₁₆O₈ | 1025.2 | 1024.1 | 16.8 | 94 |
| H- | Ala- | Ala- | Lys- | Arg- | Arg- | Leu- | *diClPhe*- | Phe | -NH₂ | C₄₈H₇₆Cl₂N₁₆O₈ | 1076.1 | 1074.9 | 18.9 | 92 |
| H- | Ala- | Ala- | Lys- | Arg- | Arg- | Leu- | *pClPhe-* | Phe | -NH₂ | C₄₈H₇₇ClN₁₆O₈ | 1041.7 | 1041.1 | 17.8 | 95 |
| H- | Ala- | Ala- | Lys- | Arg- | Arg- | Leu- | *mClPhe-* | Phe | -NH₂ | C₄₈H₇₇ClN₁₆O₈ | 1041.7 | 1058.1 | 17.9 | 95 |
| H- | Ala- | Ala- | Lys- | Arg- | Arg- | Leu- | *oClPhe-* | Phe | -NH₂ | C₄₈H₇₇ClN₁₆O₈ | 1041.7 | 1041.0 | 17.2 | 95 |
| H- | Ala- | Ala- | Lys- | Arg- | Arg- | Leu- | *pIPhe-* | Phe | -NH₂ | C₄₈H₇₇₁N₁₆O₈ | 1133.1 | 1132.6 | 18.5 | 95 |
| H- | Ala- | Ala- | Lys- | Arg- | Arg- | Leu- | *TyrMe-* | Phe | -NH₂ | C₄₉H₈₀N₁₆O₈ | 1037.3 | 1036.7 | 16.4 | 91 |
| H- | Ala- | Ala- | Lys- | Arg- | Arg- | Leu- | *Thi-* | Phe | -NH₂ | C₄₆H₇₆N₁₆O₈S | 1013.3 | 1012.7 | 16.1 | 95 |
| H- | Ala- | Ala- | Lys- | Arg- | Arg- | Leu- | *Pya-* | Phe | -NH₂ | C₄₇H₇₇N₁₇O₈ | 1008.2 | 1007.1 | 13.5 | 86 |
| H- | Ala- | Ala- | Lys- | Arg- | Arg- | Leu- | Ile- | *diClPhe* | -NH₂ | C₄₅H₇₈Cl₂N₁₆O₈ | 1042. 1 | 1005.8 | 18.6 | 91 |
| H- | Ala- | Ala- | Lys- | Arg- | Arg- | Leu- | Ile- | *pClPhe* | -NH₂ | C₄₅H₇₉ClN₁₆O₈ | 1007.7 | 1004.2 | 17.3 | 88 |
| H- | Ala- | Ala- | Lys- | Arg- | Arg- | Leu- | Ile- | *mClPhe* | -NH₂ | C₄₅H₇₉ClN₁₆O₈ | 1007.7 | 1006.8 | 17.3 | 88 |
| H- | Ala- | Ala- | Lys- | Arg- | Arg- | Leu- | Ile- | *oClPhe* | -NH₂ | C₄₅H₇₉ClN₁₆O₈ | 1007.7 | 1007.0 | 16.5 | 84 |
| H- | Ala- | Ala- | Lys- | Arg- | Arg- | Leu- | Ile- | *Phg* | -NH₂ | C₄₄H₇₈N₁₆O₈ | 959.2 | 958.8 | 14.6, 15.8*^{c}* | 95 |
| H- | Ala- | Ala- | Lys- | Arg- | Arg- | Leu- | Ile- | *TyrMe* | -NH₂ | C₄₆H₈₂N₁₆O₉ | 1003.3 | 1002.8 | 15.7 | 90 |
| H- | Ala- | Ala- | Lys- | Arg- | Arg- | Leu- | Ile- | *Thi* | -NH₂ | C₄₁H₇₈N₁₆O₈S | 979.3 | 978.6 | 15.1 | 87 |
| H- | Ala- | Ala- | Lys- | Arg- | Arg- | Leu- | Ile- | *Pya* | -NH₂ | C₄₄H₇₉N₁₇O₈ | 974.2 | 973.7 | 11.5 | 90 |
| H- | Ala- | Ala- | Lys- | Arg- | Arg- | Leu- | Ile- | *Inc* | -NH₂ | C₄₅H₇₉FN₁₆O₈ | 971.2 | (878.99) | 16.1 | 95 |

| | | | | | | | | | | | | | | |
|---|---|---|---|---|---|---|---|---|---|---|---|---|---|---|
| *^{a}* DE MALDI-TOF MS, +ve mode, α-cyano-4-hydroxycinnamic acid matrix, calibration on authentic H-His-Ala-Lys-Arg-Arg-Leu-Ile-Phe-NH₂ *^{b}*Vydac21 8TP54, 1 mL/min, 25 °C, 0 - 40 % MeCN in 0. 1% aq TFA over 20 min *^{c}* Mixture of diastereomers (racemic Fmoc-Phg-OH used) | | | | | | | | | | | | | | |

### Example 26: Peptide analogues of H-Ala-Ala-Lys-Arg-Arg-Leu-Phe-Gly-NH₂

| **Compound** | | | | | | | | | | **Formula** | **Mᵣ** | **MS*^{a}*** | **RP-HPLC*^{b}*** | |
|---|---|---|---|---|---|---|---|---|---|---|---|---|---|---|
| | | | | | | | | | | | | [M+H]⁺ | *^{t}*R (min) | Purity (%) |
| H- | Ala- | Ala- | Lys- | Arg- | Arg- | Leu- | Phe- | Gly | -NH₂ | C₄₁H₇₂N₁₆O₈ | 917.1 | 916.1 | 13.7 | 94 |
| H- | Ala- | Ala- | Lys- | *hArg-* | Arg- | Leu- | Phe- | Gly | -NH₂ | C₄₂H₇₄N₁₆O₈ | 931.2 | 929.4 | 13.8 | 93 |
| H- | Ala- | Ala- | Lys- | *Ser-* | Arg- | Leu- | Phe- | Gly | -NH₂ | C₃₈H₆₅N₁₃O₉ | 848.0 | 847.4 | 14.1 | 95 |
| H- | Ala- | Ala- | Lys- | *Hse-* | Arg- | Leu- | Phe- | Gly | -NH₂ | C₃₉H₆₇N₁₃O₉ | 862.0 | 861.1 | 13.9 | 90 |
| H- | Ala- | Ala- | Lys- | Arg- | *Lys-* | Leu- | Phe- | Gly | -NH₂ | C₄₁H₇₂N₁₄O₈ | 889.1 | 888.8 | 13.5 | 90 |
| H- | Ala- | Ala- | Lys- | Arg- | *Orn-* | Leu- | Phe- | Gly | -NH₂ | C₄₀H₇₀N₁₄O₈ | 875.1 | 874.6 | 13.5 | 95 |
| H- | Ala- | Ala- | Lys- | Arg- | *Gln-* | Leu- | Phe- | Gly | -NH₂ | C₄₀H₆₈N₁₄O₉ | 889.1 | 887.7 | 13.7 | 86 |
| H- | Ala- | Ala- | Lys- | Arg- | *Hse-* | Leu- | Phe- | Gly | -NH₂ | C₃₉H₆₇N₁₃O₉ | 862.0 | 861.3 | 13.9 | 88 |
| H- | Ala- | Ala- | Lys- | Arg- | *Thr-* | Leu | Phe- | Gly | -NH₂ | C₃₉H₆₇N₁₃O₉ | 862.0 | 860.4 | 14.3 | 90 |
| H- | Ala- | Ala- | Lys- | Arg- | *Nva-* | Leu- | Phe- | Gly | -NH₂ | C₄₀H₆₉N₁₃O₈ | 860.1 | 858.7 | 15.6 | 85 |
| H- | Ala- | Ala- | Lys- | Arg- | Arg- | *Met-* | Phe- | Gly | -NH₂ | C₄₀H₇₀N₁₆O₈S | 935.2 | 934.1 | 10.9 | 93 |
| H- | Ala- | Ala- | Lys- | Arg- | Arg- | *Ala-* | Phe- | Gly | -NH₂ | C₃₈H₆₆N₁₆O₈ | 875.0 | 872.2 | 12.7 | 95 |
| H- | Ala- | Ala- | Lys- | Arg- | Arg- | *Hof-* | Phe- | Gly | -NH₂ | C₄₅H₇₂N₁₆O₈ | 965.2 | 962.9 | 15.1 | 81 |
| H- | Ala- | Ala- | Lys- | Arg- | Arg- | *Hle-* | Phe- | Gly | -NH₂ | C₄₂H₇₄N₁₆O₈ | 931.2 | 930.1 | 15.2 | 94 |
| H- | Ala- | Ala- | Lys- | Arg- | Arg- | *aIle-* | Phe- | Gly | -NH₂ | C₄₁H₇₂N₁₆O₈ | 917.1 | 915.9 | 13.2 | 95 |
| H- | Ala- | Ala- | Lys- | Arg- | Arg- | Leu- | *Tic-* | Gly | -NH₂ | C₄₂H₇₂N₁₆O₈ | 929.1 | 928.3 | 13.7 | 93 |
| H- | Ala- | Ala- | Lys- | Arg- | Arg- | Leu- | *Phg-* | Gly | -NH₂ | C₄₀H₇₀N₁₆O₈ | 903.1 | 902.0 | 12.3, 13.7*^{c}* | 95 |
| H- | Ala- | Ala- | Lys- | Arg- | Arg- | Leu- | *pFPhe-* | Gly | -NH₂ | C₄₁H₇₁FN₁₆O₈ | 935.1 | 933.7 | 14.3 | 95 |
| H- | Ala- | Ala- | Lys- | Arg- | Arg- | Leu- | *pIPhe-* | Gly | -NH₂ | C₄₁H₇₁IN₁₆O₈ | 1043.0 | 1041.3 | 16.4 | 92 |
| H- | Ala- | Ala- | Lys- | Arg- | Arg- | Leu- | *Thi-* | Gly | -NH₂ | C₃₉H₇₀N₁₆O₈S | 923.2 | 920.8 | 13.2 | 96 |
| H- | Ala- | Ala- | Lys- | Arg- | Arg- | Leu- | *Pya-* | Gly | -NH₂ | C₄₀H₇₁N₁₇O₈ | 918.1 | 915.1 | 9.3 | 90 |
| H- | Ala- | Ala- | Lys- | Arg- | Arg- | Leu- | *diClPhe-* | Gly | -NH₂ | C₄₁H₇₀Cl₂N₁₆O₈ | 986.0 | 984.2 | 17 | 95 |
| H- | Ala- | Ala- | Lys- | Arg- | Arg- | Leu- | *pClPhe-* | Gly | -NH₂ | C₄₁H₇₁ClN₁₆O₈ | 951.6 | 950.2 | 15.5 | 95 |
| H- | Ala- | Ala- | Lys- | Arg- | Arg- | Leu- | *mClPhe-* | Gly | -NH₂ | C₄₁H₇₁ClN₁₆O₈ | 951.6 | 949.8 | 15.5 | 95 |
| H- | Ala- | Ala- | Lys- | Arg- | Arg- | Leu- | *oClPhe-* | Gly | -NH₂ | C₄₁H₇₁ClN₁₆O₈ | 951.6 | 949.9 | 15 | 95 |
| H- | Ala- | Ala- | Lys- | Arg- | Arg- | Leu- | *1Nap.* | Gly | -NH₂ | C₄₅H₇₄N₁₆O₈ | 967.2 | 965.7 | 16.3 | 95 |
| H- | Ala- | Ala- | Lys- | Arg- | Arg- | Leu- | *2Nap-* | Gly | -NH₂ | C₄₅H₇₄N₁₆O₈ | 967.2 | 966.1 | 16.4 | 95 |
| H- | Ala- | Ala- | Lys- | Arg- | Arg- | Leu- | *Inc-* | Gly | -NH₂ | C₄₁H₇₀N₁₆O₈ | 915.1 | 917.8 | 14.36 | 90 |
| H- | Ala- | Ala- | Lys- | Arg- | Arg- | Leu- | Phe- | *Asp* | -NH₂ | C₄₃H₇₄N₁₆O₁₀ | 975.2 | 972.5 | 13.6 | 95 |
| H- | Ala- | Ala- Lys- | | Arg- | Arg- | Leu- | Phe- | *Glu* | -NH₂ | C₄₄H₇₆N₁₆O₁₀ | 989.2 | 987.5 | 13.3 | 93 |
| H- | Ala- | Ala- | Lys- | Arg- | Arg- | Leu- | Phe- | *Ser* | -NH₂ | C₄₂H₇₄N₁₆O₉ | 947.2 | 944.7 | 13.1 | 95 |
| H- | Ala- | Ala- | Lys- | Arg- | Arg- | Leu- | Phe- | *Asn* | -NH₂ | C₁₃H₇₅N₁₇O₉ | 974.2 | 972.6 | 13.3 | 95 |
| H- | Ala- | Ala- | Lys- | Arg- | Arg- | Leu- | Phe- | *Gln* | -NH₂ | C₄₄H₇₇N₁₇O₉ | 988.2 | 986.9 | 12.5 | 95 |
| H- | Ala- | Ala- | Lys- | Arg- | Arg- | Leu- | Phe- | *Lys* | -NH₂ | C₄₅H₈₁N₁₇O₈ | 988.2 | 987.0 | 13.6 | 95 |

| | | | | | | | | | | | | | | |
|---|---|---|---|---|---|---|---|---|---|---|---|---|---|---|
| *^{a}*DE MALDI-TOF MS, +ve mode, α-cyano-4-hydroxycinnamic acid matrix, calibration on authentic H-His-Ala-Lys-Arg-Arg-Leu-Ile-Phe-NH₂ *^{b}* Vydac218TP54, 1 mL/min, 25 °C, 0 - 40 % MeCN in 0.1 % aq TFA over 20 min *^{c}* Mixture of diastereomers (racemic Fmoc-Phg-OH used) | | | | | | | | | | | | | | |

### Example 27 :ASSAYS

### Example of a cyclin affinity capture method for the identification of peptide inhibitors

Peptides were synthesized as described above. Cyclin D1 was expressed in E coli BL21 (DE3) using PET expression vector and purified from the inclusion bodies. After refolding Cyclin D1 was cross-linked on SulfoLink agarose support (PIERCE). CDK4-6 x His was expressed in Sf9 insect cells infected with the appropriate baculovirus construct and purified by metal-affinity chromatography (Quiagen). GST-Rb (773-924) was expressed in E coli and purified on a Glutathione-Sepharose column according the manufacturers instructions (Pharmacia). CDK4/Cyclin D1 phosphorilation of Rb was determined by incorporation of radio-labeled phosphate in GST-Rb in 96-well format kinase assay. The phosphorylation reaction mixture consisted of 50 mM HEPES pH 7.4, 20 mM MgCl₂, 5 mM EDTA, 2 mM DTT, 20 mM -glicerophosphate, 2 mM NaF, 1 mM Na₃VO₄, 0.5 g CDK4, 0.5 g Cyclin D1, 10 1 GST-Rb Sepharose beads, 100 M ATP and 0.2 Ci ³²P-ATP. The reaction was carried out for 30 min at 30 C at constant shaking. The GST-Rb-Sepharose beads were washed with 50 mM HEPES and 1 mM ATP and the radioactivity was measured on Scintillation counter (Topcount, HP)

### Three Dimensional Models

As described in Example 4 above, a computer generated model of a preferred peptide of the present invention (HAKRRLIF) complexed to cyclin A has been generated using AFFINITY (Molecular Simulations Inc.). A representation of this complex is shown in Figure 4. Using the bond dimension analysis the following cyclin A amno acids have been determined as important in forming associations with this peptide:

| p21 residue | Cyclin A residues | | |
|---|---|---|---|
| | Major Interaction | Intermediate Interaction | Minor Interaction |
| H | E223, E224 | W217, V219, V221 | G222, Y225, 1281 |
| | | S408, E411 | |
| A | | Y225 | E223 |
| K | D284 | | E220, V279 |
| R | | 1213 | A212, V215, L218 |
| | | | Q406, S408 |
| R | D283 | 1213, L214 | M210, L253 |
| L | L253 | G257 | L218, I239, V256 |
| I | | R250, Q254 | |
| F | 1206, R211 | T207, L214 | M200 |

These results demonstrate that the p21^{WAF1}-derived *C*-terminal peptides inhibit the phosphorylation of CDK substrates by binding to the cyclin regulatory subunit of the complex. Through the homology of this sequence with the sequences that have been determined crystallographically in complex with cyclins (Brown, N. R.; Noble, M. E.; Endicott, J. A.; Johnson, L. N. Nat. Cell Biol. 1999, 1, 438-443; Russo, A. A.; Jeffrey, P. D.; Patten, A. K.; Massague, J.; Pavletich, N. P. Nature 1996, 382, 325-31), as well as by virtue of our experimental results, we can conclude that the p21 ^{WAF1} peptide interacts with the same region of the protein as observed in these structures. The substrate recruitment site from these complexes consists mainly of residues of the α1 and α3 helices, which form a shallow groove on the surface, comprised predominantly of hydrophobic residues. These residues are largely conserved in the A, B, E and D1 cyclins. Analysis of the X-ray crystallographically determined structure of the ternary complex of p27^{KIP1}, CDK2 and cyclin A gives considerable insight into the structural basis for the interactions of the p21^{WAF1} peptides examined here. In addition to the available experimentally derived information, a model of cyclin A-bound form of p21(152-159)Ser153Ala has been generated using computational docking procedures. These allow for the complex nature of protein - protein interactions to be delineated in terms of side-chain and backbone flexibility and using a routine employing full molecular mechanics description of non-bonded interactions. The generated model **(*****Fig. 4*****)** gives additional understanding of the molecular basis of the affinity of the peptide for the cyclin groove since it reveals the residues that make important contacts with the protein.

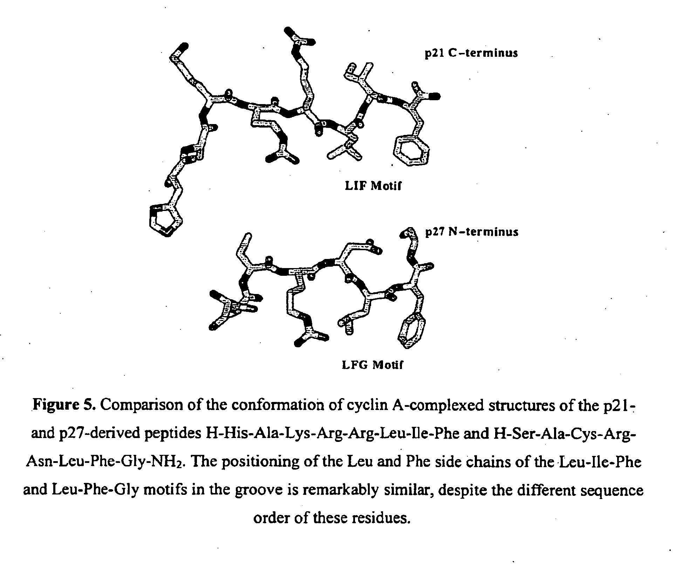

As with Examples 12-22, the following discussion relates to observations made in respect of the peptide HAKRRLIF and all conclusions drawn in respect of potency increasing or decreasing are to be so interpreted. Two immediate conclusions can be drawn from the structure regarding the explanation of the functional significance of residues and which cannot be readily made from the available experimental data. The first is the rationale for the significant potency increase observed in the Ser153Ala substitution and the second is the accommodation of an aromatic residue in either position 7 or 8 of the cyclin binding motif (position 7 in conjunction with Gly at position 8). The basis for this can be ascertained by comparing the X-ray structure of the p27^{KIP1} ternary complex with the binary docked model structure. For the interaction of the LFG motif in the p27 structure, the Leu and Phe residues insert into the hydrophobic pocket formed by Met²¹⁰, Ile²¹³, Trp²¹⁷, and Leu²⁵³ provide the majority of the binding interaction of this region with the cyclin molecule. For the interactions of the LIF motif, the backbone torsion angles of the peptide at positions 6, 7 and 8 adjust in order to allow the Phe side chain to rotate into the hydrophobic pocket and form a high degree of complementarity with the hydrophobic pocket residue of the groove. The Ile side chain at position 7 (158 of p21) rotates out of the pocket to accommodate the Phe and no longer makes any hydrophobic contacts (see ***Fig. 5*****)**. The conformational changes that the peptide undergoes relative to the p27 structure in order to adapt the position 8 Phe residue into the hydrophobic pocket are quite marked. The comparison of the bound peptide structures in ***Fig. 5*** illustrates how the turn structure on the NLFG sequence in p27 which forms both intra- and inter-molecular hydrogen bonds is no longer present in the p21 peptide structure and is replaced by a more extended backbone conformation.

This observation explains the ability of the spacer residue between the Leu and Phe not only to lie tolerated but also to increase affinity significantly as suggested by the observation that HAKRRLIF is more potent than is the hybrid peptide HAKRRLFG. The ability of position 7 analogues including Ala to retain binding with cyclin A also supports this conclusion. The second observation and explanation that can be extracted from the model is the reason for the ability of the Ala replacement at position 153 dramatically to increase binding. This residue in the model forms hydrophobic contact with a second minor pocket which is made up by the second face of the Trp involved in the major pocket and two other residues. In the docked model, this second minor pocket is more pronounced and forms more complementary interactions with Ala than is observed in the crystal structure. It is apparent from this site that placement of the polar Ser residue in this hydrophobic environment would not be favoured and in fact would destabilise the binding interaction of the p21 peptide for the cyclin.

Further examination of the cyclin-bound p21 complex gives further indications of the nature of the residues that contribute to the affinity of the peptide to the recruitment site and that are different to those in the cyclin binding motif of p27. These include the His at position1 (Ser²⁷ in p27), Lys at position 2 (Cys), and Arg at position 5 (Asn). The Ser to His change from p27 to p21 does not appear to be a critical one since both the Ala replacement peptide (p21(149-160)His152Ala) and the truncated peptide minus the residue at position 1 are essentially equipotent. This result is consistent with the binding model since this residue does not form any contacts with the protein with the exception of an H-bond donation of the terminal amino group. By contrast of the Cys to Lys variant, functional data indicates that the Ala mutant undergoes a two-fold reduction in its ability to phosphorylate pRb. From the calculated model, Lys¹⁵⁴ forms an ion pair interaction with Asp²⁸⁴ thus suggesting the basis for the potency decrease with this residue. Finally the Asn to Arg (156 in p21) change leads to a six-fold reduction in potency suggesting that the guanidino function of position 5 contributes to the binding interaction. Again the model indicates that this residue plays an important role in forming hydrogen bonds corresponding to those observed to the Asn residue in the p27 structure and thereby contributing to validation of the docked model. In addition, the recently published structure of a p107 peptide bound to cyclin A verifies the model since the homologous Arg in this structure H-bonds to Asp²⁸³, an interaction which is also observed in the docked complex (Brown, N. R.; Noble, M. E.; Endicott, J. A.; Johnson, L. N. Nat. Cell Biol. 1999, 1,438-443).

Other than those interactions identified as being unique to the peptides of the present invention, there are residues that are conserved between p27 and the p21 C-terminally optimised peptides that form similar interactions to those observed in the experimentally derived structure. In particular, Arg¹⁵⁵, forms H-bonding and electrostatic interactions with Asp²¹⁶ and Glu²⁰⁰ and Leu¹⁵⁷ of the hydrophobic motif inserts into the pocket in a similar orientation to that observed in the crystal structure.

In summary, the model structure of the potent CDK2 and CDK4 inhibitor peptide H-His-Ala-Lys-Arg-Arg-Leu-Ile-Phe-NH₂ in complex with CDK2/cyclin A gives considerable insight into the intermolecular interactions involved in cyclin binding and hence into blocking of substrate recruitment. In conjunction with kinase activity data for the series of p21 truncation and substitution analogues, this model clearly defines the sequence and structural requirements of the cyclin binding motif.

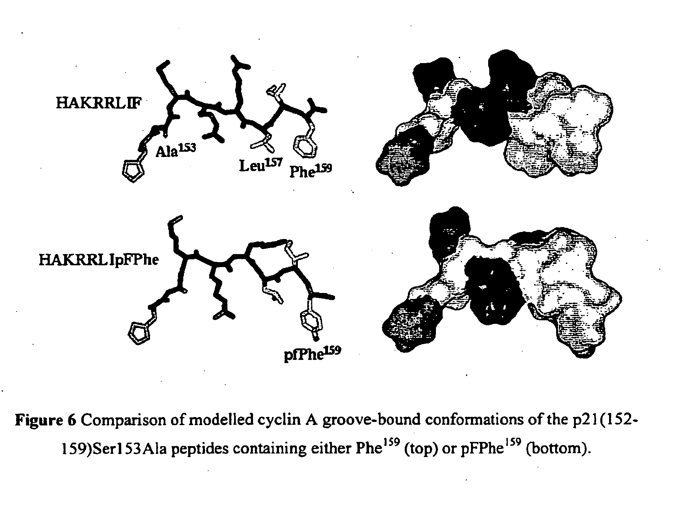

The pFPhe⁸ derivative of the peptide H-His-Ala-Lys-Arg-Arg-Leu-Ile-Phe-NH₂ was found to possess increased activity in binding assays with cyclin A. Molecular modelling docking simulations performed with this analogue **(*****Fig. 6*****)** suggested that the pFPhe derivative inserts deeper into the hydrophobic pocket of the cyclin groove. This appears to result from rearrangement of the residues of the pocket forming more complementary interactions with the pFPhe residue and probably results from the change in charge distribution of the ring relative to the unsubstituted amino acid. This apparent gain in peptide-receptor affinity due to improved hydrophobic interactions of the pFPhe residue suggests that reduction of molecular mass through further *N*-terminal truncation will be possible without severe loss of biological activity.

### References:

- Valerio, R. M., Bray, A. M., Campbell, M., Dipasquale, A., Margellis, C., Rodda, S. J., Geysen, H. M., and Maeji, N. J. (1993) Int. J. Peptide Protein Res. 42,1-9.
- Fields, G. B., and Noble, R. L. (1990) Int. J Peptide Protein Res. 35, 161-214.
- King, D. S., Fields, C. G., and Fields, G. B. (1990) Int. J. Peptide Protein Res. 36, 255-266.
- Ball, K. L., Lain, S., Fåhraeus, R., Smythe, C., and Lane, D. P. (1996) Current Biol. 7, 71-80.
- Harper, JW, Tsai L-H, Zhang, P, Dobrovolski C, Connel-Crowley, (1995) Cell, 6:387-400.
- Holstein, M., Sidranski, D., Vogelstein, B., Harris, C. (1991) Science, 253:49-53.
- Lane DP (1992) Nature 358:15-16.
- Momand, J., Zambetti ,G.P., Olson, D.C., Levine, A.J. (1992) Cell, 69:1237-1245.
- Deng, C., Zhang, P., Harper, J.W., Elledge S.J., Leder P. (1995) Cell, 82: 675-864.
- Adams, P., Sellers, W., Sharma, S., Wu, A., Nalin, C., Kaelin W. (1996) Mol. and Cell. Biol. 16: 6623-6633.
- Chen et al. Mol Cell Biol (1996) 16 4673-4682
- Lin J. et al., Mol Cell Biol (1996) 16 1768-1793
- Russo AA et al., Nature (1996) 382: 325-331
- Chen, et al., (1995) Nature, 374: 386-388
- Flores-Rozas, et al. (1994) Prof. Natl. Acad. Sci. U.S.A., 91: 8655-8659
- Luo, et al. (1995) Nature, 375: 159-161
- Nakanishi, et al. (1995b) J. Biol. Chem., 270: 17060-17063
- Warbrick, et al. (1995) Curr. Biol., 5: 275-282
- Waga, et al. (1994) Nature, 369: 574-578

## Claims

1. A peptide preferentially inhibiting CDK2 activity over CDK4 activity of formula;
DFYHSKRRJLIF (SEQ ID No. 1)
or such a peptide
(i) bearing a further amino acid residue at either end; or,
(ii) having up to 7 amino acid residues deleted from the N-terminal end;
and variants thereof wherein at least one amino acid residue is replaced by an alternative natural or unnatural replacement amino acid residue, with the proviso that the motif XLXF is retained, wherein said peptide is other than of formula
FLRF;
WLRF;
FHLRF;
XXXLRF, XXKLRF, XXRLRF, XXHLRF, XXTLRF, XXFLRF, XXSLRF,
XXILRF, XXLLRF, XXALRF, XXWLRY OR XXMLRF, where X is any amino acid.

2. A peptide according to claim 1, wherein the further amino acid residue is selected from the polar residues C, N, Q, S, T and Y.

3. A peptide according to claim 2, wherein the amino acid residue is added to the N-termimal end.

4. A peptide according to claim 3, wherein the amino acid residue added is threonine.

5. A peptide according to claim 2, wherein the amino acid residue is added to the C-terminal end.

6. A peptide according to claim 5, wherein the amino acid residue added is serine.

7. A peptide according to claim 1, wherein up to 5 amino acid residues are deleted from the N-terminal end of SEQ ID No. 1.

8. A peptide according to claim 7, wherein from 2-4 amino acid residues are deleted from the N-terminal end of SEQ ID No, 1.

9. A peptide according to claim 8, wherein 3 amino acid residues are deleted from the N-terminal end of SEQ ID No. 1.

10. A peptide according to any of claims 7 to 9, wherein a further amino acid residue is added to the C-terminal end of the peptide.

11. A peptide according to claim 10, wherein the amino acid residue added is serine.

12. A peptide according to claim 1, wherein 6 or 7 amino acid residues are deleted from the N-terminal end of SEQ ID No. 1.

13. A variant of peptide according to any preceding claim, wherein the serine residue corresponding to p21(153Ser), is replaced by an alanine residue.

14. A peptide according to any preceding claim, or variant thereof, selected from;
D F Y H S K R R L I F S
T D F Y H S K R R L I F,
A F Y H S K R R L I F S,
D A Y H S K R R L I F S,
D F A H S K R R L I F S,
D F Y A S K R R L I F S,
D F Y H A K R R L I F S,
D F Y H S A R R L I F S,
D F Y H S K R A L I F S,
D F Y H S K R R L A F S,
D F Y H S K R R L I F A,
F Y H S K R R L I F S,
Y H S K R R L I F S,
H S K R R L I F S,
D F Y H S K R R L I F,
F Y H S K R R L I F
Y H S K R R L I F
H S K R R L I F,
S K R R L I F,
K R R L I F,
H- Arg- Leu- Ile- Phe -NH2
H- Arg- Arg- Leu- Ile- Phe -NH2
H- Lys- Arg- Arg- Leu- Ile- Phe -NH2
H- Ala- Lys- Arg- Arg- Leu Ile- Phe -NH2
H- His- Ala- Lys- Arg- Arg- Leu- Ile- Phe -NH2
H- Asn- Leu- Phe- Gly NH2
H- Arg- Asn- Leu- Phe- Gly -NH2
H- Abu- Arg- Asn- Leu- Phe- Gly -NH2
H- Ala- Abu- Arg- Asn- Leu- Phe- Gly -NH2 and
H- Ser- Ala Abu- Arg- Asn- Leu- Phe- Gly -NH2

15. A peptide preferentially inhibiting CDK2 activity over CDK4 activity of formula;
X₁X₂X₃RX₄LX₅F (SEQ ID No. 2)
wherein X₁, X₃, X₄ and X₅ may be any amino acid and X₂ is serine or alanine.

16. A peptide according to claim 15, wherein X₅ is a non polar amino acid residue.

17. A peptide according to claim 15 or claim 16, wherein X₅ is selected from isoleucine and glycine.

18. A peptide according to any one of claims 15 to 17, wherein X₁ and X₄ are both basic amino acid residues.

19. A peptide according to claim 15, wherein X₃ is a basic or polar residue.

20. A peptide according to claim 18 or claim 19, wherein X₁ is histidine, X₄ is arginine, and X₃ is lysine or cysteine.

21. A peptide according to any of claims 15 to 18, wherein X₂ is alanine.

22. A peptide according to any of claims 15 to 18, wherein X₅ is isoleucine.

23. A peptide according to any of claims 15 to 22, or variant thereof, selected from:
H S K R R L I F,
H A K R R L I F,
H S K R R L F G,
H A K R R L F G,
K A C R R L F G,
K A C R R L I F,
| | X1 | X2 | X3 | R | X4 | L | X5 | F | |
|---|---|---|---|---|---|---|---|---|---|
| H- | Ala- | Ala- | Lys- | Arg- | Arg- | Leu- | Ile- | Phe | -NH2 |
| | H- | Ala- | Lys- | Arg- | Arg- | Leu- | Ile- | Phe | -NH2 |
| H- | Pya- | Ala- | Lys- | Arg- | Arg- | Leu- | Ile- | Phe | -NH2 |
| H- | Thi- | Ala- | Lys- | Arg- | Arg- | Leu- | Ile- | Phe | -NH2 |
| H- | Hse- | Ala- | Lys- | Arg- | Arg- | Leu- | Ile- | Phe | -NH2 |
| H- | Phe- | Ala- | Lys- | Arg- | Arg- | Leu- | Ile- | Phe | -NH2 |
| H- | Dab- | Ala- | Lys- | Arg- | Arg- | Leu- | Ile- | Phe | -NH2 |
| H- | His- | Gly- | Lys- | Arg- | Arg- | Leu- | Ile- | Phe | -NH2 |
| H- | His- | Abu- | Lys- | Arg- | Arg- | Leu | Ile- | Phe | -NH2 |
| H- | His- | Nva- | Lys- | Arg- | Arg- | Leu- | Ile- | Phe | -NH2 |
| H- | His- | Bug- | Lys- | Arg- | Arg- | Leu- | Ile- | Phe | -NH2 |
| H- | His- | Val- | Lys- | Arg- | Arg- | Leu- | Ile- | Phe | -NH2 |
| H- | His- | Ile- | Lys- | Arg- | Arg- | Leu- | Ile- | Phe | -NH2 |
| H- | His- | Phg- | Lys- | Arg- | Arg- | Leu- | Ile- | Phe | -NH2 |
| H- | His- | Phe- | Lys- | Arg- | Arg- | Leu- | Ile- | Phe | -NH2 |
| H- | His- | Ala- | Lys- | Arg- | Arg- | Leu- | Ile- | Phe | -NH2 |
| H- | His- | Ala- | Ala- | Arg- | Arg- | Leu- | Ile- | Phe | -NH2 |
| H- | His- | Ala- | Nle- | Arg- | Arg- | Leu- | Ile- | Phe | -NH2 |
| H- | His- | Ala- | Abu- | Arg- | Arg- | Leu- | Ile- | Phe | -NH2 |
| H- | His- | Ala- | Leu- | Arg- | Arg- | Leu- | Ile- | Phe | -NH2 |
| H- | His- | Ala- | Arg- | Arg- | Arg- | Leu- | Ile- | Phe | -NH2 |
| H- | His- | Ala- | Lys- | Ala- | Arg- | Leu- | Ile- | Phe | -NH2 |
| H- | His- | Ala- | Lys- | Cit- | Arg- | Leu- | Ile- | Phe | -NH2 |
| H- | His- | Ala- | Lys- | Hse- | Arg- | Leu- | Ile- | Phe | -NH2 |
| H- | His- | Ala- | Lys- | His- | Arg- | Leu- | Ile- | Phe | -NH2 |
| H- | His- | Ala- | Lys- | Nle- | Arg- | Leu | Ile- | Phe | -NH2 |
| H- | His- | Ala- | Lys- | Gln- | Arg- | Leu- | Ile- | Phe | -NH2 |
| H- | His- | Ala- | Lys- | Lys- | Arg- | Leu- | Ile- | Phe | -NH2 |
| H- | His- | Ala- | Lys- | Arg- | Ala- | Leu- | Ile- | Phe | -NH2 |
| H- | His- | Ala- | Lys- | Arg- | Asn- | Leu- | Ile- | Phe | -NH2 |
| H- | His- | Ala- | Lys- | Arg- | Pro- | Leu- | Ile- | Phe | -NH2 |
| H- | His- | Ala- | Lys- | Arg- | Ser- | Leu- | Ile- | Phe | -NH2 |
| H- | His- | Ala- | Lys- | Arg- | Aib- | Leu- | Ile- | Phe | -NH2 |
| H- | His- | Ala- | Lys- | Arg- | Sar- | Leu- | Ile- | Phe | -NH2 |
| H- | His- | Ala- | Lys- | Arg- | Cit- | Leu- | Ile- | Phe | -NH2 |
| H- | His- | Ala- | Lys- | Arg- | Arg- | Leu- | Ile- | Phe | -NH2 |
| H- | His- | Ala- | Lys- | Arg- | Arg- | Ala- | Ile- | Phe | -NH2 |
| H- | His- | Ala- | Lys- | Arg- | Arg- | leu- | Ile- | Phe | -NH2 |
| H- | His- | Ala- | Lys- | Arg- | Arg- | Ile- | Ile- | Phe | -NH2 |
| H- | His- | Ala- | Lys- | Arg- | Arg- | Val- | Ile- | Phe | -NH2 |
| H- | His- | Ala- | Lys- | Arg- | Arg- | Nle- | Ile- | Phe | -NH2 |
| H- | His- | Ala- | Lys- | Arg- | Arg- | Nva- | Ile- | Phe | -NH2 |
| H- | His- | Ala- | Lys- | Arg- | Arg- | Cha- | Ile- | Phe | -NH2 |
| H- | His- | | Lys- | Arg- | Arg- | Phe- | Ile- | Phe | -NH2 |
| H- | His- | Ala- | Lys- | Arg- | Arg- | 1Nap- | Ile- | Phe | -NH2 |
| H- | His- | Ala- | Lys- | Arg- | Arg- | Leu- | Ala- | Phe | -NH2 |
| H- | His- | Ala- | Lys- | Arg- | Arg- | Leu- | Leu- | Phe | -NH2 |
| H- | His- | Ala- | Lys- | Arg- | Arg- | Leu- | Val- | Phe | -NH2 |
| H- | His- | Ala- | Lys- | Arg- | Arg- | Leu- | Nle- | Phe | -NH2 |
| H- | His- | Ala- | Lys- | Arg- | Arg- | Leu- | Nva- | Phe | -NH2 |
| H- | His- | Ala- | Lys- | Arg- | Arg- | Leu- | Cha- | Phe | -NH2 |
| H- | His- | Ala- | Lys- | Arg- | Arg- | Leu- | Phe- | Phe | -NH2 |
| H- | His- | Ala- | Lys- | Arg- | Arg- | Leu- | INap- | Phe | -NH2 |
| | H- | His- | Ala- | Lys- | Arg- | Arg- | Leu- | Phe | -NH2 |
| H- | His- | Ala- | Lys- | Arg- | Arg- | Leu- | Ile- | Phe | -NH2 |
| H- | His- | Ala- | Lys- | Arg- | Arg- | Leu- | Ile- | Leu | -NH2 |
| H- | His- | Ala- | Lys- | Arg- | Arg- | Leu- | Ile- | Cha | -NH2 |
| H- | His- | Ala- | Lys- | Arg- | Arg- | Leu- | Ile- | Hof | -NH2 |
| H- | His- | Ala- | Lys- | Arg- | Arg- | Leu- | Ile- | Tyr | -NH2 |
| H- | His- | Ala- | Lys- | Arg- | Arg- | Leu- | Ile- | pFPhe | -NH2 |
| H- | His- | Ala- | Lys- | Arg- | Arg- | Leu- | Ile- | mFPhe | -NH2 |
| H- | His- | Ala- | Lys- | Arg- | Arg- | Leu- | Ile- | Trp | -NH2 |
| H- | His- | Ala- | Lys- | Arg- | Arg- | Leu- | Ile- | 1Nap | -NH2 |
| H- | His- | Ala- | Lys- | Arg- | Arg- | Leu- | Ile- | 2Nap | -NH2 |
| H- | His- | Ala- | Lys- | Arg- | Arg- | Leu | Ile- | Lys | -NH2 |
| H- | His- | Ala- | Lys- | Arg- | Arg- | Leu- | Ile- | Tic | -NH2 |
| H- | His | Ala | Lys | Arg | Arg | Leu | Ile | L-Pse | OH |
| H- | His | Ala | Lys | Arg | Arg | Leu | Ile | D-Pse | OH |
| H- | His | Ser | Lys | Arg | Arg | Leu | Ile | L-Pse | OH |
| H- | His | Ser | Lys | Arg | Arg | Leu | Ile | D-Pse | OH |
| H- | His | Ala | Lys | Arg | Arg | Leu | Ile | L-Psa | OH |
| H- | His | Ala | Lys | Arg | Arg | Leu | Ile | D-Psa | OH |
| H- | His | Ser | Lys | Arg | Arg | Leu | Ile | L-Psa | OH |
| H- | His | Ser | Lys | Arg | Arg | Leu | Ile | D-Psa | OH |
| H- | His | Ala | Lys | Arg | Arg | Leu | Ile | Dhp | OH |
| H- | His | Ser | Lys | Arg | Arg | Leu | Ile | Dhp | OH |
| H- | His | Ala | Lys | Arg | Arg | Leu | Ile | Pheol | |
| H- | His | Ser | Lys | Arg | Arg | Leu | Ile | Pheol | |
| H- | Ala- | Ala- | Abu- | Arg- | Arg- | Leu- | Ile- | pFPhe | -NH2 |
| H- | Ala- | Ala- | Lys- | Arg- | Arg- | Leu- | Ile- | pFPhe | -NH2 |
| H- | Ala- | Ala- | Lys- | Arg- | Cit- | Leu- | Ile- | pFPhe | -NH2 |
| H- | Ala- | Ala- | Lys- | Arg- | Arg- | Leu- | Ala- | pFPhe | -NH2 |
| H- | Ala- | Ala- | Abu- | Arg- | Ser- | Leu- | Ile- | pFPhe | -NH2 |
| H- | Ala- | Ala- | Lys- | Gln- | Arg- | Leu- | Ile- | pFPhe | -NH2 |
| H- | Ala- | Ala- | Lys- | Arg- | Arg- | Leu- | Ile- | pFPhe | -NH2 |
| H- | Gly- | Ala- | Lys- | Arg- | Arg- | Leu- | Ile- | pFPhe | -NH2 |
| H- | Ala | Ala- | Lys- | hArg- | Arg- | Leu- | Ile- | pFPhe | -NH2 |
| H- | Ala- | Ala- | Lys- | Ser- | Arg- | Leu- | Ile- | pFPhe | -NH2 |
| H- | Ala- | Ala- | Lys- | Hse- | Arg- | Leu- | Ile- | pFPhe | -NH2 |
| H- | Ala- | Ala- | Lys- | Arg- | Lys- | Leu- | Ile- | pFPhe | -NH2 |
| H- | Ala- | Ala- | Lys- | Arg- | Om- | Leu- | Ile- | pFPhe | -NH2 |
| H- | Ala- | Ala- | Lys- | Arg- | GIn- | Leu- | Ile- | pFPhe | -NH2 |
| H- | Ala- | Ala- | Lys- | Arg- | Hse- | Leu- | Ile- | pFPhe | -NH2 |
| H- | Ala- | Ala- | Lys- | Arg- | Thr- | Leu- | Ile- | pFPhe | -NH2 |
| H- | Ala- | Ala- | Lys- | Arg- | Nva- | Leu- | | pFPhe | -NH2 |
| H- | Ala- | Ala- | Lys- | Arg- | Arg- | Phg- | Ile- | pFPhe | -NH2 |
| H- | Ala- | Ala- | Lys- | Arg- | Arg- | Met- | Ile- | pFPhe | -NH2 |
| H- | Ala- | Ala- | Lys- | Arg- | Arg- | Ala- | Ile- | pFPhe | -NH2 |
| H- | Ala- | Ala- | Lys- | Arg- | Arg- | Hof- | Ile- | pFPhe | -NH2 |
| H- | Ala- | Ala- | Lys- | Arg- | Arg- | hLeu- | Ile- | pFPhe | -NH2 |
| H- | Ala- | Ala- | Lys- | Arg- | Arg- | aIle- | Ile- | pFPhe | -NH2 |
| H- | Ala- | Ala- | Lys- | Arg- | Arg- | Leu- | Gly- | pFPhe | -NH2 |
| H- | Ala- | Ala- | Lys- | Arg- | Arg- | Leu- | βAla- | pFPhe | -NH2 |
| H- | Ala- | Ala- | Lys- | Arg- | Arg- | Leu- | Phg- | pFPhe | -NH2 |
| H- | Ala- | Ala- | Lys- | Arg- | Arg- | Leu- | Aib- | pFPhe | -NH2 |
| H- | Ala- | Ala- | Lys- | Arg- | Arg- | Leu- | Sar- | pFPhe | -NH2 |
| H- | Ala- | Ala- | Lys- | Arg- | Arg- | Leu- | Pro- | pFPhe | -NH2 |
| H- | Ala- | Ala- | Lys- | Arg- | Arg- | Leu- | Bug- | pFPhe | -NH2 |
| H- | Ala- | Ala- | Lys- | Arg- | Arg- | Leu- | Ser- | pFPhe | -NH2 |
| H- | Ala- | Ala- | Lys- | Arg- | Arg- | Leu- | Asp- | pFPhe | -NH2 |
| H- | Ala- | Ala- | Lys- | Arg- | Arg- | Leu- | Asn- | pFPhe | -NH2 |
| H- | Ala- | Ala- | Lys- | Arg- | Arg- | Leu- | pFPhe- | Phe | -NH2 |
| H- | Ala- | Ala- | Lys- | Arg- | Arg- | Leu- | diClPhe | Phe | -NH2 |
| H- | Ala- | Ala- | Lys- | Arg- | Arg- | Leu- | pClPhe- | Phe | -NH2 |
| H- | Ala- | Ala- | Lys- | Arg- | Arg- | Leu- | mClPhe | Phe | -NH2 |
| H- | Ala- | Ala- | Lys- | Arg- | Arg- | Leu- | oClPhe- | Phe | -NH2 |
| H- | Ala- | Ala- | Lys- | Arg- | Arg- | Leu- | pIPhe- | Phe | -NH2 |
| H- | Ala- | Ala- | Lys- | Arg- | Arg- | Leu- | TyrMe- | Phe | -NH2 |
| H- | Ala- | Ala- | Lys- | Arg- | Arg- | Leu- | Thi- | Phe | -NH2 |
| H- | Ala- | Ala- | Lys- | Arg- | Arg- | Leu- | Pya- | Phe | -NH2 |
| H- | Ala- | Ala- | Lys- | Arg- | Arg- | Leu- | Ile- | diClPhe | -NH2 |
| H- | Ala- | Ala- | Lys- | Arg- | Arg- | Leu- | Ile- | pClPhe | -NH2 |
| H- | Ala- | Ala- | Lys- | Arg- | Arg- | Leu- | Ile- | mClPhe | -NH2 |
| H- | Ala- | Ala- | Lys- | Arg- | Arg- | Leu- | Ile- | oClPhe | -NH2 |
| H- | Ala- | Ala- | Lys- | Arg- | Arg- | Leu- | Ile- | Phg | -NH2 |
| H- | Ala- | Ala- | Lys- | Arg- | Arg- | Leu- | Ile- | TyrMe | -NH2 |
| H- | Ala- | Ala- | Lys- | Arg- | Arg- | Leu- | Ile- | Thi | -NH2 |
| H- | Ala- | Ala- | Lys- | Arg- | Arg- | Leu- | Ile- | Pya | -NH2 |
| H- | Ala- | Ala- | Lys- | Arg- | Arg- | Leu- | Ile- | Inc | -NH2 |
and the cyclic peptides;
5,8-cyclo-[H-His-Ala-Lys-Arg-Lys-Leu-Phe-Gly]
5,8-cyclo-[H-His-Ala-Lys-Arg-Orn-Leu-Phe-Gly]

24. A peptide preferentially inhibiting CDK2 activity over CDK4 activity of the formula;
HX₂KRRLX₅F (SEQ ID No. 3)
where X₂ is serine or alanine and X₅ is any amino acid.

25. A peptide according to claim 24, wherein X₂ is alanine.

26. A peptide according to claim 24, wherein X₅ isoleucine.

27. A variant of a peptide according to any of claims 24, 25 or 26, wherein the order of X₅ and F are reversed.

28. A peptide or variant according to any of claims 24 to 27, wherein;
(a) His is unchanged, deleted or replaced by D-His, Ala, Thi, Hse, Phe, or Dab,
(b) X₂ is Ala unchanged or replaced by Ser, Abu or Val,
(c) Lys is unchanged or replaced by Arg or Abu,
(d) Arg is unchanged or replaced by Lys or Gln,
(e) Arg is unchanged or modified to form a cyclic peptide with the C-terminal residue, or replaced by Ser,
(f) Leu is unchanged or replaced by Ile,
(g) X₅ is Ile unchanged, replaced by Leu or Gly if reversed with Phe,
(h) Phe is unchanged or replaced by para-fluoroPhe, meta-fluoroPhe, L-Psa, 2-Nap or Dhp,
(i) the two C-terminal residue are reversed, or
(j) the peptide is in cyclic form by virtue of a linkage between the C-terminal residue and the residue 3 upstream to it.

29. A peptide according to claim 28, wherein X₂ is Ala and X₅ is Ile.

30. A peptide according to claim 28 or 29, wherein Phe is replaced by pira-fluoro-Phe and His is replaced by Ala or is deleted.

31. A peptide according to claim 30, wherein;
(a) Lys is replaced by Abu,
(b) the first Arg residue is replaced by GIn and
(c) the second Arg residue is replaced by Cit or Ser and,
(d) Ile is replaced by Ala.

32. A peptide according to any one of claims 28 to 31, selected from;
| | | | | | | | | | |
|---|---|---|---|---|---|---|---|---|---|
| H- | his- | Ala- | Lys- | Arg- | Arg- | Leu- | Ile- | Phe | -NH2 |
| H- | Ala- | Ala- | Lys- | Arg- | Arg- | Leu- | Ile- | Phe | -NH2 |
| | H- | Ala- | Lys- | Arg- | Arg- | Leu- | Ile- | Phe | -NH2 |
| H- | Thi- | Ala- | Lys- | Arg- | Arg- | Leu- | Ile- | Phe | -NH2 |
| H- | Hse- | Ala- | Lys- | Arg- | Arg- | Leu- | Ile- | Phe | -NH2 |
| H- | Phe- | Ala- | Lys- | Arg- | Arg- | Leu- | Ile- | Phe | -NH2 |
| H- | Dab- | Ala- | Lys- | Arg- | Arg- | Leu- | Ile- | Phe | -NH2 |
| H- | His- | Abu | Lys- | Arg- | Arg- | Leu- | Ile- | Phe | -NH2 |
| | | | | | | | | | |
| H- | His- | Val- | Lys- | Arg- | Arg- | Leu- | Ile- | Phe | -NH2 |
| H- | His- | Ala- | Arg- | Arg- | Arg- | Leu- | Ile- | Phe | -NH2 |
| H- | His- | Ala- | Lys- | Arg- | Arg- | Ile- | Ile- | Phe | -NH2 |
| H- | His- | Ala- | Lys- | Arg- | Arg- | Leu- | Leu. | Phe | -NH2 |
| | | | | | | | | | |
| H- | His- | Ala | Lys- | Arg- | Arg- | Leu- | Ile- | PFPhe | -NH2 |
| H- | His- | Ala- | Lys- | Arg- | Arg- | Leu- | Ile- | 2Nap | -NH2 |
| H- | His | Ala | Lys | Arg | Arg | Leu | Ile | D-Psa | OH |
| H- | His | Ser | Lys | Arg | Arg | Leu | Ile | Dhp | OH |
| H- | Ala- | Ala- | Abu- | Arg- | Arg- | Leu- | Ile- | PFPhe | -NH2 |
| H- | Ala- | Ala- | Lys- | Arg- | Arg- | Leu- | Ile- | PFPhe | -NH2 |
| H- | Ala- | Ala- | Lys- | Arg- | Cit- | Leu- | Ile- | PFPhe | -NH2 |
| H- | Ala- | Ala- | Lys- | Arg- | Arg- | Leu- | Ala- | PFPhe | -NH2 |
| H- | Ala- | Ala- | Abu- | Arg- | Ser- | Leu- | Ile- | PFPhe | -NH2 |
| H- | Ala- | Ala- | Lys- | Gln- | Arg- | Leu- | Ile- | pFPhe | -NH2 |
| | H- | Ala- | Lys- | Arg- | Arg- | Leu- | Ile- | pFPhe | -NH2 |

33. An *in vitro* assay for identifying candidate substances capable of binding to a cyclin associated with a G1 control CDK enzyme and/or inhibiting said enzyme, comprising;
(a) bringing into contact a peptide as defined in any of claims 1-32, said cyclin, said CDK and said candidate substance, under conditions wherein, in the absence of the candidate substance being an inhibitor of interaction of the cyclin/CDK interaction, the p21 derived peptide would bind to said cyclin, and
(b) monitoring any change in the expected binding of the peptide and the cyclin.

34. An *in vitro* assay for the identification of compounds that interact a cyclin or a cyclin when complexed with the physiologically relevant CDK, comprising;
(a) incubating a candidate compound and a peptide of the formula X₁X₂X₃RX₄LX₅F (SEQ ID No. 2) or of formula HX₂KRRLX₅F (SEQ ID No.3) where X₂ is serine or alanine, and X₁, X₃, X₄ and X₅ may be any amino acid; or variants thereof as defined in any of claims 15 to 32 and a cyclin or cyclin/CDK complex,
(b) detecting binding of either the candidate compound or the peptide SEQ ID No. 2 or SEQ ID No. 3 with the cyclin.

35. An assay according to any of claims 33 or 34 wherein the cyclin is selected from cyclin A, cyclin E or cyclin D.

36. An assay according to claim 35 wherein the cyclin is cyclin A.

37. An assay according to any of claims 33 to 36, comprising use of a three dimensional model of a cyclin and a candidate compound.

38. An assay according to any of claims 33 to 36, wherein at least one of the assay components is bound to a solid phase.

39. An assay according to claim 38, wherein the peptide is labeled such as to emit a signal when bound to said cyclin.

40. An assay according to claim 38, wherein the cyclin is labeled such as to emit a signal when bound to the peptide.

41. An assay according to claim 39 or 40, wherein one of the assay components is labeled with a fluorescence emitter and the signal is detected using fluorescence polarisation techniques.

42. A. method or assay according to any of claims 33 to 36, wherein the method of detection comprises monitoring G0 and/or G1/S cell cycle, cell cycle-related apoptosis, suppression of E2F transcription factor, hypophosphorylation of cellular pRb, or in vitro anti-proliferative effects.

43. Use of a peptide defined in any of claims 1 to 32 in the preparation of medicament for use in (a) inhibition of CDK2 or (b) in the treatment of proliferative disorders such as cancers and leukaemias where inhibition of CDK2 would be beneficial.

## Patentansprüche

1. Peptid, welches bevorzugt CDK2-Aktivität gegenüber CDK4-Aktivität hemmt, mit der Formel
DFYHSKRRLIF (SEQ ID NO: 1)
oder ein solches Peptid,
(i) welches einen weiteren Aminosäurerest an einem der Enden trägt oder
(ii) bei dem bis zu 7 Aminosäurereste vom N-terminalen Ende deletiert sind,
und Varianten davon, wobei wenigstens ein Aminosäurerest durch einen alternativen natürlichen oder nicht-natürlichen Ersatz-Aminosäurerest ersetzt ist, mit der Maßgabe, daß das Motiv XLXF behalten wird, wobei das Peptid eine andere Formel hat als
FLRF,
WLRF,
FHLRF,
XXXLRF, XXKLRF, XXRLRF, XXHLRF, XXTLRF, XXFLRF, XXSLRF, XXILRF, XXLLRF, XXALRF, XXWLRF oder XXMLRF, wobei X irgendeine Aminosäure ist.

2. Peptid nach Anspruch 1, wobei der weitere Aminosäurerest unter den polaren Resten C, N, Q, S, T und Y ausgewählt ist.

3. Peptid nach Anspruch 2, wobei der Aminosäurerest zu dem N-terminalen Ende hinzugefügt ist.

4. Peptid nach Anspruch 3, wobei der hinzugefügte Aminosäurerest Threonin ist.

5. Peptid nach Anspruch 2, wobei der Aminosäurerest zum C-terminalen Ende hinzugefügt ist.

6. Peptid nach Anspruch 5, wobei der hinzugefügte Aminosäurerest Serin ist.

7. Peptid nach Anspruch 1, wobei bis zu 5 Aminosäurereste vom N-terminalen Ende von SEQ ID NO: 1 deletiert sind.

8. Peptid nach Anspruch 7, wobei 2-4 Aminosäurereste vom N-terminalen Ende von SEQ ID NO: 1 deletiert sind.

9. Peptid nah Anspruch 8, wobei 3 Aminosäurereste vom N-terminalen Ende von SEQ ID NO: 1 deletiert sind.

10. Peptid nach einem der Ansprüche 7 bis 9, wobei ein weiterer Aminosäurerest zum C-terminalen Ende des Peptids hinzugefügt ist.

11. Peptid nach Anspruch 10, wobei der hinzugefügte Aminosäurerest Serin ist.

12. Peptid nach Anspruch 1, wobei 6 oder 7 Aminosäurereste vom N-terminalen Ende von SEQ ID NO: 1 deletiert sind.

13. Variante des Peptids nach einem der vorangegangenen Ansprüche, wobei der p21(153Ser) entsprechende Serinrest durch einen Alaninrest ersetzt ist.

14. Peptid nach einem der vorangegangenen Ansprüche oder Variante davon, ausgewählt unter:
D F Y H S K R R L I F S,
T D F Y H S K R R L I F,
A F Y H S K R R L I F S,
D A Y H S K R R L I F S,
D F A H S K R R L I F S,
D F Y A S K R R L I F S,
D F Y H A K R R L I F S,
D F Y H S A R R L I F S,
D F Y H S K R A L I F S,
D F Y H S K R R L A F S,
D F Y H S K R R L I F A,
F Y H S K R R L I F S,
Y H S K R R L I F S,
H S K R R L I F S,
D F Y H S K R R L I F,
F Y H S K R R L I F,
Y H S K R R L I F,
H S K R R L I F,
S K R R L I F,
K R R L I F,
H- Arg- Leu- Ile- Phe -NH2
H- Arg- Arg- Leu- Ile- Phe -NH2
H- Lys- Arg- Arg- Leu- Ile- Phe -NH2
H- Ala- Lys- Arg- Arg- Leu- Ile- Phe -NH2
H- His- Ala- Lys- Arg- Arg- Leu- Ile- Phe -NH2
H- Asn- Leu- Phe- Gly -NH2
H- Arg- Asn- Leu- Phe- Gly -NH2
H- Abu- Arg- Asn- Leu- Phe- Gly -NH2
H- Ala- Abu- Arg- Asn- Leu- Phe- Gly -NH2 und
H- Ser- Ala- Abu- Arg- Asn- Leu- Phe- Gly -NH2.

15. Peptid, welches bevorzugt CDK2-Aktivität gegenüber CDK4-Aktivität hemmt, mit der Formel
X₁X₂X₃RX₄LX₅F (SEQ ID NO: 2)
wobei X₁, X₃, X₄ und X₅ irgendwelche Aminosäuren sein können und X₂ Serin oder Alanin ist.

16. Peptid nach Anspruch 15, wobei X₅ ein unpolarer Aminosäurerest ist.

17. Peptid nach Anspruch 15 oder Anspruch 16, wobei X₅ unter Isoleucin und Glycin ausgewählt ist.

18. Peptid nach einem der Ansprüche 15 bis 17, wobei X₁ und X₄ beide basische Aminosäurereste sind.

19. Peptid nach Anspruch 15, wobei X₃ ein basischer oder polarer Rest ist.

20. Peptid nach Anspruch 18 oder Anspruch 19, wobei X₁ Histidin ist, X₄ Arginin ist und X₃ Lysin oder Cystein ist.

21. Peptid nach einem der Ansprüche 15 bis 18, wobei X₂ Alanin ist.

22. Peptid nach einem der Ansprüche 15 bis 18, wobei X₅ Isoleucin ist.

23. Peptid nach einem der Ansprüche 15 bis 22 oder Variante davon, ausgewählt unter:
H S K R R L I F,
H A K R R L I F,
H S K R R L F G,
H A K R R L F G,
K A C R R L F G,
K A C R R L I F,
| | X1 | X2 | X3 | R | X4 | L | X5 | F | |
|---|---|---|---|---|---|---|---|---|---|
| H- | Ala- | Ala- | Lys- | Arg- | Arg- | Leu- | Ile- | Phe | -NH2 |
| | H- | Ala- | Lys- | Arg- | Arg- | Leu- | Ile- | Phe | -NH2 |
| H- | Pya- | Ala- | Lys- | Arg- | Arg- | Leu- | Ile- | Phe | -NH2 |
| H- | Thi- | Ala- | Lys- | Arg- | Arg- | Leu- | Ile- | Phe | -NH2 |
| H- | Hse- | Ala- | Lys- | Arg- | Arg- | Leu- | Ile- | Phe | -NH2 |
| H- | Phe- | Ala- | Lys- | Arg- | Arg- | Leu- | Ile- | Phe | -NH2 |
| H- | Dab- | Ala- | Lys- | Arg- | Arg- | Leu- | Ile- | Phe | -NH2 |
| H- | His- | Gly- | Lys- | Arg- | Arg- | Leu- | Ile- | Phe | -NH2 |
| H- | His- | Abu- | Lys- | Arg- | Arg- | Leu- | Ile- | Phe | -NH2 |
| H- | His- | Nva- | Lys- | Arg- | Arg- | Leu- | Ile- | Phe | -NH2 |
| H- | His- | Bug- | Lys- | Arg- | Arg- | Leu- | Ile- | Phe | -NH2 |
| H- | His- | Val- | Lys- | Arg- | Arg- | Leu- | Ile- | Phe | -NH2 |
| H- | His- | Ile- | Lys- | Arg- | Arg- | Leu- | Ile- | Phe | -NH2 |
| H- | His- | Phg- | Lys- | Arg- | Arg- | Leu- | Ile- | Phe | -NH2 |
| H- | His- | Phe- | Lys- | Arg- | Arg- | Leu- | Ile- | Phe | -NH2 |
| H- | His- | Ala- | Lys- | Arg- | Arg- | Leu- | Ile- | Phe | -NH2 |
| H- | His- | Ala- | Lys- | Arg- | Arg- | Leu- | Ile- | Phe | -NH2 |
| H- | His- | Ala- | Ala- | Arg- | Arg- | Leu- | Ile- | Phe | -NH2 |
| H- | His- | Ala- | Nle- | Arg- | Arg- | Leu- | Ile- | Phe | -NH2 |
| H- | His- | Ala- | Abu- | Arg- | Arg- | Leu- | Ile- | Phe | -NH2 |
| H- | His- | Ala- | Arg- | Arg- | Arg- | Leu- | Ile- | Phe | -NH2 |
| H- | His- | Ala- | Lys- | Ala- | Arg- | Leu- | Ile- | Phe | -NH2 |
| H- | His- | Ala- | Lys- | Cit- | Arg- | Leu- | Ile- | Phe | -NH2 |
| H- | His- | Ala- | Lys- | Hse- | Arg- | Leu- | Ile- | Phe | -NH2 |
| H- | His- | Ala- | Lys- | His- | Arg- | Leu- | Ile- | Phe | -NH2 |
| H- | His- | Ala- | Lys- | Nle- | Arg- | Leu- | Ile- | Phe | -NH2 |
| H- | His- | Ala- | Lys- | Gln- | Arg- | Leu- | Ile- | Phe | -NH2 |
| H- | His- | Ala- | Lys- | Lys- | Arg- | Leu- | Ile- | Phe | -NH2 |
| H- | His- | Ala- | Lys- | Arg- | Ala- | Leu- | Ile- | Phe | -NH2 |
| H- | His- | Ala- | Lys- | Arg- | Asn- | Leu- | Ile- | Phe | -NH2 |
| H- | His- | Ala- | Lys- | Arg- | Pro- | Leu- | Ile- | Phe | -NH2 |
| H- | His- | Ala- | Lys- | Arg- | Ser- | Leu- | Ile- | Phe | -NH2 |
| H- | His- | Ala- | Lys- | Arg- | Aib- | Leu- | Ile- | Phe | -NH2 |
| H- | His- | Ala- | Lys- | Arg- | Sar- | Leu- | Ile- | Phe | -NH2 |
| H- | His- | Ala- | Lys- | Arg- | Cit- | Leu- | Ile- | Phe | -NH2 |
| H- | His- | Ala- | Lys- | Arg- | Arg- | Leu- | Ile- | Phe | -NH2 |
| H- | His- | Ala- | Lys- | Arg- | Arg- | Ala- | Ile- | Phe | -NH2 |
| H- | His- | Ala- | Lys- | Arg- | Arg- | Leu- | Ile- | Phe | -NH2 |
| H- | His- | Ala- | Lys- | Arg- | Arg- | Ile- | Ile- | Phe | -NH2 |
| H- | His- | Ala- | Lys- | Arg- | Arg- | Val- | Ile- | Phe | -NH2 |
| H- | His- | Ala- | Lys- | Arg- | Arg- | Nle- | Ile- | Phe | -NH2 |
| H- | His- | Ala- | Lys- | Arg- | Arg- | Nva- | Ile- | Phe | -NH2 |
| H- | His- | Ala- | Lys- | Arg- | Arg- | Cha- | Ile- | Phe | -NH2 |
| H- | His- | Ala- | Lys- | Arg- | Arg- | Phe- | Ile- | Phe | -NH2 |
| H- | His- | Ala- | Lys- | Arg- | Arg- | 1 Nap- | Ile- | Phe | -NH2 |
| H- | His- | Ala- | Lys- | Arg- | Arg- | Leu- | Ala- | Phe | -NH2 |
| H- | His- | Ala- | Lys- | Arg- | Arg- | Leu- | Leu- | Phe | -NH2 |
| H- | His- | Ala- | Lys- | Arg- | Arg- | Leu- | Val- | Phe | -NH2 |
| H- | His- | Ala- | Lys- | Arg- | Arg- | Leu- | Nle- | Phe | -NH2 |
| H- | His- | Ala- | Lys- | Arg- | Arg- | Leu- | Nva- | Phe | -NH2 |
| H- | His- | Ala- | Lys- | Arg- | Arg- | Leu- | Cha- | Phe | -NH2 |
| H- | His- | Ala- | Lys- | Arg- | Arg- | Leu- | Phe- | Phe | -NH2 |
| H- | His- | Ala- | Lys- | Arg- | Arg- | Leu- | 1 Nap- | Phe | -NH2 |
| | H- | His- | Ala- | Lys- | Arg- | Arg- | Leu- | Phe | -NH2 |
| H- | His- | Ala- | Lys- | Arg- | Arg- | Leu- | Ile- | Phe | -NH2 |
| H- | His- | Ala- | Lys- | Arg- | Arg- | Leu- | Ile- | Leu | -NH2 |
| H- | His- | Ala- | Lys- | Arg- | Arg- | Leu- | Ile- | Cha | -NH2 |
| H- | His- | Ala- | Lys- | Arg- | Arg- | Leu- | Ile- | Hof | -NH2 |
| H- | His- | Ala- | Lys- | Arg- | Arg- | Leu- | Ile- | Tyr | -NH2 |
| H- | His- | Ala- | Lys- | Arg- | Arg- | Leu- | Ile- | pFPhe | -NH2 |
| H- | His- | Ala- | Lys- | Arg- | Arg- | Leu- | Ile- | mFPhe | -NH2 |
| H- | His- | Ala- | Lys- | Arg- | Arg- | Leu- | Ile- | Trp | -NH2 |
| H- | His- | Ala- | Lys- | Arg- | Arg- | Leu- | Ile- | 1 Nap | -NH2 |
| H- | His- | Ala- | Lys- | Arg- | Arg- | Leu- | Ile- | 2Nap | -NH2 |
| H- | His- | Ala- | Lys- | Arg- | Arg- | Leu- | Ile- | Lys | -NH2 |
| H- | His- | Ala- | Lys- | Arg- | Arg- | Leu- | Ile- | Tic | -NH2 |
| H- | His | Ala | Lys | Arg | Arg | Leu | Ile | L-Pse | OH |
| H- | His | Ala | Lys | Arg | Arg | Leu | Ile | D-Pse | OH |
| H- | His | Ser | Lys | Arg | Arg | Leu | Ile | L-Pse | OH |
| H- | His | Ser | Lys | Arg | Arg | Leu | Ile | D-Pse | OH |
| H- | His | Ala | Lys | Arg | Arg | Leu | Ile | L-Psa | OH |
| H- | His | Ala | Lys | Arg | Arg | Leu | Ile | D-Psa | OH |
| H- | His | Ser | Lys | Arg | Arg | Leu | Ile | L-Psa | OH |
| H- | His | Ser | Lys | Arg | Arg | Leu | Ile | D-Psa | OH |
| H- | His | Ala | Lys | Arg | Arg | Leu | Ile | Dhp | OH |
| H- | His | Ser | Lys | Arg | Arg | Leu | Ile | Dhp | OH |
| H- | His | Ala | Lys | Arg | Arg | Leu | Ile | Pheol | |
| H- | His | Ser | Lys | Arg | Arg | Leu | Ile | Pheol | |
| H- | Ala- | Ala- | Abu- | Arg- | Arg- | Leu- | Ile- | pFPhe | -NH2 |
| H- | Ala- | Ala- | Lys- | Arg- | Arg- | Leu- | Ile- | pFPhe | -NH2 |
| H- | Ala- | Ala- | Lys- | Arg- | Cit- | Leu- | Ile- | pFPhe | -NH2 |
| H- | Ala- | Ala- | Lys- | Arg- | Arg- | Leu- | Ala- | pFPhe | -NH2 |
| H- | Ala- | Ala- | Abu- | Arg- | Ser- | Leu- | Ile- | pFPhe | -NH2 |
| H- | Ala- | Ala- | Lys- | Gln- | Arg- | Leu- | Ile- | pFPhe | -NH2 |
| H- | Ala- | Ala- | Lys- | Arg- | Arg- | Leu- | Ile- | pFPhe | -NH2 |
| H- | Gly- | Ala- | Lys- | Arg- | Arg- | Leu- | Ile- | pFPhe | -NH2 |
| H- | Ala- | Ala- | Lys- | hArg- | Arg- | Leu- | Ile- | pFPhe | -NH2 |
| H- | Ala- | Ala- | Lys- | Ser- | Arg- | Leu- | Ile- | pFPhe | -NH2 |
| H- | Ala- | Ala- | Lys- | Hse- | Arg- | Leu- | Ile- | pFPhe | -NH2 |
| H- | Ala- | Ala- | Lys- | Arg- | Lys- | Leu- | Ile- | pFPhe | -NH2 |
| H- | Ala- | Ala- | Lys- | Arg- | Orn- | Leu- | Ile- | pFPhe | -NH2 |
| H- | Ala- | Ala- | Lys- | Arg- | Gln- | Leu- | Ile- | pFPhe | -NH2 |
| H- | Ala- | Ala- | Lys- | Arg- | Hse- | Leu- | Ile- | pFPhe | -NH2 |
| H- | Ala- | Ala- | Lys- | Arg- | Thr- | Leu- | Ile- | pFPhe | -NH2 |
| H- | Ala- | Ala- | Lys- | Arg- | Nva- | Leu- | Ile- | pFPhe | -NH2 |
| H- | Ala- | Ala- | Lys- | Arg- | Arg- | Phg- | Ile- | pFPhe | -NH2 |
| H- | Ala- | Ala- | Lys- | Arg- | Arg- | Met- | Ile- | pFPhe | -NH2 |
| H- | Ala- | Ala- | Lys- | Arg- | Arg- | Ala- | Ile- | pFPhe | -NH2 |
| H- | Ala- | Ala- | Lys- | Arg- | Arg- | Hof- | Ile- | pFPhe | -NH2 |
| H- | Ala- | Ala- | Lys- | Arg- | Arg- | hLeu- | Ile- | pFPhe | -NH2 |
| H- | Ala- | Ala- | Lys- | Arg- | Arg- | alle- | Ile- | pFPhe | -NH2 |
| H- | Ala- | Ala- | Lys- | Arg- | Arg- | Leu- | Gly- | pFPhe | -NH2 |
| H- | Ala- | Ala- | Lys- | Arg- | Arg- | Leu- | βAla- | pFPhe | -NH2 |
| H- | Ala- | Ala- | Lys- | Arg- | Arg- | Leu- | Phg- | pFPhe | -NH2 |
| H- | Ala- | Ala- | Lys- | Arg- | Arg- | Leu- | Aib- | pFPhe | -NH2 |
| H- | Ala- | Ala- | Lys- | Arg- | Arg- | Leu- | Sar- | pFPhe | -NH2 |
| H- | Ala- | Ala- | Lys- | Arg- | Arg- | Leu- | Pro- | pFPhe | -NH2 |
| H- | Ala- | Ala- | Lys- | Arg- | Arg- | Leu- | Bug- | pFPhe | -NH2 |
| H- | Ala- | Ala- | Lys- | Arg- | Arg- | Leu- | Ser- | pFPhe | -NH2 |
| H- | Ala- | Ala- | Lys- | Arg- | Arg- | Leu- | Asp- | pFPhe | -NH2 |
| H- | Ala- | Ala- | Lys- | Arg- | Arg- | Leu- | Asn- | pFPhe | -NH2 |
| H- | Ala- | Ala- | Lys- | Arg- | Arg- | Leu- | pFPhe- | Phe | -NH2 |
| H- | Ala- | Ala- | Lys- | Arg- | Arg- | Leu- | diClPhe | Phe | -NH2 |
| H- | Ala- | Ala- | Lys- | Arg- | Arg- | Leu- | pClPhe- | Phe | -NH2 |
| H- | Ala- | Ala- | Lys- | Arg- | Arg- | Leu- | mClPhe | Phe | -NH2 |
| H- | Ala- | Ala- | Lys- | Arg- | Arg- | Leu- | oClPhe- | Phe | -NH2 |
| H- | Ala- | Ala- | Lys- | Arg- | Arg- | Leu- | plPhe- | Phe | -NH2 |
| H- | Ala- | Ala- | Lys- | Arg- | Arg- | Leu- | TyrMe- | Phe | -NH2 |
| H- | Ala- | Ala- | Lys- | Arg- | Arg- | Leu- | Thi- | Phe | -NH2 |
| H- | Ala- | Ala- | Lys- | Arg- | Arg- | Leu- | Pya- | Phe | -NH2 |
| H- | Ala- | Ala- | Lys- | Arg- | Arg- | Leu- | Ile- | diClPhe | -NH2 |
| H- | Ala- | Ala- | Lys- | Arg- | Arg- | Leu- | Ile- | pClPhe | -NH2 |
| H- | Ala- | Ala- | Lys- | Arg- | Arg- | Leu- | Ile- | mClPhe | -NH2 |
| H- | Ala- | Ala- | Lys- | Arg- | Arg- | Leu- | Ile- | oClPhe | -NH2 |
| H- | Ala- | Ala- | Lys- | Arg- | Arg- | Leu- | Ile- | Phg | -NH2 |
| H- | Ala- | Ala- | Lys- | Arg- | Arg- | Leu- | Ile- | TyrMe | -NH2 |
| H- | Ala- | Ala- | Lys- | Arg- | Arg- | Leu- | Ile- | Thi | -NH2 |
| H- | Ala- | Ala- | Lys- | Arg- | Arg- | Leu- | Ile- | Pya | -NH2 |
| H- | Ala- | Ala- | Lys- | Arg- | Arg- | Leu- | Ile- | Inc | -NH2 |
und den zyklischen Peptiden:
5,8-Cyclo-[H-His-Ala-Lys-Arg-Lys-Leu-Phe-Gly]
5,8-Cyclo-[H-His-Ala-Lys-Arg-Orn-Leu-Phe-Gly].

24. Peptid, welches bevorzugt CDK2-Aktivität gegenüber CDK4-Aktivität hemmt, mit der Formel:
HX₂KRRLX₅F (SEQ ID NO: 3)
wobei X₂ Serin oder Alanin ist und X₅ irgendeine Aminosäure ist.

25. Peptid nach Anspruch 24, wobei X₂ Alanin ist.

26. Peptid nach Anspruch 24, wobei X₅ Isoleucin ist.

27. Variante eines Peptids nach einem der Ansprüche 24, 25 oder 26, wobei die Reihenfolge von X₅ und F umgekehrt ist.

28. Peptid oder Variante nach einem der Ansprüche 24 bis 27, wobei:
(a) His unverändert, deletiert oder durch D-His, Ala, Thi, Hse, Phe oder Dab ersetzt ist,
(b) X₂ Ala, unverändert oder durch Ser, Abu oder Val ersetzt ist,
(c) Lys unverändert oder durch Arg oder Abu ersetzt ist,
(d) Arg unverändert oder durch Lys oder Gln ersetzt ist,
(e) Arg unverändert oder unter Bildung eines zyklischen Peptids mit dem C-terminalen Rest modifiziert oder durch Ser ersetzt ist,
(f) Leu unverändert oder durch Ile ersetzt ist,
(g) X₅ Ile, unverändert, durch Leu ersetzt oder Gly ist, falls mit Phe vertauscht,
(h) Phe unverändert oder durch para-FluorPhe, meta-FluorPhe, L-Psa, 2-Nap oder Dhp ersetzt ist,
(i) die beiden C-terminalen Reste umgekehrt sind oder
(j) das Peptid aufgrund einer Verknüpfung zwischen dem C-terminalen Rest und dem Rest 3 aufstromig davon eine zyklische Form hat.

29. Peptid nach Anspruch 28, wobei X₂ Ala ist und X₅ Ile ist.

30. Peptid nach Anspruch 28 oder 29, wobei Phe durch para-FluorPhe ersetzt ist und His durch Ala ersetzt oder deletiert ist.

31. Peptid nach Anspruch 30, wobei:
(a) Lys durch Abu ersetzt ist,
(b) der erste Arg-Rest durch Gln ersetzt ist und
(c) der zweite Arg-Rest durch Cit oder Ser ersetzt ist und
(d) Ile durch Ala ersetzt ist.

32. Peptid nach einem der Ansprüche 28 bis 31, ausgewählt unter:
| | | | | | | | | | |
|---|---|---|---|---|---|---|---|---|---|
| H- | His- | Ala- | Lys- | Arg- | Arg- | Leu- | Ile- | Phe | -NH2 |
| H- | Ala- | Ala- | Lys- | Arg- | Arg- | Leu- | Ile- | Phe | -NH2 |
| | H- | Ala- | Lys- | Arg- | Arg- | Leu- | Ile- | Phe | -NH2 |
| H- | Thi- | Ala- | Lys- | Arg- | Arg- | Leu- | Ile- | Phe | -NH2 |
| H- | Hse- | Ala- | Lys- | Arg- | Arg- | Leu- | Ile- | Phe | -NH2 |
| H- | Phe- | Ala- | Lys- | Arg- | Arg- | Leu- | Ile- | Phe | -NH2 |
| H- | Dab- | Ala- | Lys- | Arg- | Arg- | Leu- | Ile- | Phe | -NH2 |
| H- | His- | Abu- | Lys- | Arg- | Arg- | Leu- | Ile- | Phe | -NH2 |
| H- | His- | Val- | Lys- | Arg- | Arg- | Leu- | Ile- | Phe | -NH2 |
| H- | His- | Ala- | Arg- | Arg- | Arg- | Leu- | Ile- | Phe | -NH2 |
| H- | His- | Ala- | Lys- | Arg- | Arg- | Ile- | Ile- | Phe | -NH2 |
| H- | His- | Ala- | Lys- | Arg- | Arg- | Leu- | Leu- | Phe | -NH2 |
| H- | His- | Ala- | Lys- | Arg- | Arg- | Leu- | Ile- | PFPhe | -NH2 |
| H- | His- | Ala- | Lys- | Arg- | Arg- | Leu- | Ile- | 2Nap | -NH2 |
| H- | His | Ala | Lys | Arg | Arg | Leu | Ile | D-Psa | OH |
| H- | Ala- | Ser | Lys | Arg | Arg | Leu | Ile | Dhp | OH |
| H- | Ala- | Ala- | Abu- | Arg- | Arg- | Leu- | Ile- | PFPhe | -NH2 |
| H- | Ala- | Ala- | Lys- | Arg- | Arg- | Leu- | Ile- | PFPhe | -NH2 |
| H- | Ala- | Ala- | Lys- | Arg- | Cit- | Leu- | Ile- | PFPhe | -NH2 |
| H- | Ala- | Ala- | Lys- | Arg- | Arg- | Leu- | Ala- | PFPhe | -NH2 |
| H- | Ala- | Ala- | Abu- | Arg- | Ser- | Leu- | Ile- | PFPhe | -NH2 |
| H- | Ala- | Ala- | Lys- | Gln- | Arg- | Leu- | Ile- | pFPhe | -NH2 |
| | H- | Ala- | Lys- | Arg- | Arg- | Leu- | Ile- | pFPhe | -NH2 |

33. *In* vitro-Test zum Identifizieren von Kandidatensubstanzen, die in der Lage sind, an ein Cyclin zu binden, welches mit einem G1-Kontroll-CDK-Enzym assoziiert ist, und/oder das Enzym zu hemmen, wobei der Test folgendes umfaßt:
(a) Inkontaktbringen eines Peptids wie in einem der Ansprüche 1 bis 32 definiert, des Cyclins, der CDK und der Kandidatensubstanz unter Bedingungen, unter denen in Abwesenheit der Kandidatensubstanz, die ein Inhibitor der Wechselwirkung der Cyclin/CDK-Interaktion ist, das von p21 abgeleitete Peptid an das Cyclin binden würde, und
(b) Überwachen jeglicher Veränderung in der erwarteten Bindung des Peptids und des Cyclins.

34. *In* vitro-Test zum Identifizieren von Verbindungen, die mit einem Cyclin oder einem Cyclin, wenn es mit der physiologisch relevanten CDK komplexiert ist, wechselwirken, wobei der Test folgendes umfaßt:
(a) Inkubieren einer Kandidatenverbindung und eines Peptids der Formel X₁X₂X₃RX₄LX₅F (SEQ ID NO: 2) oder der Formel HX₂KRRLX₅F (SEQ ID NO: 3), wobei X₂ Serin oder Alanin ist und X₁, X₃, X₄ und X₅ irgendeine Aminosäure sein können, oder von Varianten davon wie in einem der Ansprüche 15 bis 32 definiert und einem Cyclin oder einem Cyclin/CDK-Komplex,
(b) Detektieren der Bindung entweder der Kandidatenverbindung oder des Peptids von SEQ ID NO: 2 oder SEQ ID NO: 3 mit dem Cyclin.

35. Test nach einem der Ansprüche 33 oder 34, wobei das Cyclin unter Cyclin A, Cyclin E oder Cyclin D ausgewählt ist.

36. Test nach Anspruch 35, wobei das Cyclin Cyclin A ist.

37. Test nach einem der Ansprüche 33 bis 36, welcher die Verwendung eines dreidimensionalen Modells eines Cyclins und einer Kandidatenverbindung umfaßt.

38. Test nach einem der Ansprüche 33 bis 36, wobei wenigstens eine der Testkomponenten an eine feste Phase gebunden ist.

39. Test nach Anspruch 38, wobei das Peptid markiert wird, um ein Signal auszusenden, wenn es an das Cyclin gebunden ist.

40. Test nach Anspruch 38, wobei das Cyclin markiert wird, um ein Signal auszusenden, wenn es an das Peptid gebunden ist.

41. Test nach Anspruch 39 oder 40, wobei eine der Testkomponenten mit einem Fluoreszenzemitter markiert ist und das Signal unter Verwendung von Fluoreszenzpolarisationstechniken detektiert wird.

42. Verfahren oder Test nach einem der Ansprüche 33 bis 36, wobei das Verfahren zur Detektion das Überwachen von G0 und/oder G1/S im Zellzyklus, von mit dem Zellzyklus zusammenhängender Apoptose, der Suppression des Transkriptionsfaktors E2F, der Hypophosphorylierung von zellulärem pRb oder antiproliferativer Wirkungen *in vitro* umfaßt.

43. Verwendung eines Peptids wie in einem der Ansprüche 1 bis 32 definiert bei der Herstellung eines Medikaments zur Verwendung bei (a) der Inhibition von CDK2 oder (b) der Behandlung von proliferativen Störungen, wie Krebs und Leukämien, bei denen die Inhibition von CDK2 vorteilhaft wäre.

## Revendications

1. Peptide inhibant préférentiellement l'activité de CDK2 par rapport à l'activité de CDK4, de formule :
**DFYHSKRRLIF** **(SEQ ID No. 1)**
ou un tel peptide
(i) portant un résidu d'aminoacide supplémentaire à l'une ou l'autre extrémité ; ou
(ii) ayant jusqu'à 7 résidus d'aminoacides éliminés par délétion de l'extrémité N-terminale ;
et ses variants dans lesquels au moins un résidu d'aminoacide est remplacé par un autre résidu d'aminoacide naturel ou non naturel de remplacement, sous réserve que le motif XLXF soit retenu, ledit peptide étant autre qu'un peptide de formule
FLRF ;
WLRF ;
FHLRF ;
XXXLRF, XXKLRF, XXRLRF, XXHLRF, XXTLRF, XXFLRF,
XXSLRF, XXILRF, XXLLRF, XXALRF, XXWLRF ou XXMLRF, dans laquelle X représente n'importe quel aminoacide.

2. Peptide suivant la revendication 1, dans lequel le résidu d'aminoacide supplémentaire est choisi parmi les résidus polaires C, N, Q, S, T et Y.

3. Peptide suivant la revendication 2, dans lequel le résidu d'aminoacide est ajouté à l'extrémité N-terminale.

4. Peptide suivant la revendication 3, dans lequel le résidu d'aminoacide ajouté est la thréonine.

5. Peptide suivant la revendication 2, dans lequel le résidu d'aminoacide est ajouté à l'extrémité C-terminale.

6. Peptide suivant la revendication 5, dans lequel le résidu d'aminoacide ajouté est la sérine.

7. Peptide suivant la revendication 1, dans lequel un nombre allant jusqu'à 5 résidus d'aminoacides est éliminé par délétion de l'extrémité N-terminale de la SEQ ID N° 1.

8. Peptide suivant la revendication 7, dans lequel 2 à 4 résidus d'aminoacides sont éliminés par délétion de l'extrémité N-terminale de la SEQ ID N° 1.

9. Peptide suivant la revendication 8, dans lequel 3 résidus d'aminoacides sont éliminés par délétion de l'extrémité N-terminale de la SEQ ID N° 1.

10. Peptide suivant l'une quelconque des revendications 7 à 9, dans lequel un résidu d'aminoacide supplémentaire est ajouté à l'extrémité C-terminale du peptide.

11. Peptide suivant la revendication 10, dans lequel le résidu d'aminoacide ajouté est la sérine.

12. Peptide suivant la revendication 1, dans lequel 6 ou 7 résidus d'aminoacides sont éliminés par délétion de l'extrémité N-terminale de la SEQ ID N° 1.

13. Variant du peptide suivant l'une quelconque des revendications précédentes, dans lequel le résidu sérine correspondant à p21 (153Ser) est remplacé par un résidu alanine.

14. Peptide suivant l'une quelconque des revendications précédentes, ou un de ses variants, choisi entre :
**D F Y H S K R R L I F S**
**T D F Y H S K R R L I F,**
**A F Y H S K R R L I F S,**
**D A Y H S K R R L I F S,**
**D F A H S K R R L I F S,**
**D F Y A S K R R L I F S,**
**D F Y H A K R R L I F S,**
**D F Y H S A R R L I F S,**
**D F Y H S K R A L I F S,**
**D F Y H S K R R L A F S,**
**D F Y H S K R R L I F A,**
**F Y H S K R R L I F S,**
**Y H S K R R L I F S,**
**H S K R R L I F S,**
**D F Y H S K R R L I F,**
**F Y H S K R R L I F**
**Y H S K R R L I F**
**H S K R R L I F,**
**S K R R L I F,**
**K R R L I F,**
| | | | | | | | | | |
|---|---|---|---|---|---|---|---|---|---|
| H- | Arg- | Leu- | Ile- | Phe | -NH2 | | | | |
| H- | Arg- | Arg- | Leu- | ne- | Phe | -NH2 | | | |
| H- | Lys- | Arg- | Arg- | Leu- | Ile- | Phe | -NH2 | | |
| H- | Ala- | Lys- | Arg- | Arg- | Leu- | De- | Phe | NH2 | |
| H- | His- | Ala- | Lys- | Arg- | Arg- | Leu- | Leu- | Phe | -NH2 |
| H- | Asn- | Leu- | Phe- | Gly | -NH2 | | | | |
| H- | Arg- | Asn- | Leu- | Phe- | Gly | -NH2 | | | |
| H- | Abu- | Erg- | Asn- | Leu- | Phe- | Gly | -NH2 | | |
| H- | Ala- | Abu- | Arg- | Asn- | Leu- | Phe- | Gly | -NH2 | et |
| H- | Ser- | Ala- | Abu- | Are- | Asn- | Leu- | Phe- | Gly | -NH2 |

15. Peptide inhibant préférentiellement l'activité de CDK2 par rapport à l'activité de CDK4, de formule :
**X₁X₂X₃RX₄LX₅F** **(SEQ ID No. 2)**
dans laquelle X₁, X₃, X₄ et X₅ peuvent représenter n'importe quel aminoacide et X₂ représente la sérine ou l'alanine.

16. Peptide suivant la revendication 15, dans lequel X₅ représente un résidu d'aminoacide non polaire.

17. Peptide suivant la revendication 15 ou la revendication 16, dans lequel X₅ est choisi entre l'isoleucine et la glycine.

18. Peptide suivant l'une quelconque des revendications 15 à 17, dans lequel X₁ et X₄ représentent l'un et l'autre des résidus d'aminoacides basiques.

19. Peptide suivant la revendication 15, dans lequel X₃ représente un résidu basique ou polaire.

20. Peptide suivant la revendication 18 ou la revendication 19, dans lequel X₁ représente l'histidine, X₄ représente l'arginine et X₃ représente la lysine ou la cystéine.

21. Peptide suivant l'une quelconque des revendications 15 à 18, dans lequel X₂ représente l'alanine.

22. Peptide suivant l'une quelconque des revendications 15 à 18, dans lequel X₅ représente l'isoleucine.

23. Peptide suivant l'une quelconque des revendications 15 à 22, ou un de ses variants, choisi entre :
**H S K R R L I F,**
**H A K R R L I F,**
**H S K R R L F G,**
**H A K R R L F G,**
**K A C R R L F G,**
**K A C R R L I F,**
| | X1 | X2 | X3 | R | X4 | L | X5 | F | |
|---|---|---|---|---|---|---|---|---|---|
| H- | Ala- | Ala- | Lys- | Arg- | Arg- | Leu- | Ile- | Phe | -NH2 |
| | H- | Ala- | Lys- | Arg- | Arg- | Leu- | Ile- | Phe | -NH2 |
| H- | Pya- | Ala- | Lys- | Arg- | Arg- | Leu- | Ile- | Phe | -NH2 |
| H- | Thi- | Ala- | Lys- | Arg- | Arg- | Leu- | Ile- | Phe | -NH2 |
| H- | Hse- | Ala- | Lys- | Arg- | Arg- | Leu- | Ile- | Phe | -NH2 |
| H- | Phe- | Ala- | Lys- | Arg- | Arg- | Leu- | Ile- | Phe | -NH2 |
| H- | Dab- | Ala- | Lys- | Arg- | Arg- | Leu- | Ile- | Phe | -NH2 |
| H- | His- | Gly- | Lys- | Arg- | Arg- | Leu- | Ile- | Phe | -NH2 |
| H- | His- | Abu- | Lys- | Arg- | Arg- | Leu- | Ile- | Phe | -NH2 |
| H- | His- | Nva- | Lys- | Arg- | Arg- | Leu- | Ile- | Phe | -NH2 |
| H- | His- | Bug- | Lys- | Arg- | Arg- | Leu- | Ile- | Phe | -NH2 |
| H- | His- | Val- | Lys- | Arg- | Arg- | Leu- | Ile- | Phe | -NH2 |
| H- | His- | Ile- | Lys- | Arg- | Arg- | Leu- | Ile- | Phe | -NH2 |
| H- | His- | Phg- | Lys- | Arg- | Arg- | Leu- | Ile- | Phe | -NH2 |
| H- | His- | Phe- | Lys- | Arg- | Arg- | Leu- | Ile- | Phe | -NH2 |
| H- | His- | Ala- | Lys- | Arg- | Arg- | Leu- | Ile- | Phe | -NH2 |
| H- | His- | Ala- | Nle- | Arg- | Arg- | Leu- | Ile- | Phe | -NH2 |
| H- | His- | Ala- | Nle- | Arg- | Arg- | Leu- | Ile- | Phe | -NH2 |
| H- | His- | Ala- | Abu- | Arg- | Arg- | Leu- | Ile- | Phe | -NH2 |
| H- | His- | Ala- | Leu- | Arg- | Arg- | Leu- | Ile- | Phe | -NH2 |
| H- | His- | Ala- | Arg- | Arg- | Arg- | Leu- | Ile- | Phe | -NH2 |
| H- | His- | Ala- | Lys- | Ala- | Arg- | Leu- | Ile- | Phe | -NH2 |
| H- | His- | Ala- | Lys- | Cit- | Arg- | Leu- | Ile- | Phe | -NH2 |
| H- | His- | Ala- | Lys- | Hse- | Arg- | Leu- | Ile- | Phe | -NH2 |
| H- | His- | Ala- | Lys- | His- | Arg- | Leu- | Ile- | Phe | -NH2 |
| H- | His- | Ala- | Lys- | Nle- | Arg- | Leu- | Ile- | Phe | -NH2 |
| H- | His- | Ala- | Lys- | Gln- | Arg- | Leu- | Ile- | Phe | -NH2 |
| H- | His- | Ala- | Lys- | Lys- | Arg- | Leu- | Ile- | Phe | -NH2 |
| H- | His- | Ala- | Lys- | Arg- | Ala- | Leu- | Ile- | Phe | -NH2 |
| H- | His- | Ala- | Lys- | Arg- | Asn- | Leu- | Ile- | Phe | -NH2 |
| H- | His- | Ala- | Lys- | Arg- | Pro- | Leu- | Ile- | Phe | -NH2 |
| H- | His- | Ala- | Lys- | Arg- | Ser- | Leu- | Ile- | Phe | -NH2 |
| H- | His- | Ala- | Lys- | Arg- | Aib- | Leu- | Ile- | Phe | -NH2 |
| H- | His- | Ala- | Lys- | Arg- | Sar- | Leu- | Ile- | Phe | -NH2 |
| H- | His- | Ala- | Lys- | Arg- | Cit- | Leu- | Ile- | Phe | -NH2 |
| H- | His- | Ala- | Lys- | Arg- | Arg- | Leu- | Ile- | Phe | -NH2 |
| H- | His- | Ala- | Lys- | Arg- | Arg- | Ala- | Ile- | Phe | -NH2 |
| H- | His- | Ala- | Lys- | Arg- | Arg- | leu- | Ile- | Phe | -NH2 |
| H- | His- | Ala- | Lys- | Arg- | Arg- | Ile- | Ile- | Phe | -NH2 |
| H- | His- | Ala- | Lys- | Arg- | Arg- | val- | Ile- | Phe | -NH2 |
| H- | His- | Ala- | Lys- | Arg- | Arg- | Nle- | Ile- | Phe | -NH2 |
| H- | His- | Ala- | Lys- | Arg- | Arg- | Nva- | Ile- | Phe | -NH2 |
| H- | His- | Ala- | Lys- | Arg- | Arg- | Cha- | Ile- | Phe | -NH2 |
| H- | His- | Ala- | Lys- | Arg- | Arg- | Phe- | Ile- | Phe | -NH2 |
| H- | His- | Ala- | Lys- | Arg- | Arg- | INap- | Ile- | Phe | -NH2 |
| H- | His- | Ala- | Lys- | Arg- | Arg- | Leu- | Ala- | Phe | -NH2 |
| H- | His- | Ala- | Lys- | Arg- | Arg- | Leu- | Leu- | Phe | -NH2 |
| H- | His- | Ala- | Lys- | Arg- | Arg- | Leu- | Val- | Phe | -NH2 |
| H- | His- | Ala- | Lys- | Arg- | Arg- | Leu- | Nle- | Phe | -NH2 |
| H- | His- | Ala- | Lys- | Arg- | Arg- | Leu- | Nva- | Phe | -NH2 |
| H- | His- | Ala- | Lys- | Arg- | Arg- | Leu- | Cha- | Phe | -NH2 |
| H- | His- | Ala- | Lys- | Arg- | Arg- | Leu- | Phe- | Phe | -NH2 |
| H- | His- | Ala- | Lys- | Arg- | Arg- | Leu- | INap- | Phe | -NH2 |
| | H- | His- | Ala- | Lys- | Arg- | Arg- | Leu- | Phe | -NH2 |
| H- | His- | Ala- | Lys- | Arg- | Arg- | Leu- | Ile- | Phe | -NH2 |
| H- | His- | Ala- | Lys- | Arg- | Arg- | Leu- | Ile- | Leu | -NH2 |
| H- | His- | Ala- | Lys- | Arg- | Arg- | Leu- | Ile- | Cha | -NH2 |
| H- | His- | Ala- | Lys- | Arg- | Arg- | Leu- | Ile- | Hof | -NH2 |
| H- | His- | Ala- | Lys- | Arg- | Arg- | Leu- | Ile- | Tyr | -NH2 |
| H- | His- | Ala- | Lys- | Arg- | Arg- | Leu- | Ile- | pFPhe | -NH2 |
| H- | His- | Ala- | Lys- | Arg- | Arg- | Leu- | Ile- | mFPhe | -NH2 |
| H- | His- | Ala- | Lys- | Arg- | Arg- | Leu- | Ile- | Trp | -NH2 |
| H- | His- | Ala- | Lys- | Arg- | Arg- | Leu- | Ile- | 1Nap | -NH2 |
| H- | His- | Ala- | Lys- | Arg- | Arg- | Leu- | Ile- | 2Nap | -NH2 |
| H- | His- | Ala- | Lys- | Arg- | Arg- | Leu | Ile- | Lys | -NH2 |
| H- | His- | Ala- | Lys- | Arg- | Arg- | Leu- | Ile- | Tic | -NH2 |
| H- | His | Ala | Lys | Arg | Arg | Leu | Ile | L-Pse | OH |
| H- | His | Ala | Lys | Arg | Arg | Leu | Ile | D-Pse | OH |
| H- | His | Ser | Lys | Arg | Arg | Leu | Ile | L-Pse | OH |
| H- | His | Ser | Lys | Arg | Arg | Leu | Ile | D-Pse | OH |
| H- | His | Ala | Lys | Arg | Arg | Leu | Ile | L-Psa | OH |
| H- | His | Ala | Lys | Arg | Arg | Leu | Ile | D-Psa | OH |
| H- | His | Ser | Lys | Arg | Arg | Leu | Ile | L-Psa | OH |
| H- | His | Ser | Lys | Arg | Arg | Leu | Ile | D-Psa | OH |
| H- | His | Ala | Lys | Arg | Arg | Leu | Ile | Dhp | OH |
| H- | His | Ser | Lys | Arg | Arg | Leu | Ile | Dhp | OH |
| H- | His | Ala | Lys | Arg | Arg | Leu | Ile | Pheol | |
| H- | His | Ser | Lys | Arg | Arg | Leu | Ile | Pheol | |
| H- | Ala- | Ala- | Abu- | Arg- | Arg- | Leu- | Ile- | pFPhe | -NH2 |
| H- | Ala- | Ala- | Lys- | Arg Arg- | Arg- | Leu- | Ile- | pFPhe | -NH2 |
| H- | Ala- | Ala- | Lys- | Arg- | Cit- | Leu- | Ile- | pFPhe | -NH2 |
| H- | Ala- | Ala- | Lys- | Arg- | Arg- | Leu- | Ala- | pFPhe | -NH2 |
| H- | Ala- | Ala- | Abu- | Arg- | Ser- | Leu- | Ile- | pFPhe | -NH2 |
| H- | Ala- | Ala | Lys- | Gln- | Arg- | Leu- | Ile- | pFPhe | -NH2 |
| H- | Ala- | Ala- | Lys- | Arg- | Arg- | Leu- | Ile- | pFPhe | -NH2 |
| H- | Gly- | Ala- | Lys- | Arg- | Arg- | Leu- | Ile- | pFPhe | -NH2 |
| H- | Ala- | Ala- | Lys- | hArg- | Arg- | Leu- | Ile- | pFPhe | -NH2 |
| H- | Ala- | Ala- | Lys- | Ser- | Arg- | Leu- | Ile- | pFPhe | -NH2 |
| H- | Ala- | Ala- | Lys- | Hse- | Arg- | Leu- | Ile- | pFPhe | -NH2 |
| H- | Ala- | Ala- | Lys- | Arg- | Lys- | Leu- | Ile- | pFPhe | -NH2 |
| H- | Ala- | Ala- | Lys- | Arg- | Orn- | Leu- | Ile- | pFPhe | -NH2 |
| H- | Ala- | Ala- | Lys- | Arg- | Gln- | Leu- | Ile- | pFPhe | -NH2 |
| H- | Ala- | Ala- | Lys- | Arg- | Hse- | Leu- | Ile- | pFPhe | -NH2 |
| H- | Ala- | Ala- | Lys- | Arg- | Thr- | Leu- | Ile- | pFPhe | -NH2 |
| H- | Ala- | Ala- | Lys- | Arg- | Nva- | Leu- | Ile- | pFPhe | -NH2 |
| H- | AIa- | Ala- | Lys- | Arg- | Arg- | Phg- | Ile- | pFPhe | -NH2 |
| H- | Ala- | Ala- | Lys- | Arg- | Arg- | Met- | Ile- | pFPhe | -NH2 |
| H- | Ala- | Ala- | Lys- | Arg- | Arg- | Ala- | Ile- | pFPhe | -NH2 |
| H- | Ala- | Ala- | Lys- | Arg- | Arg- | Hof- | Ile- | pFPhe | -NH2 |
| H- | Ala- | Ala- | Lys- | Arg- | Arg- | hLeu- | Ile- | pFPhe | -NH2 |
| H- | Ala- | Ala- | Lys- | Arg- | Arg- | alle- | Ile- | pFPhe | -NH2 |
| H- | Ala- | Ala- | Lys- | Arg- | Arg- | Leu- | Gly- | pFPhe | -NH2 |
| H- | Ala- | Ala- | Lys- | Arg- | Arg- | Leu- | βAla- | pFPhe | *-*NH2 -NH2 |
| H- | Ala- | Ala- | Lys- | Arg- | Arg- | Leu- | Phg- | pFPhe | -NH2 |
| H- | Ala- | Ala- | Lys- | Arg- | Arg- | Leu- | Aib- | pFPhe | -NH2 |
| H- | Ala- | Ala- | Lys- | Arg- | Arg- | Leu- | Sar- | pFPhe | -NH2 |
| H- | Ala- | Ala- | Lys- | Arg- | Arg- | Leu- | Pro- | pFPhe | -NH2 |
| H- | Ala- | Ala- | Lys- | Arg- | Arg- | Leu- | Bug- | pFPhe | -NH2 |
| H- | Ala- | Ala- | Lys- | Arg- | Arg- | Leu- | Ser- | pFPhe | -NH2 |
| H- | Ala- | Ala- | Lys- | Arg- | Arg- | Leu- | Asp- | pFPhe | -NH2 |
| H- | Ala- | Ala- | Lys- | Arg- | Arg- | Leu- | Asn- | pFPhe | -NH2 |
| H- | Ala- | Ala- | Lys- | Arg- | Arg- | Leu- | pFPhe- | Phe | -NH2 |
| H- | Ala- | Ala- | Lys- | Arg- | Arg- | Leu- | diClPhe | Phe | -NH2 |
| H- | Ala- | Ala- | Lys- | Arg- | Arg- | Leu- | pClPhe- | Phe | -NH2 |
| H- | Ala- | Ala- | Lys- | Arg- | Arg- | Leu- | mClPhe | Phe | -NH2 |
| H- | Ala- | Ala- | Lys- | Arg- | Arg- | Leu- | oClPhe- | Phe | -NH2 |
| H- | Ala- | Ala- | Lys- | Arg- | Arg- | Leu- | pIPhe- | Phe | -NH2 |
| H- | Ala- | Ala- | Lys- | Arg- | Arg- | Leu- | TyrMe- | Phe | -NH2 |
| H- | Ala- | Ala- | Lys- | Arg- | Arg- | Leu- | Thi- | Phe | -NH2 |
| H- | Ala- | Ala- | Lys- | Arg- | Arg- | Leu- | Pya- | Phe | -NH2 |
| H- | Ala- | Ala- | Lys- | Arg- | Arg- | Leu- | Ile- | diClPhe | -NH2 |
| H- | Ala- | Ala- | Lys- | Arg- | Arg- | Leu- | Ile- | pClPhe | -NH2 |
| H- | Ala- | Ala- | Lys- | Arg- | Arg- | Leu- | Ile- | mClPhe | -NH2 |
| H- | Ala- | Ala- | Lys- | Arg- | Arg- | Leu- | Ile- | oClPhe | -NH2 |
| H- | Ala- | Ala- | Lys- | Arg- | Arg- | Leu- | Ile- | Phg | -NH2 |
| H- | Ala- | Ala- | Lys- | Arg- | Arg- | Leu- | Ile- | TyrMe | -NH2 |
| H- | Ala- | Ala- | Lys- | Arg- | Arg- | Leu- | Ile- | Thi | -NH2 |
| H- | Ala- | Ala- | Lys- | Arg- | Arg- | Leu- | Ile- | Pya | -NH2 |
| H- | Ala- | Ala- | Lys- | Arg- | Arg- | Leu- | Ile- | Inc | -NH2 |
et les peptides cycliques :
**5,8-cyclo-[H-His-Ala-Lys-Arg-Lys-Leu-Phe-Gly]**
**5,8-cyclo-[H-His-Ala-Lys-Arg-Orn-Leu-Phe-Gly]**

24. Peptide inhibant préférentiellement l'activité de CDK2 par rapport à l'activité de CDK4, de formule :
**HX₂KRRLX₅F** **(SEQ ID No. 3)**
dans laquelle X₂ représente la sérine ou l'alanine et X₅ représente n'importe quel aminoacide.

25. Peptide suivant la revendication 24, dans lequel X₂ représente l'alanine.

26. Peptide suivant la revendication 24, dans lequel X₅ représente l'isoleucine.

27. Variant d'un peptide suivant l'une quelconque des revendications 24, 25 et 26, dans lequel l'ordre de X₅ et F est inversé.

28. Peptide ou variant suivant l'une quelconque des revendications 24 à 27, dans lequel :
(a) His est inchangé, éliminé par délétion ou remplacé par D-His, Ala, Thi, Hse, Phe ou Dab,
(b) X₂ représente Ala, inchangé ou remplacé par Ser, Abu ou Val,
(c) Lys est inchangé ou remplacé par Arg ou Abu,
(d) Arg est inchangé ou remplacé par Lys ou Gln,
(e) Arg est inchangé ou modifié pour former un peptide cyclique avec le résidu C-terminal, ou est remplacé par Ser,
(f) Leu est inchangé ou remplacé par Ile,
(g) X₅ représente un résidu Ile inchangé, remplacé par Leu ou Gly s'il est inversé avec Phe,
(h) Phe est inchangé ou remplacé par para-fluoroPhe, méta-fluoroPhe, L-Psa, 2-Nap ou Dhp,
(i) les deux résidus C-terminaux sont inversés, ou
(j) le peptide est sous forme cyclique en raison d'une liaison entre le résidu C-terminal et le résidu 3 en amont de celui-ci.

29. Peptide suivant la revendication 28, dans lequel X₂ représente Ala et X₅ représente Ile.

30. Peptide suivant la revendication 28 ou 29, dans lequel Phe est remplacé par para-fluoro-Phe et His est remplacé par Ala ou est éliminé par délétion.

31. Peptide suivant la revendication 30, dans lequel :
(a) Lys est remplacé par Abu,
(b) le premier résidu Arg est remplacé par Gln et
(c) le second résidu Arg est remplacé par Cit ou Ser, et
(d) Ile est remplacé par Ala.

32. Peptide suivant l'une quelconque des revendications 28 à 31, choisi entre :
| | | | | | | | | | |
|---|---|---|---|---|---|---|---|---|---|
| H- | his- | Ala- | Lys- | Arg- | Arg- | Leu- | Ile- | Phe | -NH2 |
| H- | Ala- | Ala- | Lys- | Arg- | Arg- | Leu- | Ile- | Phe | -NH2 |
| | H- | Ala- | Lys- | Arg- | Arg- | Leu- | Ile- | Phe | -NH2 |
| H- | Thi- | Ala- | Lys- | Arg- | Arg- | Leu- | Ile- | Phe | -NH2 |
| H- | Hse- | Ala- | Lys- | Arg- | Arg- | Leu- | Ile- | Phe | -NH2 |
| H- | Phe- | Ala- | Lys- | Arg- | Arg- | Leu- | Ile- | Phe | -NH2 |
| H- | Dab- | Ala- | Lys- | Arg- | Arg- | Leu- | Ile- | Phe | -NH2 |
| H- | His- | Abu | Lys- | Arg- | Arg- | Leu- | Ile- | Phe | -NH2 |
| H- | His- | Val- | Lys- | Arg- | Arg- | Leu- | Ile- | Phe | -NH2 |
| H- | His- | Ala- | Arg- | Arg- | Arg- | Leu- | Ile- | Phe | -NH2 |
| H- | His- | Ala- | Lys- | Arg- | Arg- | Ile- | Ile- | Phe | -NH2 |
| H- | His- | Ala- | Lys- | Arg- | Arg- | Leu- | Leu | Phe | -NH2 |
| | | | | | | | | | |
| H- | His- | Ala- | Lys- | Arg- | Arg- | Leu- | Ile- | PFPhe | -NH2 |
| H- | His- | Ala- | Lys- | Arg- | Arg- | Leu- | Ile- | 2Nap | -NH2 |
| H- | His | Ala | Lys | Arg | Arg | Leu | Ile | D-Psa | OH |
| H- | His | Ser | Lys | Arg | Arg | Leu | Ile | Dhp | OH |
| H- | Ala- | Ala- | Abu- | Arg- | Arg- | Leu- | Ile- | PFPhe | -NH2 |
| H- | Ala- | Ala- | Lys- | Arg- | Arg- | Leu- | Ile- | PFPhe | -NH2 |
| H- | Ala | Ala- | Lys- | Arg- | Cit- | Leu- | Ile- | PFPhe | -NH2 |
| H- | Ala- | Ala- | Lys- | Arg- | Arg- | Leu- | Ala- | PFPhe | -NH2 |
| H- | Ala- | Ala- | Abu- | Arg- | Ser- | Leu- | Ile- | PFPhe | -NH2 |
| H- | Ala- | Ala- | Lys- | Gln- | Arg- | Leu- | Ile- | pFPhe | -NH2 |
| | H- | Ala- | Lys- | Arg- | Arg- | Leu- | Ile- | pFPhe | -NH2 |

33. Analyse *in vitro* pour identifier des substances candidates capables de se lier à une cycline associée à une enzyme CDK de régulation de G1 et/ou d'inhiber ladite enzyme, comprenant :
(a) la mise en contact d'un peptide tel que défini dans l'une quelconque des revendications 1 à 32, de ladite cycline, de ladite CDK et de ladite substance candidate, dans des conditions dans lesquelles, en l'absence de la substance candidate consistant en un inhibiteur de l'interaction cycline/CDK, le peptide dérivé de p21 se lie à ladite cycline, et
(b) le contrôle de toute variation de la liaison prévue du peptide et de la cycline.

34. Analyse *in vitro* pour l'identification de composés qui interagissent avec une cycline ou une cycline à l'état complexé avec la CDK physiologiquement appropriée, comprenant :
(a) la mise en incubation d'un composé candidat et d'un peptide de formule X₁X₂X₃RX₄LX₅F (SEQ ID N° 2) et de formule HX₂KRRLX₅F (SEQ ID N° 3) dans lesquelles X₂ représente la sérine ou l'alanine, et X₁, X₃, X₄ et X₅ peuvent représenter n'importe quel aminoacide ; ou leurs variants tels que définis dans l'une quelconque des revendications 15 à 32, et d'une cycline ou d'un complexe cycline/CDK,
(b) la détection de la liaison du composé candidat ou du peptide de SEQ ID N° 2 ou SEQ ID N° 3 avec la cycline.

35. Analyse suivant l'une quelconque des revendications 33 et 34, dans laquelle la cycline est choisie entre la cycline A, la cycline E et la cycline D.

36. Analyse suivant la revendication 35, dans laquelle la cycline est la cycline A.

37. Analyse suivant l'une quelconque des revendications 33 à 36, comprenant l'utilisation d'un modèle tridimensionnel d'une cycline et d'un composé candidat.

38. Analyse suivant l'une quelconque des revendications 33 à 36, dans laquelle au moins un des constituants analytiques est lié à une phase solide.

39. Analyse suivant la revendication 38, dans laquelle le peptide est marqué de manière à émettre un signal lorsqu'il est lié à ladite cycline.

40. Analyse suivant la revendication 38, dans laquelle la cycline est marquée de manière à émettre un signal lorsqu'elle est liée au peptide.

41. Analyse suivant la revendication 39 ou 40, dans laquelle un des constituants analytiques est marqué avec un agent émetteur de fluorescence et le signal est détecté en utilisant des techniques de polarisation de fluorescence.

42. Méthode ou analyse suivant l'une quelconque des revendications 33 à 36, dans laquelle la méthode de détection comprend le contrôle du cycle cellulaire G0 et/ou G1/S, de l'apoptose liée au cycle cellulaire, de la suppression du facteur de transcription E2F, de l'hypophosphorylation du pRb cellulaire, ou d'effets antiprolifératifs *in vitro.*

43. Utilisation d'un peptide défini dans l'une quelconque des revendications 1 à 32, dans la préparation d'un médicament destiné à être utilisé dans (a) l'inhibition de CDK2 ou (b) le traitement de troubles prolifératifs tels que des cancers et des leucémies où l'inhibition de CDK2 serait bénéfique.
